(19) **Europäisches Patentamt / European Patent Office / Office européen des brevets**

(11) **EP 4 190 383 A1**

(12) **EUROPEAN PATENT APPLICATION**
published in accordance with Art. 153(4) EPC

(43) Date of publication:
07.06.2023 Bulletin 2023/23

(21) Application number: 20947262.0

(22) Date of filing: 30.10.2020

(51) International Patent Classification (IPC):
$A61M\ 21/02^{(2006.01)}$    $A61M\ 16/10^{(2006.01)}$
$A61M\ 16/00^{(2006.01)}$    $A61M\ 21/00^{(2006.01)}$

(52) Cooperative Patent Classification (CPC):
A61M 16/00; A61M 16/10; A61M 21/00;
A61M 21/02

(86) International application number:
PCT/KR2020/015013

(87) International publication number:
WO 2022/025351 (03.02.2022 Gazette 2022/05)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 28.07.2020  KR 20200094110
28.07.2020  KR 20200094111
28.07.2020  KR 20200094112

(71) Applicant: **NYX, Inc.**
**Jeollanam-do 58325 (KR)**

(72) Inventors:
• **KIM, Dong Sin**
**Seoul 05553 (KR)**
• **LEE, Jae Hong**
**Seoul 05601 (KR)**

(74) Representative: **Dantz, Jan Henning et al**
**Loesenbeck - Specht - Dantz**
**Patent- und Rechtsanwälte**
**Am Zwinger 2**
**33602 Bielefeld (DE)**

(54) **SLEEP ASSISTING DEVICE AND METHOD FOR CONTROLLING SLEEP ASSISTING DEVICE**

(57)    In this specification, there may be provided a sleep assisting device including a flow path unit configured to provide a movement path through which a gas moves, a flow rate adjustment unit configured to introduce a first gas to the flow path unit from the outside, a supply adjustment unit configured to adjust a supply amount of the carbon dioxide supplied to the flow path unit, an injection port connected to the flow path unit, and configured to provide a second gas in which the first gas and the carbon dioxide supplied to the flow path unit are mixed to the outside, a distance detection unit configured to acquire a distance indication value related to a distance between the injection port and an object, and a control unit configured to control the flow rate adjustment unit to adjust an amount of the first gas introduced into the flow path unit, and control the supply adjustment unit to adjust the supply amount, wherein the control unit controls the supply adjustment unit to supply the carbon dioxide to the flow path unit when the distance indication value acquired through the distance detection unit is greater than a first reference value and smaller than a second reference value.

FIG. 15

| SLEEP ASSISTING DEVICE | |
|---|---|
| CONTROL UNIT | CARBON DIOXIDE SUPPLY UNIT — 101 |
| | FLOW PATH UNIT — 104 |
| | SUPPLY ADJUSTMENT UNIT — 102 |
| | FLOW RATE ADJUSTMENT UNIT — 106 |

100 — 103 —

EP 4 190 383 A1

**Description**

[Technical Field]

This application relates to a sleep assisting device and a method of controlling the sleep assisting device, and more specifically, to a device and method for assisting a user's sleep by introducing outside air into the inside of a sleep assisting device, and supplying the user with a mixed gas in which the introduced outside air and carbon dioxide are mixed.

[Background Art]

As of 2020, the number of patients with sleep disorders has increased by an average of 8.1 % per year for the past five years. The sleep disorder is a disease that is commonly seen as a physical symptom of stress in modern society, and as a reflection thereof, sleep inducing agents using antihistamines, such as diphenhydramine or doxylamine may be easily obtained even without a prescription under the medical law.

However, there are problems in that the antihistamines easily accompany side effects such as headache and dizziness, it is difficult to determine an accurate dosage suitable for an individual, and the antihistamines interfere with daily life when taken because of a sleep inducing effect that persists even after waking up.

[Technical Problem]

In this specification, there may be provided a device for inducing sleep by supplying a mixed gas for inducing sleep at a concentration of carbon dioxide and a flow rate suitable for an individual to reduce an oxygen partial pressure in a user's body, and a method of controlling the device.

[Technical Solution]

In this specification, there may be provided a sleep assisting device including a flow path unit configured to provide a movement path through which a gas moves, a flow rate adjustment unit configured to introduce a first gas into the flow path unit from the outside, a supply adjustment unit configured to adjust a supply amount of carbon dioxide supplied to the flow path unit, an injection port connected to the flow path unit and configured to provide a second gas in which the first gas and the carbon dioxide supplied to the flow path unit are mixed to the outside, a distance detection unit configured to acquire a distance indication value related to a distance between the injection port and an object, and a control unit configured to control the flow rate adjustment unit to adjust an amount of the first gas introduced into the flow path unit, and control the supply adjustment unit to adjust the supply amount, wherein the control unit controls the supply adjustment unit to adjust the carbon dioxide to the flow path unit when the distance indication value acquired through the distance detection unit is greater than a first reference value and smaller than a second reference value.

In this specification, there may be provided a method of controlling a sleep assisting device including a flow path unit configured to provide a movement path through which a gas moves, a flow rate adjustment unit configured to introduce a first gas into the flow path unit from the outside, a supply adjustment unit configured to adjust a supply amount of carbon dioxide supplied to the flow path unit, an injection port connected to the flow path unit and configured to provide a second gas in which the first gas and the carbon dioxide supplied to the flow path unit are mixed to the outside, a distance detection unit configured to acquire a distance indication value related to a distance between the injection port and an object, and a control unit configured to control the flow rate adjustment unit to adjust an amount of the first gas introduced into the flow path unit, and control the supply adjustment unit to adjust the supply amount, the method including acquiring a first distance indication value related to the distance between the injection port and the object through the distance detection unit and controlling the supply adjustment unit to supply the carbon dioxide to the flow path unit by a control unit when the first distance indication value is greater than a first reference value and smaller than a second reference value.

In this specification, a sleep assisting device includes a flow path unit configured to provide a movement path through which a gas moves, a flow rate adjustment unit configured to adjust a flow rate by introducing a first gas into the flow path unit from the outside, a supply adjustment unit configured to adjust a supply amount of carbon dioxide supplied to the flow path unit, an injection port connected to the flow path unit and configured to provide a second gas in which the first gas and the carbon dioxide supplied to the flow path unit are mixed to the outside, a distance detection unit configured to acquire a distance indication value related to a distance between the injection port and an object, and a control unit configured to control the flow rate adjustment unit to adjust an amount of the first gas introduced into the flow path unit, and control the supply adjustment unit to adjust the supply amount, wherein the control unit may control the supply adjustment unit to adjust the supply amount based on the distance indication value.

In this specification, there may be provided a method of controlling a sleep assisting device including a flow path unit configured to provide a movement path through which a gas moves, a flow rate adjustment unit configured to adjust a flow rate by introducing a first gas into the flow path unit from the outside, a supply adjustment unit configured to adjust a supply amount of carbon dioxide supplied to the flow path unit, an injection port connected to the flow path unit and configured to provide a second gas in which the first gas and the carbon dioxide supplied to the flow path unit are mixed to the outside, a distance detection unit configured to acquire a distance indication value related to a distance between the injection port and an object, and a control unit configured to control the flow rate adjustment unit to adjust an amount of the first gas introduced into the flow path unit, and control the supply adjustment unit to adjust the supply amount, the method including acquiring the distance indication value through the distance detection unit and controlling the supply adjustment unit to adjust the supply amount based on the distance indication value by the control unit.

[Advantageous Effects]

In this specification, it is possible to provide a sleep assisting device and method for assisting a user's sleep who has difficulty in hypnagogue by supplying a mixed gas containing carbon dioxide.

In this specification, it is possible to provide a sleep assisting device and method for assisting a user's sleep by supplying a mixed gas containing carbon dioxide to decrease an oxygen partial pressure in a user's body.

In this specification, it is possible to provide a sleep assisting device and method for assisting the user's wake-up by supplying a gas or stimulating user's visual, auditory, and/or olfactory senses.

[Description of Drawings]

FIG. 1 is first experimental data representing a user's recognition result for a flow rate of a gas supplied by a sleep assisting device.

FIG. 2 is second experimental data representing the user's recognition result for the flow rate of the gas supplied by the sleep assisting device.

FIG. 3 is third experimental data representing the user's recognition result for the flow rate of the gas supplied by the sleep assisting device.

FIG. 4 is fourth experimental data representing a user's evaluation result for a sleepiness inducing effect according to the supply of carbon dioxide.

FIG. 5 is fifth experimental data representing the user's evaluation result for the sleepiness inducing effect according to the supply of the carbon dioxide.

FIG. 6 is sixth experimental data representing a carbon dioxide concentration in a target area according to a voltage of a flow rate adjustment unit.

FIG. 7 is a view for describing a target distance (d) of a sleep assisting device according to one embodiment.

FIG. 8 is a block diagram showing a configuration of a sleep assisting device according to a first embodiment.

FIG. 9 is a block diagram showing a configuration of a sleep assisting device according to a second embodiment.

FIG. 10 is a view showing a specific embodiment of an injection port of the sleep assisting device according to one embodiment.

FIG. 11 is a view showing the sleep assisting device including one distance adjustment unit according to one embodiment.

FIG. 12 is a view showing the sleep assisting device including two distance adjustment units according to one embodiment.

FIG. 13 is a flowchart showing a method of controlling a power source of the sleep assisting device based on a distance indication value related to a distance between the injection port and an object.

FIG. 14 is a flowchart showing a method of controlling a supply adjustment unit and a flow rate adjustment unit based on the distance indication value related to the distance between the injection port and the object.

FIG. 15 is a block diagram showing a configuration of a sleep assisting device according to a third embodiment.

FIG. 16 is a flowchart showing a method of controlling the supply adjustment unit and the flow rate adjustment unit to adjust a flow rate and/or a carbon dioxide concentration based on a user input.

FIG. 17 is seventh experimental data representing a carbon dioxide concentration at the injection port according to a carbon dioxide supply pressure.

FIG. 18 is eighth experimental data representing a flow rate at the injection port according to a voltage applied to the flow rate adjustment unit.

FIG. 19 is ninth experimental data representing a carbon dioxide concentration at the injection port according to the voltage applied to the flow rate adjustment unit.

FIG. 20 is tenth experimental data representing a carbon dioxide concentration according to a distance from the injection port when the carbon dioxide supply pressure is 0.1 [bar].

FIG. 21 is eleventh experimental data representing a carbon dioxide concentration according to a distance from the injection port when the carbon dioxide supply pressure is 0.2 [bar].

FIG. 22 is twelfth experimental data representing a carbon dioxide concentration according to a distance from the injection port when the carbon dioxide supply pressure is 0.3 [bar].

FIG. 23 is thirteenth experimental data representing a flow rate of a gas according to a distance (or target distance) from the injection port.

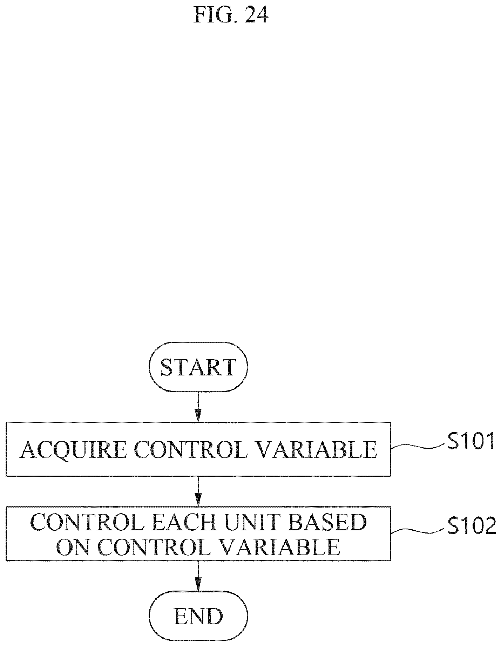

FIG. 24 is a flowchart showing a method of controlling the sleep assisting device based on a control variable.

FIG. 25 is a block diagram showing a configuration of a sleep assisting system.

FIG. 26 is a flowchart showing a method of operating the sleep assisting device in an operation mode determined based on the user input.

FIG. 27 is a flowchart showing a method of operating the sleep assisting device in a wake-up assisting operation mode determined based on the user input.

FIG. 28 is thirteenth experimental data comparing an oxygen concentration change rate and a carbon dioxide concentration change rate according to a sleep assisting operation of the sleep assisting device.

FIG. 29 is fourteenth experimental data comparing the oxygen concentration change rate and the carbon dioxide concentration change rate according to the sleep assisting operation of the sleep assisting device.

FIG. 30 is fifteenth experimental data comparing the oxygen concentration change rate and the carbon dioxide concentration change rate according to the sleep assisting operation of the sleep assisting device.

[Modes of the Invention]

Hereinafter, specific embodiments according to the present invention will be described in detail with reference to the accompanying drawings. However, the spirit of the present invention is not limited to the presented embodiments, and those skilled in the art who understand the spirit of the present invention may easily suggest other degenerative inventions or other embodiments included within the scope of the spirit of the invention by adding changing, deleting, and the like other components within the scope of the same spirit, but this will also be included within the scope of the spirit of the present invention. In addition, components having the same function within the scope of the same spirit shown in the drawings of each embodiment will be described using the same reference numerals.

When it is determined that a detailed description of a known function or configuration related to the present invention may unnecessarily obscure the gist of the present invention, a detailed description thereof will be omitted. In addition, suffixes "module" and "unit" for the components used in the following description are given or used interchangeably in consideration of only the ease of preparing the specification, and do not have distinct meanings or roles by themselves.

1. Object of the invention

This specification is directed to providing a device for providing or supplying a gas having an appropriate carbon dioxide concentration for sleep induction.

This specification is directed to providing a device for appropriately controlling a flow rate of a gas and a carbon dioxide concentration of the gas supplied from an injection port in order to induce a user's sleep in a manner of maintaining a carbon dioxide concentration in the air haled by a user who is positioned within a constant distance from the injection port according to the user's respiration at a level suitable for sleep induction.

Specifically, the device provided by this specification can induce the user's sleep by providing a gas having an appropriate carbon dioxide concentration, and introducing the gas into a body according to the user's respiration to temporarily decrease an oxygen partial pressure in the user's body.

In this specification, there is provided a sleep assisting device including a flow path unit configured to provide a movement path through which a gas moves, a flow rate adjustment unit configured to introduce a first gas to the flow path unit from the outside, a supply adjustment unit configured to adjust a supply amount of the carbon dioxide supplied to the flow path unit, an injection port connected to the flow path unit, and configured to provide a second gas in which the first gas and the carbon dioxide supplied to the flow path unit are mixed to the outside, a distance detection unit configured to acquire a distance indication value related to a distance between the injection port and an object, and a control unit configured to control the flow rate adjustment unit to adjust an amount of the first gas introduced into the flow path unit, and control the supply adjustment unit to adjust the supply amount, wherein the control unit controls the supply adjustment unit to supply the carbon dioxide to the flow path unit when the distance indication value acquired through the distance detection unit is greater than a first reference value and smaller than a second reference value.

Here, the control unit may control the supply adjustment unit to supply the carbon dioxide to the flow path unit at a first supply pressure when the distance indication value is a first value that is greater than the first reference value and smaller than the second reference value, and control the supply adjustment unit to supply the carbon dioxide to the flow path unit at a second supply pressure greater than the first supply pressure when the distance indication value is a second value that is greater than the first reference value and smaller than the second reference value, and greater than the first value.

Here, the control unit may control the supply adjustment unit to supply the carbon dioxide to the flow path unit at the first supply pressure when the distance indication value is the first value that is greater than the first reference value and smaller than the second reference value, and control the supply adjustment unit to supply the carbon dioxide to the flow path unit at the second supply pressure smaller than the first supply amount when the distance indication value is the second value that is greater than the first reference value and smaller than the second reference value, and greater than the first value.

Here, when the distance indication value indicates that a distance between the injection port and the object is within a reference distance range, the control unit may control the supply adjustment unit to supply the carbon dioxide to the flow path unit.

Here, when the distance indication value is smaller than the first reference value, the control unit may control the supply adjustment unit to stop the supply of the carbon dioxide to the flow path unit.

Here, when the distance indication value is greater than the second reference value, the control unit may control the supply adjustment unit to stop the supply of carbon dioxide to the flow path unit.

Here, when the distance indication value is smaller than the first reference value, the control unit may control the flow rate adjustment unit so as not to introduce the first gas into the flow path unit.

Here, when the distance indication value is greater than the second reference value, the control unit may control the flow rate adjustment unit so as not to introduce the first gas into the flow path unit.

Here, the carbon dioxide concentration in an area including at least a part of the object is 5,000 [ppm] or more and 30,000 [ppm] or less.

In this specification, there is provided a method of controlling a sleep assisting device including a flow path unit configured to provide a movement path through which a gas moves, a flow rate adjustment unit configured to introduce a first gas into the flow path unit from the outside, a supply adjustment unit configured to adjust a supply amount of the carbon dioxide supplied to the flow path unit, an injection port connected to the flow path unit, and configured to provide a second gas in which the first gas and the carbon dioxide supplied to the flow path unit are mixed to the outside, a distance detection unit configured to acquire a distance indication value related to a distance between the injection port and an object, and a control unit configured to control the flow rate adjustment unit to adjust an amount of the first gas introduced into the flow path unit, and control the supply adjustment unit to adjust the supply amount, the method including: acquiring a first distance indication value related to the distance between the injection port and the object through the distance detection unit; and controlling the supply adjustment unit to supply the carbon dioxide to the flow path unit by the control unit when the first distance indication value is greater than a first reference value and smaller than a second reference value.

Here, the controlling of the supply adjustment unit to supply the carbon dioxide to the flow path unit by the control unit when the first distance indication value is greater than the first reference value and smaller than the second reference value may further include controlling the supply adjustment unit to supply the carbon dioxide to the flow path unit at a first supply pressure by the control unit, and the method of controlling the sleep assisting device may further include: acquiring a second distance indication value related to the distance between the injection port and the object through the distance detection unit; and controlling the supply adjustment unit to supply the carbon dioxide to the flow path unit at a second supply pressure greater than the first supply amount by the control unit when the second distance indication value is greater than the first reference value and smaller than the second reference value, and greater than the first distance indication value.

Here, the controlling of the supply adjustment unit to supply the carbon dioxide to the flow path unit by the control unit when the first distance indication value is greater than the first reference value and smaller than the second reference value may further include controlling the supply adjustment unit to supply the carbon dioxide to the flow path unit at a first supply pressure by the control unit, and the method of controlling the sleep assisting device may further include acquiring the second distance indication value related to the distance between the injection port and the object through the distance detection unit and controlling the supply adjustment unit to supply the carbon dioxide to the flow path unit at a second supply pressure smaller than the first supply amount when the second distance indication value is greater than the first reference value and smaller than the second reference value, and greater than the first distance indication value.

Here, the controlling of the supply adjustment unit to supply the carbon dioxide to the flow path unit by the control unit when the first distance indication value is greater than the first reference value and smaller than the second reference value may further include controlling the supply adjustment unit to supply the carbon dioxide to the flow path unit by the control unit when the first distance indication value acquired through the distance detection unit indicates that the distance between the injection port and the object is within a reference range.

Here, the acquiring of the first distance indication value related to the distance between the injection port and the object through the distance detection unit by the control unit may further include controlling the supply unit so as not to supply the carbon dioxide to the flow path unit by the control unit when the first distance indication value is smaller than the first reference value.

Here, the acquiring of the first distance indication value related to the distance between the injection port and the object through the distance detection unit by the control unit may further include controlling the supply adjustment unit so as not to supply the carbon dioxide to the flow path unit by the control unit when the first distance indication value is greater than the second reference value.

Here, the acquiring of the first distance indication value related to the distance between the injection port and the object through the distance detection unit by the control unit may further include controlling the flow rate adjustment unit so as not to introduce the first gas into the flow path unit by the control unit when the first distance indication value is smaller than the first reference value.

Here, the acquiring of the first distance indication value related to the distance between the injection port and the object through the distance detection unit by the control unit may further include controlling the flow rate adjustment unit so as not to introduce the first gas into the flow path unit by the control unit when the first distance indication value is greater than the second reference value.

Here, a concentration of intake carbon dioxide of the object may be 5,000 [ppm] or more and 30,000 [ppm] or less.

In this specification, a sleep assisting device includes a flow path unit configured to provide a movement path through which a gas moves, a flow rate adjustment unit configured to adjust a flow rate by introducing a first gas into the flow path unit from the outside, a supply adjustment unit configured to adjust a supply amount of the carbon dioxide supplied to the flow path unit, an injection port connected to the flow path unit, and configured to provide a second gas in which the first gas and the carbon dioxide supplied to the flow path unit are mixed to the outside, a distance detection unit configured to acquire a distance indication value related to a distance between the injection port and an object, and a control unit configured to control the flow rate adjustment unit to adjust an amount of the first gas introduced into the flow path unit, and control the supply adjustment unit to adjust the supply amount, wherein the control unit may control the supply adjustment unit to adjust the supply amount based on the distance indication value.

Here, the control unit may control the supply adjustment unit so that the supply amount is a first supply amount when the distance indication value is a first value, and control the supply adjustment unit so that the supply amount is supplied at a second supply pressure greater than the first supply amount when the distance indication value is a second value greater than the first value.

Here, the control unit may control the supply adjustment unit so that the supply amount is a first supply amount when the distance indication value is a first value, and control the supply adjustment unit so that the supply amount is a second supply amount smaller than the first supply amount when the distance indication value is a second value greater than the first value.

Here, the control unit may control the supply adjustment unit so that the supply amount is a first supply amount when the distance indication value is greater than a first reference value, and control the supply adjustment unit so that the supply amount is a second supply amount greater than the first supply amount when the distance indication value is smaller than the first reference value.

Here, the control unit may control the supply adjustment unit so that the supply amount is a first supply amount when the distance indication value is greater than a first reference value, and control the supply adjustment unit so that the supply amount is a second supply amount smaller than the first supply amount when the distance indication value is smaller than the first reference value.

Here, the control unit may control the supply adjustment unit to supply the carbon dioxide to the flow path unit when the distance indication value is greater than a first reference value, and control the supply adjustment unit to stop the supply of the carbon dioxide to the flow path unit when the distance indication value is smaller than the first reference value.

Here, the control unit may control the supply adjustment unit to supply the carbon dioxide to the flow path unit when the distance indication value is smaller than the first reference value, and control the supply adjustment unit to stop the supply of the carbon dioxide to the flow path unit when the distance indication value is greater than the first reference value.

Here, the control unit may control the flow rate adjustment unit to introduce the first gas into the flow path unit by a first flow rate when the distance indication value is greater than a first reference value, and control the flow rate adjustment unit to introduce the first gas into the flow path unit by a second flow rate smaller than the first flow rate when the distance indication value is smaller than the first reference value.

Here, the control unit may control the flow rate adjustment unit to introduce the first gas into the flow path unit by a first flow rate when the distance indication value is greater than a distance reference value, and control the flow rate adjustment unit to introduce the first gas into the flow path unit by a second flow rate greater than the first flow rate when the distance indication value is greater than the distance reference value.

Here, the control unit may control the flow rate adjustment unit so as not to introduce the first gas into the flow path unit when the distance indication value is greater than a first reference value.

Here, the control unit may control the flow rate adjustment unit so as not to introduce the first gas into the flow path unit when the distance indication value is smaller than the first reference value.

In this specification, there is provided a method of controlling a sleep assisting device including a flow path unit configured to provide a movement path through which a gas moves, a flow rate adjustment unit configured to adjust a flow rate by introducing a first gas into the flow path unit from the outside, a supply adjustment unit configured to adjust a supply amount of the carbon dioxide supplied to the flow path unit, an injection port connected to the flow path unit, and configured to provide a second gas in which the first gas and the carbon dioxide supplied to the flow path unit are mixed to the outside, a distance detection unit configured to acquire a distance indication value related to a distance between the injection port and an object, and a control unit configured to control the flow rate adjustment unit to adjust an amount of the first gas introduced into the flow path unit, and control the supply adjustment unit to adjust the supply amount, the method including: acquiring the distance indication value through the distance detection unit; and controlling the supply adjustment unit to adjust the supply amount based on the distance indication value by the control unit.

Here, the controlling of the supply adjustment unit to adjust the supply amount based on the distance indication value by the control unit may further include controlling the supply adjustment unit so that the supply amount is a first supply amount by the control unit when the distance indication value is a first value, and controlling the supply adjustment unit so that the supply amount is a second supply amount greater than the first supply amount when the distance indication value is a second value greater than the first value.

Here, the controlling of the supply adjustment unit to adjust the supply amount of the carbon dioxide supplied to the flow path unit based on the distance indication value by the control unit may further include controlling the supply adjustment unit so that the supply amount is a first supply amount when the distance indication value is a first value, and controlling the supply adjustment unit so that the supply amount is a second supply amount smaller than the first supply amount when the distance indication value is a second value greater than the first value by the control unit.

Here, the controlling of the supply adjustment unit to adjust the supply amount of the carbon dioxide supplied to the flow path unit based on the distance indication value by the control unit may further include controlling the supply adjustment unit so that the supply amount is a first supply amount when the distance indication value is a first value, and controlling the supply adjustment unit so that the supply amount is a second supply amount greater than the first supply amount when the distance indication value is a second value greater than the first value by the control unit.

Here, the controlling of the supply adjustment unit to adjust the supply amount of the carbon dioxide supplied to the flow path unit based on the distance indication value by the control unit may further include controlling the supply adjustment unit so that the supply amount is a first supply amount when the distance indication value is greater than a distance reference value, and controlling the supply adjustment unit so that the supply amount is a second supply amount smaller than the first supply amount when the distance indication value is smaller than the distance reference value.

Here, the controlling of the supply adjustment unit to adjust the supply amount of the carbon dioxide supplied to the flow path unit based on the distance indication value by the control unit may further include controlling the supply adjustment unit to supply the carbon dioxide to the flow path unit when the distance indication value is greater than a first reference value, and controlling the supply adjustment unit to stop the supply of the carbon dioxide to the flow path unit when the distance indication value is smaller than the first reference value.

Here, the controlling of the supply adjustment unit to adjust the supply amount of the carbon dioxide supplied to the flow path unit based on the distance indication value by the control unit may further include controlling the supply adjustment unit to supply the carbon dioxide to the flow path unit when the distance indication value is smaller than a first reference value, and controlling the supply adjustment unit to stop the supply of the carbon dioxide to the flow path unit when the distance indication value is greater than the first reference value by the control unit.

Here, the controlling of the supply adjustment unit to adjust the supply amount of the carbon dioxide supplied to the flow path unit based on the distance indication value by the control unit may further include controlling the flow rate adjustment unit to introduce the first gas into the flow path unit by a first flow rate when the distance indication value is smaller than a distance reference value, and controlling the flow rate adjustment unit to introduce the first gas into the flow path unit by a second flow rate smaller than the first flow rate when the distance indication value is greater than the distance reference value.

Here, the controlling of the supply adjustment unit to adjust the supply amount of the carbon dioxide supplied to the flow path unit based on the distance indication value by the control unit may further include controlling the flow rate adjustment unit to introduce the first gas into the flow path unit by a first flow rate when the distance indication value is smaller than a distance reference value, and controlling the flow rate adjustment unit to introduce the first gas into the flow path unit by a second flow rate greater than the first flow rate when the distance indication value is greater than the distance reference value.

In this specification, the controlling of the supply adjustment unit to adjust the supply amount of the carbon dioxide supplied to the flow path unit based on the distance indication value by the control unit may further include controlling the flow rate adjustment unit so as not to introduce the first gas into the flow path unit by the control unit when the distance indication value is greater than a first reference value.

Here, the controlling of the supply adjustment unit to adjust the supply amount of the carbon dioxide supplied to the flow path unit based on the distance indication value by the control unit may further include controlling the flow rate adjustment unit so as not to introduce the first gas into the flow path unit by the control unit when the distance indication value is smaller than a first reference value.

2. Background knowledge

2.1 Relationship between oxygen partial pressure in body and sleep induction

The oxygen partial pressure in the body refers to a ratio of hemoglobin (Hb) bound to oxygen among hemoglobin (Hb) in arterial blood, and both arterial blood oxygen partial pressure (PaO2) and percutaneous oxygen saturation (SpO2) are used as an index of the oxygen partial pressure in the body. Although individual differences are present, it is generally known that an oxygen partial pressure (PaO2) in arterial blood of 79 to 100 (mmHg) and a blood oxygen saturation (SpO2) of 95 to 96 (%) are in a normal range.

In this regard, the results of analyzing the correlation between the oxygen partial pressure in the body and parameters representing a level of drowsiness have been published in a paper. In the paper, with respect to i) subjective evaluation of an individual, ii) the number of blinks per minute, and iii) LF/HF that refers to a ratio of parasympathetic nervous system and sympathetic nervous system activities, which are parameters representing the level of drowsiness, it can be seen that when the oxygen partial pressure in the body is decreased to a normal value or less, the level of drowsiness increases (Issey Takahashi, Tetsuo Takaishi, and Kiyoko Yokoyama (2014). Overcoming Drowsiness by Inducing Cardiorespiratory Phase Synchronization. IEEE TRANSACTIONS ON INTELLIGENT TRANSPORTATION SYSTEMS, VOL. 15, NO. 3).

Accordingly, it can be confirmed that a decrease in the oxygen partial pressure in the body may act as a cause of the sleep induction.

2.2 Relationship between oxygen partial pressure in body and partial pressure of intake oxygen

In general, the oxygen partial pressure in the body is closely related to the partial pressure of intake oxygen.

Oxygen in the atmosphere is transmitted to lungs according to respiration, and moves back to capillaries by a diffusion phenomenon between the lungs and the capillaries around the lungs, and at this time, the oxygen moving to the capillaries is bound to the hemoglobin (Hb) in the blood.

In other words, an amount of oxygen bound to hemoglobin (Hb) is determined according to the oxygen partial pressure transmitted to the lungs through intake.

Accordingly, in this specification, as a means for inducing sleep by decreasing the oxygen partial pressure in the body, a device for decreasing the oxygen partial pressure in an environment in which a user breathes may be considered.

2.3 Method of adjusting partial pressure of intake oxygen

In order to decrease the partial pressure of intake oxygen to a certain level or less, according to [Equation 1] below,

[Equation 1]

$$(\text{Mol number of oxygen gas per unit volume}/\text{mol number of entire gas per unit volume})*10^6 = \text{oxygen concentration (ppm)}$$

there is a method of i) partially removing an oxygen gas per unit volume in the atmosphere, or ii) injecting a gas other than oxygen into the atmosphere.

However, the method of selectively removing only oxygen gas molecules from the atmosphere requires a more complicated process than simply injecting a gas, and thus it is difficult to efficiently configure the device, so that the method of injecting the gas other than oxygen into the atmosphere is applied to the sleep assisting device disclosed by this specification.

2.4 Relationship between oxygen partial pressure in body and concentration of intake carbon dioxide

Carbon dioxide may be considered as the gas other than the oxygen to be injected to decrease the partial pressure of intake oxygen.

Since the carbon dioxide may be bound to hemoglobin (Hb) like oxygen, a carbon dioxide partial pressure in the body increases when a partial pressure of intake carbon dioxide increases, and as a result, the oxygen partial pressure in the body decreases.

In addition, since the carbon dioxide is a gas involved in human respiration due to the presence of an average of 0.04 % in the atmosphere, it is possible to safely decrease the partial pressure of intake oxygen of the user when the partial pressure is adjusted based on objective data.

Accordingly, the sleep assisting device disclosed by this specification induces the user's sleep in a manner of decreasing the oxygen partial pressure in the user's body by supplying carbon dioxide.

3. Considerations upon supplying carbon dioxide for sleep assistance

3.1 Safety

3.1.1 Overview

The sleep assisting device may supply the user with a mixed gas having an adjusted carbon dioxide concentration to decrease the oxygen partial pressure in the body for the purpose of sleep induction.

At this time, as a result of introducing the mixed gas having the adjusted carbon dioxide concentration into the body through intake, when the oxygen partial pressure in the body continues for a long time in a state of being significantly decreased from a value in the normal range or being smaller than the value in the normal range, it may result in a harmful result to the human body, and thus, safety should be considered based on objective data in designing the configuration and operation of the sleep assisting device.

3.1.2 Domestic safety standards

Article 2 in exposure standards for chemical substances and physical factors (Ministry of Employment and Labor Notification No. 2018-62) and <Attached Table 1> exposure standards for chemical substances announce the exposure standards for a case in which workers are exposed to harmful factors in order to protect the health of the workers from gases or the like that are harmful to the human body (hereinafter referred to as "harmful factors").

The exposure standard refers to a standard that does not adversely affect the health of almost all workers at a level that is smaller than or equal to the exposure standards when the workers are exposed to the harmful factors.

According to Article 3 of the General Rule (Notes on the use of the exposure standards) announced by the exposure standards for chemical substances and physical factors, the exposure standard refers to an exposure standard for a case in which a corresponding harmful factor is present alone, and an exposure standard separately calculated should be used when two or more harmful factors are present together.

Since the sleep assisting device to be described below in this specification corresponds to a gas in which the atmosphere and carbon dioxide are mixed, and unless the gas is used for purposes other than sleep assistance, there is no case in which the supplied carbon dioxide coexists with other types of harmful factors, so that there is no unreasonableness in applying the exposure standards for the carbon dioxide concentration announced in the exposure standards of chemical substances and physical factors to the sleep assisting device provided in this specification.

The exposure standards for chemical substances and physical factors were established based on 8-hour work a day with respect to the exposure standard of carbon dioxide, and announce the exposure standard of carbon dioxide as an average of 5000 ppm based on 8 hours a day, and an average of 30,000 ppm based on 4 times or less a day and 15 minutes at a time.

### 3.1.3 Overseas safety standards

In the United States, in NIOSH POCKET GUIDE TO CHEMICAL HAZARDS (DHHS Publication No. 2005-149), the exposure standard of carbon dioxide is announced as an average of 5000 ppm based on 8 hours a day and an average of 30,000 ppm based on 15 minutes.

In Europe, in EH40/2005 WORKPLACE EXPOSURE LIMITS (Fourth Edition 2020), the exposure standard of carbon dioxide is announced as an average of 5000 ppm based on 8 hours a day and an average of 15000 ppm based on 15 minutes.

### 3.1.4 Appropriate carbon dioxide concentration and injection time considering safety

According to the domestic and overseas exposure standards of carbon dioxide, exposure to an environment in which the carbon dioxide concentration is an average of 5000 ppm based on 8 hours a day or exposure to an environment in which the carbon dioxide concentration is an average of 30,000 ppm based on 4 times or less a day and 15 minutes at a time does not harmfully affect the human body.

In addition, the exposure to the environment having a maximum carbon dioxide concentration announced by the domestic and overseas carbon dioxide exposure standards or the exposure for the time of 15 minutes or less does not harmfully affect the human body.

The sleep assisting device may inject a carbon dioxide gas to satisfy the carbon dioxide concentration/supply time in consideration of safety.

The sleep assisting device may supply a gas having a carbon dioxide concentration that satisfies the domestic and overseas carbon dioxide exposure standards.

The sleep assisting device may supply a gas containing carbon dioxide for a supply time that satisfies the domestic and overseas carbon dioxide exposure standards. The carbon dioxide concentration that satisfies the exposure standard of carbon dioxide may be determined with respect to an area around the user's respiratory organ. In addition, the carbon dioxide concentration that satisfies the exposure standard of the carbon dioxide may be determined with respect to an area positioned to be spaced apart from the sleep assisting device by a predetermined distance.

The supply time that satisfies the exposure standard of carbon dioxide may be determined with respect to the area around the user's respiratory organ. In addition, the supply time that satisfies the exposure standard of carbon dioxide may be determined with respect to an area positioned to be spaced apart from the sleep assisting device by a predetermined distance.

According to one embodiment, the sleep assisting device may supply a gas having the adjusted carbon dioxide concentration for a first time (e.g., for 15 minutes) so that the carbon dioxide concentration has a value that is a first concentration (e.g., 5000 [ppm]) or more and a second concentration (e.g., 30,000 [ppm]) or less in an area (e.g., target area) where the user's respiratory organ is positioned.

For example, the sleep assisting device may supply a gas in which the carbon dioxide concentration is adjusted to 5000 [ppm] or more and 30,000 [ppm] or less. For another example, the sleep assisting device may supply the gas in which the carbon dioxide concentration is adjusted to 5000 [ppm] or more and 30,000 [ppm] or less for 15 minutes.

For another example, the sleep assisting device may supply the gas having the adjusted carbon dioxide concentration so that the carbon dioxide concentration has a value of 5000 [ppm] or more and 30,000 [ppm] or less for 15 minutes in the area (e.g., target area) where the user's respiratory organ is positioned.

According to another embodiment, the sleep assisting device may supply the gas having the adjusted carbon dioxide concentration for a second time (e.g., 10 minutes) shorter than the first time. At this time, the carbon dioxide concentration in the area (e.g., target area) around the user's respiratory organ may be adjusted to a third concentration (e.g., 5500 [ppm]) or more that is greater than the first concentration and a fourth concentration (e.g., 33,000 [ppm]) or less that is greater than the second concentration.

According to another embodiment, the sleep assisting device may supply the gas having the adjusted carbon dioxide concentration for a third time (e.g., 20 minutes) longer than the first time. At this time, the carbon dioxide concentration in the area (e.g., target area) around the user's respiratory organ may be adjusted to a fifth concentration (e.g., 4,500 [ppm]) or more that is smaller than the first concentration and a sixth concentration (e.g., 27,000 [ppm]) or less that is smaller than the second concentration.

The carbon dioxide concentration and gas injection time of the gas supplied by the sleep assisting device are not limited to the above-described values. The carbon dioxide concentration and gas injection time of the gas supplied by the sleep assisting device may be changed within a range of values based on the announced exposure standard for carbon dioxide.

The carbon dioxide concentration and carbon dioxide supply time of the gas supplied by the sleep assisting device may be changed according to an update of the domestic or overseas carbon dioxide exposure standard.

The carbon dioxide concentration and carbon dioxide supply time of the gas supplied by the sleep assisting device may be changed based on other objective data on the safety of carbon dioxide not described in this specification.

In addition, the carbon dioxide concentration and carbon dioxide supply time of the gas supplied by the sleep assisting device may be changed based on laws or regulations on the safety of carbon dioxide.

However, the carbon dioxide concentration of the gas supplied by the sleep assisting device is not limited to a value within a numerical range considering the exposure standards. The carbon dioxide concentration of the gas supplied by the sleep assisting device may be determined as a value within other numerical ranges according to other factors affecting safety. The other factors affecting safety may include an individual's sensitivity to carbon dioxide, a volume of a space where the sleep assisting device is positioned, whether the space is sealed, and temperature and humidity in the space where the sleep assisting device is positioned.

3.2 Effectiveness

Since the sleep assisting device is directed to providing the mixed gas that decreases the oxygen partial pressure in the user's body, the carbon dioxide concentration in the area where the user is positioned should be determined as a value within a range in which the user's sleep inducing effect is expressed.

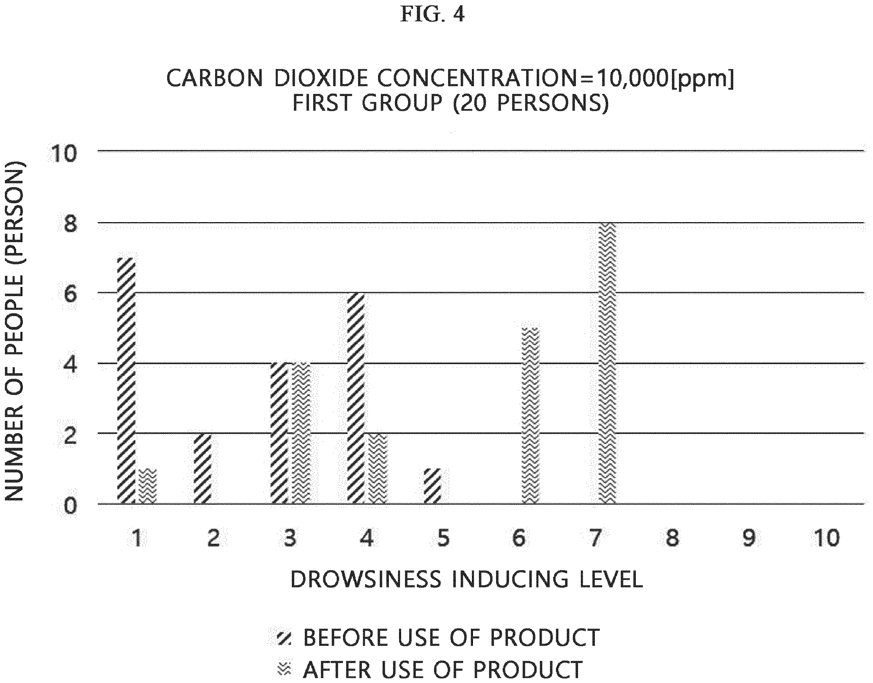

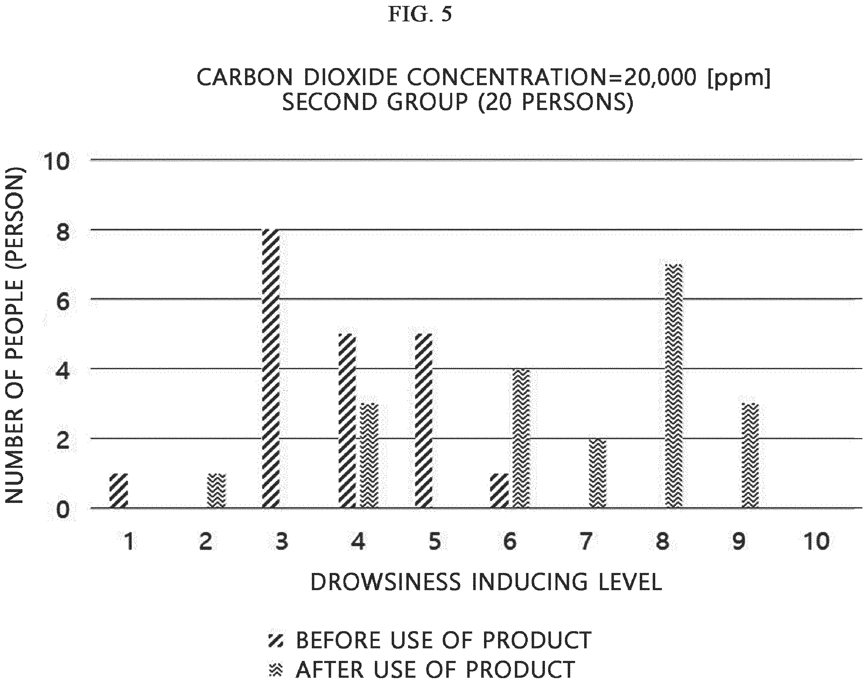

FIGS. 4 and 5 are fourth and fifth experimental data representing the user's evaluation results on the drowsiness inducing effect according to the supply of the carbon dioxide.

Referring to FIG. 4, an individual user's evaluation on the level at which drowsiness was caused before the use of a sleep assisting product was acquired from 20 users. In addition, an individual user's evaluation on the level at which drowsiness was caused after the use of the sleep assisting product that allows the concentration of intake carbon dioxide to be 10,000 [ppm] was acquired from the same 20 users.

As a result, it was found that the number of users who evaluated the level at which drowsiness was caused as 1 before the use of the sleep assisting product had the maximum value (7 people). In addition, it was found that the number of users who evaluated the level at which drowsiness was caused as 7 after the use of the sleep assisting product had the maximum value (8 people).

In addition, it was found that before the use of the sleep assisting product, an average value of the level at which drowsiness was caused was 2.6, whereas after the use of the sleep assisting product, the average value of the level at which drowsiness was caused was 5.35.

Referring to FIG. 5, the individual user's evaluation on the level at which drowsiness was caused before the use of the sleep assisting product was acquired from 20 users. In addition, the individual user's evaluation on the level at which drowsiness was caused after the use of the sleep assisting product that allows the concentration of intake carbon dioxide to be 20,000 [ppm] was acquired from the same 20 users.

As a result, it was found that the number of users who evaluated the level at which drowsiness was caused as 3 before the use of the sleep assisting product had the maximum value (8 people). In addition, it was found that the number of users who evaluated the level at which drowsiness was caused as 7 after the use of the sleep assisting product had the maximum value (8 people).

In addition, it was found that before the use of the sleep assisting product, an average value of the level at which drowsiness was caused was 3.8, whereas after the use of the sleep assisting product, the average value of the level at which drowsiness was caused was 6.75.

Accordingly, it can be understood that exposure to an environment in which the concentration of intake carbon dioxide is 10,000 [ppm] or more and 20,000 [ppm] or less causes the sleep inducing effect.

Accordingly, it can be understood that exposure to an environment in which the concentration of intake carbon dioxide has a value within the numerical range considering safety described in 3.1 (Safety) causes the sleep inducting effect.

However, since the experiment described in 3.2 (Effectiveness) was conducted only when the concentration of intake carbon dioxide had one exemplary value (10,000 [ppm] or 20,000 [ppm]) within the numerical range considering safety, the concentration of intake carbon dioxide at which the sleep inducing effect is caused is not limited to 10,000 [ppm] or 20,000 [ppm].

3.3 User convenience

3.3.1 Supply method

The sleep assisting device provided in this specification may be provided in a non-contact type. Since the sleep assisting device is directed to inducing the user's sleep, the sleep assisting device may be provided in the non-contact type so that the configuration of the device does not include factors that interfere with the user's sleep.

The sleep assisting device provided in this specification may assist the user's sleep in the non-contact type.

The sleep assisting device provided in this specification may assist the user's sleep in a manner of being spaced apart from the user by a predetermined distance and not coming into contact with the user's body. Specifically, the sleep assisting device may supply a gas for assisting the user's sleep in the manner of being positioned to be spaced apart from the user by the predetermined distance and not coming into contact with the user's body.

When the sleep assisting device is provided in a contact-type configuration that stimulates the user's tactile sense, the stimulation may act as a factor that interferes with the user's sleep. For example, the contact-type configuration such as a mask for sealing a periphery of the user's respiratory organ may act as a factor that interferes with the user's sleep because it comes in contact with the user's body.

In contrast, the sleep assisting device provided in this specification may be provided in the non-contact type, thereby minimizing the stimulation that interferes with the user's sleep.

Meanwhile, the sleep assisting device may also be implemented in a partial-contact type without interfering with the user's sleep.

For example, the sleep assisting device may be provided in the form built in bedding generally used in a sleep environment. In addition, the sleep assisting device may be provided integrally with the bedding generally used in the sleep environment. In addition, the sleep assisting device may be provided to be detachably attached to the bedding generally used in the sleep environment. For example, the sleep assisting device may also induce the user's sleep using a cushion or a bed.

3.3.2 Supply flow rate

The sleep assisting device provided in this specification may be provided in a non-stimulation type. Since the sleep assisting device is directed to inducing the user's sleep, the sleep assisting device may be provided in the non-stimulation type so that the operation of the device does not include a factor that interferes with the user's sleep.

For example, when the flow rate of the gas supplied by the sleep assisting device is greater than a certain flow rate, the flow rate may stimulate the user's tactile sense, and thus act as a factor that interferes with the user's sleep.

Accordingly, the flow rate of the gas supplied by the device may be maintained at a predetermined flow rate or less so as not to interfere with the user's sleep.

The sleep assisting device may inject the mixed gas at a predetermined flow rate so as not to stimulate the user's tactile sense.

The flow rate of the gas supplied by the device may be predetermined based on the user's experience. For example, the flow rate of the gas may be determined as a value within a range in which the user recognizes that the stimulation of the tactile sense does not interfere with the sleep at a position spaced apart from the injection port of the device by a predetermined distance.

At this time, the predetermined flow rate may be set based on clinical experiments.

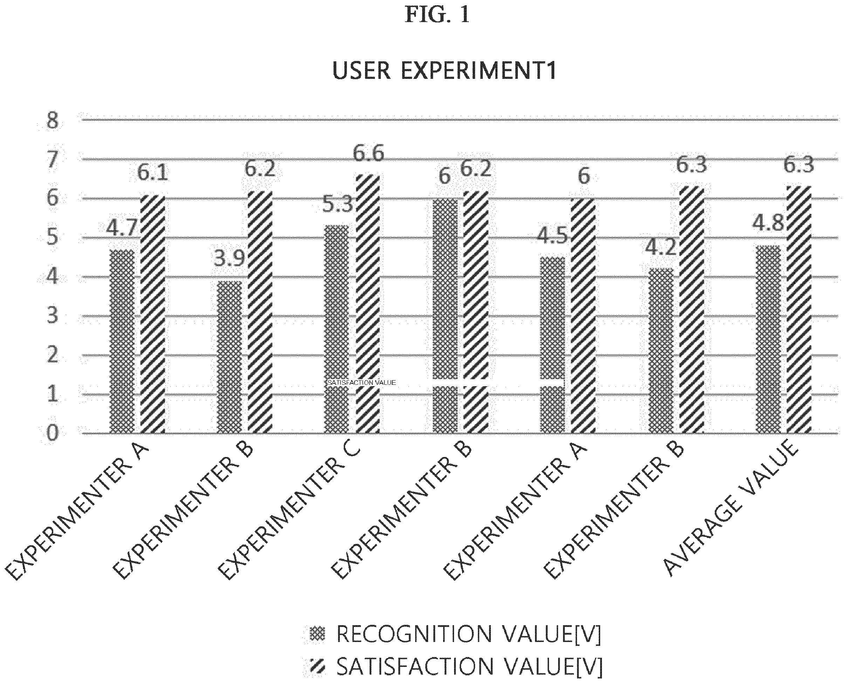

FIGS. 1 to 3 are first to third experimental data representing the user's recognition results on the flow rate of the gas supplied by the sleep assisting device.

Referring to FIG. 1, an average of minimum voltages (hereinafter referred to as a "recognition value") applied to the flow rate adjustment unit at which experimenters who participated in a first experiment recognized that the tactile sense of the user positioned to be spaced apart from the injection port of the sleep assisting device by a predetermined distance was stimulated by the gas supplied through the sleep assisting device is 4.8 [V].

Referring to FIG. 1, an average of maximum voltages (hereinafter referred to as a "satisfaction value") applied to the flow rate adjustment unit at which the experimenters who participated in the first experiment recognized that the stimulation of the tactile sense of the user positioned to be spaced apart from the injection port of the sleep assisting device by a predetermined distance by the gas supplied through the sleep assisting device did not interfere with the sleep is 6.3 [V].

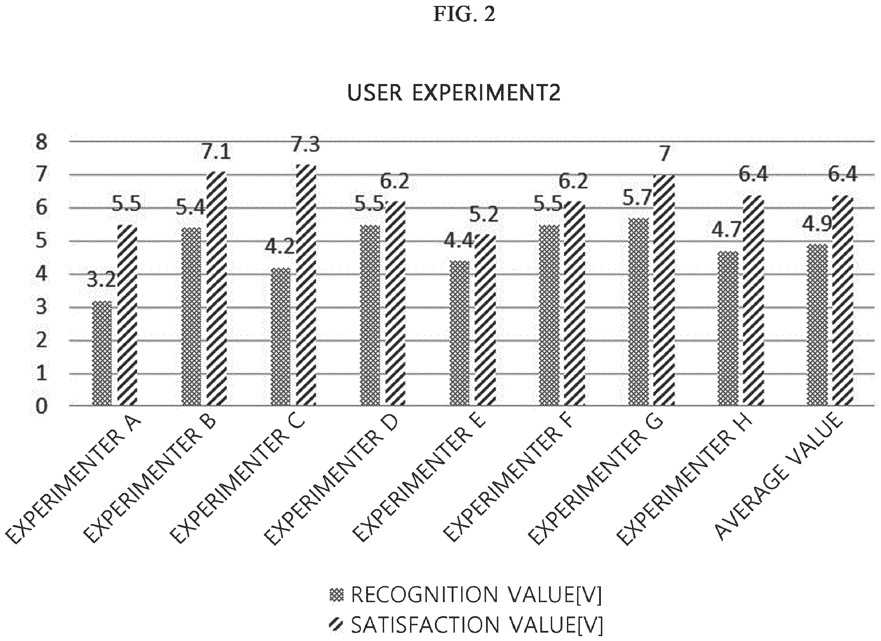

Referring to FIG. 2, an average of recognition values of experimenters who participated in a second experiment is 4.9 [V].

Referring to FIG. 2, an average of satisfaction values of the experimenters who participated in the second experiment is 6.4 [V].

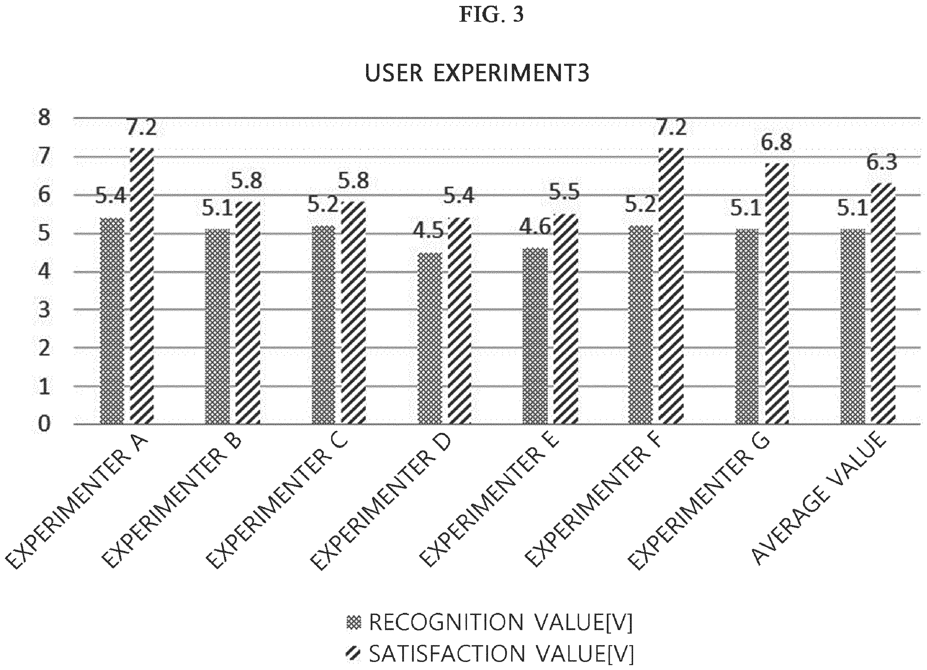

Referring to FIG. 3, an average of recognition values of the experimenters who participated in the third experiment is 5.1 [V].

Referring to FIG. 3, an average of satisfaction values of the experimenters who participated in the third experiment is 6.3 [V].

Accordingly, the average of the recognition values of the first to third experiments is 4.9 [V], and the average of the satisfaction values thereof is 6.3 [V].

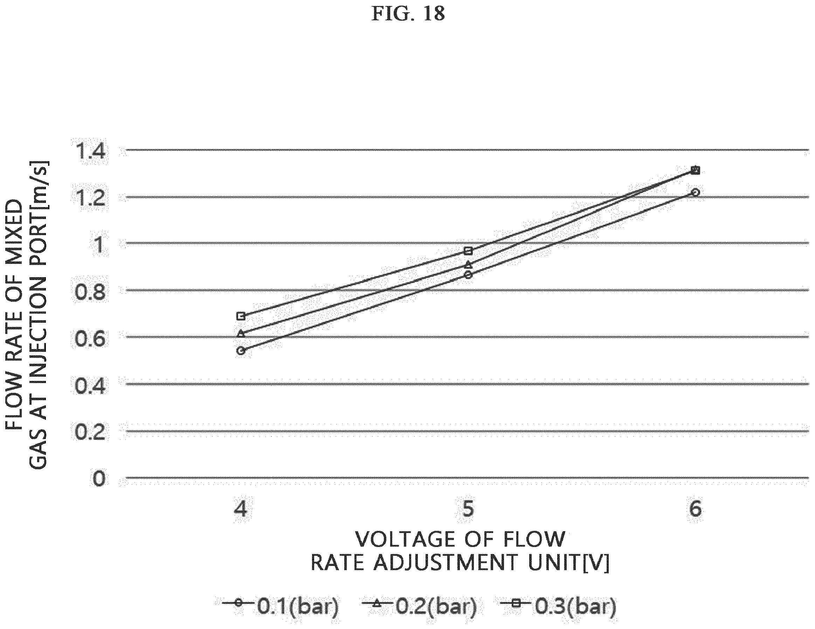

FIG. 18 is a graph of the eighth experimental data obtained by measuring the carbon dioxide concentration at the injection port according to a voltage of the flow rate adjustment unit, which will be described below.

Referring to FIG. 18, an average of the flow rates at the injection port according to the voltages applied to the flow rate adjustment unit used in the first to third experiments corresponds to 0.62 [m/s] at 4 [V].

Referring to FIG. 18, an average of the flow rates at the injection port according to the voltages applied to the flow rate adjustment unit used in the first to third experiments corresponds to 1.25 [m/s] at 6 [V].

Based on the above experimental data, the flow rate at the injection port is 0.9 [m/s] when the average of the recognition values is 4.9 [V] on the assumption that the flow rate in the target area increases linearly when the voltage applied to the flow rate adjustment unit increases by 1 [V].

In addition, based on the above experimental data, the flow rate at the injection port is 1.34 [m/s] when the average of the satisfaction values is 6.3 [V] on the assumption that the flow rate in the target area increases linearly when the voltage applied to the flow rate adjustment unit increases by 1 [V].

According to the first to third experiments, the flow rate of the gas at the injection port may be determined as a value that is greater than a first flow rate (e.g., 0.9 [m/s]) and smaller than a second flow rate (e.g., 1.34 [m/s]) determined in consideration of the user convenience.

However, the flow rate at the injection port considering the user convenience may be determined differently according to other clinical experimental results. In addition, the flow rate at the injection port considering the user convenience may be determined differently according to the user's subjective preference.

According to one embodiment, the minimum value of the flow rate at the injection port considering the user convenience may be determined as a value smaller than the first flow rate.

According to another embodiment, the minimum value of the flow rate at the injection port considering the user convenience may be determined as a value greater than the first flow rate.

According to another embodiment, in the sleep assisting device, the maximum value of the flow rate at the injection port may be determined as a value smaller than the second flow rate in consideration of the user convenience.

According to another embodiment, in the sleep assisting device, the maximum value of the flow rate at the injection port may be determined as a value greater than the second flow rate in consideration of the user convenience.

For example, the flow rate of the gas at the injection port may be determined as a value that is greater than 0.8 [m/s] and smaller than 1.24 [m/s] in consideration of the user convenience.

For another example, the flow rate of the gas at the injection port may be determined as a value that is greater than 0.8 [m/s] and smaller than 1.44 [m/s] in consideration of the user convenience.

For another example, the flow rate of the gas at the injection port may be determined as a value that is greater than 1.0 [m/s] and smaller than 1.24 [m/s] in consideration of the user convenience.

For another example, the flow rate of the gas at the injection port may be determined as a value that is greater than 1.0 [m/s] and smaller than 1.44 [m/s] in consideration of the user convenience.

3.4 Efficiency

The sleep assisting device may include a carbon dioxide supply source in order to supply a gas having the adjusted carbon dioxide concentration.

When the carbon dioxide supply source of the sleep assisting device is a consumable, the flow rate of the supplied gas may be determined in consideration of efficiency of carbon dioxide supply.

The sleep assisting device may introduce outside air so that the supplied carbon dioxide is supplied to the outside. When the amount of carbon dioxide supplied through the carbon dioxide supply source is constant, the sleep assisting device may introduce the outside air so that the carbon dioxide is supplied to a position farther away from the device or the injection port.

The sleep assisting device may adjust an amount of the introduced outside air so that the supplied carbon dioxide is supplied to the outside with directivity due to the introduced outside air. The sleep assisting device may adjust the amount of the introduced outside air so that the carbon dioxide is supplied to a position spaced apart from the device or the injection port by a certain distance in a predetermined direction at a certain concentration or more.

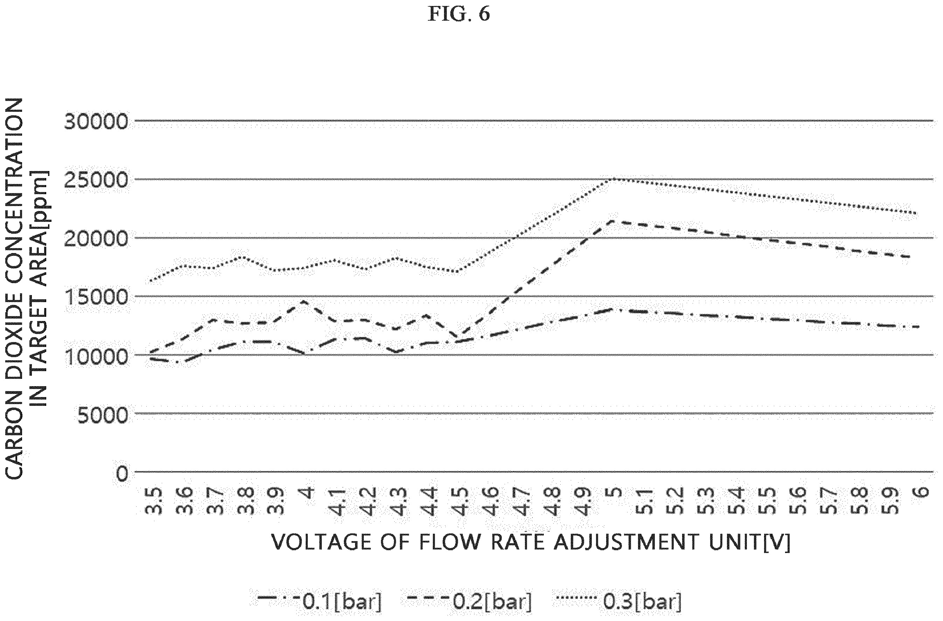

FIG. 6 is sixth experimental data representing the carbon dioxide concentration in the target area according to the voltage of the flow rate adjustment unit.

Here, when the voltage of the flow rate adjustment unit increases, the amount of the outside air introduced into the inside of the sleep assisting device per unit time may also increase. The significance of the voltage of the flow rate adjustment and the correlation between the voltage of the flow rate adjustment unit and the amount of the introduced outside air will be described below in 5.2.1 (Significance of voltage applied to flow rate adjustment unit). The significance of the target area will be described below in 4.1 (Overview).

Referring to FIG. 6, it was found that the carbon dioxide concentration in the target area (e.g., area around the user's respiratory organ) was maximum when the supply amount of the carbon dioxide was 0.1 [bar] and the voltage of the flow rate adjustment unit was 4.8 [V] or more and 5 [V] or less.

In addition, it is shown that the carbon dioxide concentration in the target area (e.g., area around the user's respiratory organ) is maximum when the supply amount of the carbon dioxide is 0.2 [bar] and the voltage of the flow rate adjustment unit is 4.8 [V] or more and 5.2 [V] or less.

In addition, it is shown that the carbon dioxide concentration in the target area (e.g., area around the user's respiratory organ) is maximum when the supply amount of the carbon dioxide is 0.3 [bar] and the voltage of the flow rate adjustment unit is 4.8 [V] or more and 5.2 [V] or less.

Accordingly, it can be understood that the supplied carbon dioxide is efficiently transmitted to the target area (e.g., area around the user's respiratory organ) when the voltage applied to the flow rate adjustment unit is 4.5 [V] or more and 5.5 [V] or less.

Accordingly, it can be understood that the supplied carbon dioxide is efficiently transmitted to the target area (e.g., area around the user's respiratory organ) when the voltage applied to the flow rate adjustment unit is 4 [V] or more and 6 [V] or less.

However, the voltage of the flow rate adjustment unit for efficiently transmitting the carbon dioxide to the target area is not limited to the above-described values.

The voltage of the flow rate adjustment unit for efficiently transmitting the carbon dioxide to the target area may be determined as a value different from the above-described values in consideration of other factors affecting the carbon dioxide concentration in the target area. The factors affecting the carbon dioxide concentration in the target area may include a carbon dioxide supply pressure, temperature, humidity, and a target distance.

4. Configuration of device

4.1 Overview

In 4 (Configuration of device), the sleep assisting device and each unit will be described in detail.

The sleep assisting device provided in this specification may be a device for providing carbon dioxide to decrease the oxygen partial pressure in the user's body.

In addition, the sleep assisting device may be a device for supplying a carbon dioxide-rich mixed gas. The carbon dioxide-rich gas may refer to a mixed gas having a greater carbon dioxide concentration than an average carbon dioxide concentration in the atmosphere. In addition, the carbon dioxide-rich gas may refer to a mixed gas having a greater carbon dioxide concentration than the carbon dioxide concentration in the outside air introduced into the sleep assisting device.

In addition, the sleep assisting device may be a device for supplying the mixed gas to an area positioned to be spaced apart from the injection port by a constant distance.

In order to clearly describe the sleep assisting device provided in this specification, terms may be defined as follows.

The mixed gas used in this specification may refer to a gas in which the carbon dioxide provided by the sleep assisting device and the outside air introduced into the sleep assisting device are mixed.

In addition, the mixed gas may refer to a gas in which the gas supplied to the outside from the sleep assisting device and the outside air present outside the sleep assisting device are mixed.

In addition, the mixed gas may refer to a gas having the adjusted carbon dioxide concentration by the operation of the unit included in the sleep assisting device.

In addition, the mixed gas may have a carbon dioxide concentration equal to or similar to the carbon dioxide concentration in the atmosphere. Alternatively, the mixed gas may also have a greater carbon dioxide concentration than the carbon dioxide concentration in the atmosphere because the carbon dioxide is injected.

The mixed gas may be a gas in which a concentration of a component other than the carbon dioxide is adjusted. For example, the sleep assisting device may provide a mixed gas having a concentration of oxygen or nitrogen different from that of the atmosphere. Alternatively, the sleep assisting device may provide a mixed gas further containing an additional effective component for assisting sleep in the atmospheric component.

The injection port used in this specification may refer to a point at which the mixed gas starts to be supplied from the sleep assisting device to the outside of the sleep assisting device.

The injection port used in 4.1 (Overview) of this specification may refer to a point at which the carbon dioxide starts to be supplied from the carbon dioxide supply unit to the outside.

The injection port used in 4.1 (Overview) of this specification may refer to a point at which the mixed gas starts to be supplied from the sleep assisting device to the outside.

The target area used in this specification may refer to an area where the carbon dioxide or a gas having the adjusted carbon dioxide concentration, which is supplied by the sleep assisting device, reaches through the movement from the injection port by a constant distance.

The target area may be a target area for providing the carbon dioxide or the gas having the adjusted carbon dioxide concentration by the sleep assisting device. The target area may be a target area where an appropriate sleep inducing environment is provided by the sleep assisting device.

In addition, the target area may refer to an area where the carbon dioxide or the gas having the adjusted carbon dioxide concentration, which is supplied by the sleep assisting device, moves from the injection port and has a concentration within the numerical range that satisfies the safety and effectiveness described in 3 (Considerations upon supplying carbon dioxide for sleep assistance).

In addition, the target area may refer to an area where the carbon dioxide or the gas having the adjusted carbon dioxide concentration, which is supplied by the sleep assisting device, moves at a flow rate within the numerical range that satisfies the user convenience and efficiency described in 3 (Considerations upon supplying carbon dioxide for sleep assistance) from the injection port, and has the concentration within the numerical range that satisfies the safety and effectiveness described in 3 (Considerations upon supplying carbon dioxide for sleep assistance).

In addition, the target area may refer to an area including at least a part of the user's respiratory organ when the user who expects the sleep assisting effect using the sleep assisting device is positioned to be spaced apart from the injection port by a constant distance.

The target distance used in this specification may refer to a distance from the injection port to the target area.

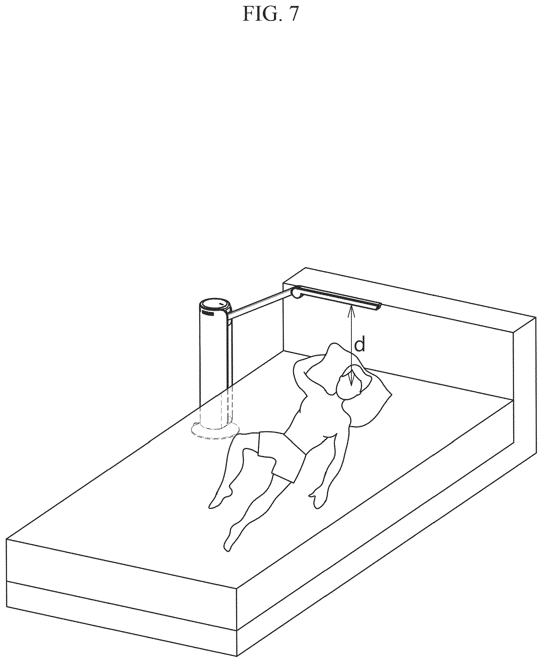

FIG. 7 is a view for describing a target distance d of a sleep assisting device according to one embodiment.

The target distance may refer to a distance from the injection port to an area where the carbon dioxide or the gas having the adjusted carbon dioxide concentration, which is supplied by the sleep assisting device, moves and has the concentration within the numerical range that satisfies the safety and effectiveness described in 3 (Considerations upon supplying carbon dioxide for sleep assistance). The target distance may refer to a distance from the injection port of the sleep inducing device to the target area where a sleep inducing device provides the mixed gas and provides the sleep inducing environment that satisfies the safety and effectiveness standards.

In addition, the target distance may refer to a distance from the injection port to the area where the carbon dioxide or the gas having the adjusted carbon dioxide concentration, which is supplied by the sleep assisting device, moves at the flow rate within the numerical range that satisfies the user convenience and efficiency described in 3 (Considerations upon supplying carbon dioxide for sleep assistance), and has the concentration within the numerical range that satisfies the safety and efficiency described in 3 (Considerations upon supplying carbon dioxide for sleep assistance).

In addition, the target distance may refer to a distance from the injection port to an area including at least a part of the user's respiratory organ. The target distance may refer to a distance from the injection port to an area around the user's respiratory organ. The target distance may refer to a distance from the injection port to an area where the user's head is generally positioned.

Hereinafter, the object and configuration of the sleep assisting device according to a specific embodiment will be described in detail.

4.2 Sleep assisting device according to first embodiment

4.2.1 Object of sleep assisting device according to first embodiment

The sleep assisting device according to the first embodiment is directed to supplying the carbon dioxide to the user to decrease the oxygen partial pressure in the user's body.

The sleep assistance used in this specification may be construed as having the same meaning as sleep induction.

The sleep assisting device according to the first embodiment is directed to supplying the carbon dioxide to the user so that the oxygen partial pressure in the user's body decreases as the carbon dioxide concentration in the target area decreases.

4.2.2 Configuration and operation of device according to first embodiment

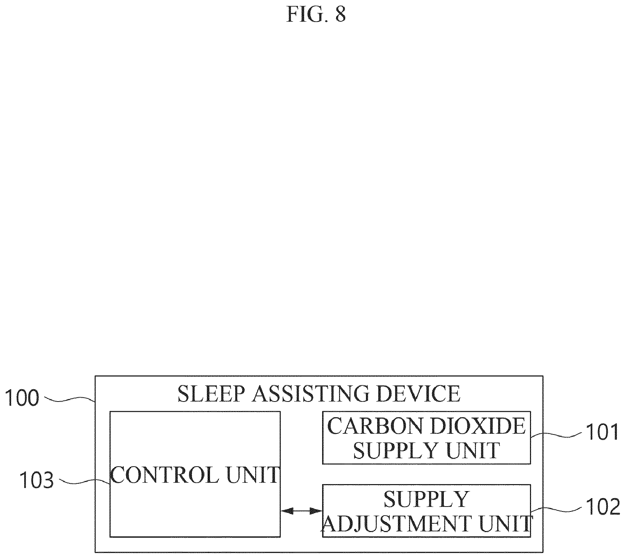

FIG. 8 is a block diagram showing the configuration of the sleep assisting device according to the first embodiment.

The sleep assisting device according to the first embodiment may supply carbon dioxide to the outside. The supply may mean that the sleep assisting device provides the carbon dioxide to the outside according to an external operation. In addition, the supply may mean that the sleep assisting device directly provides the carbon dioxide to the outside.

The sleep assisting device according to the first embodiment may supply the carbon dioxide so that the carbon dioxide concentration in the area positioned to be spaced apart from the sleep assisting device by the predetermined distance has the value within the numerical range that satisfies the safety and effectiveness described in 3 (Considerations upon supplying carbon dioxide for sleep assistance).

The sleep assisting device according to the first embodiment may supply the carbon dioxide so that the flow rate of the gas in the area positioned to be spaced apart from the sleep assisting device by the predetermined distance has the value within the numerical range that satisfies the user convenience and efficiency described in 3 (Considerations upon supplying carbon dioxide for sleep assistance).

Referring to FIG. 8, a sleep assisting device 100 according to the first embodiment may include a carbon dioxide supply unit 101, a supply adjustment unit 102, and a control unit 103.

Hereinafter, each configuration of the sleep assisting device 100 according to the first embodiment will be described in detail.

The carbon dioxide supply unit 101 may supply carbon dioxide.

The carbon dioxide supply unit 101 may supply the carbon dioxide in a gaseous state. The carbon dioxide supply unit 101 may supply the carbon dioxide in a state of being mixed with other materials. For example, the carbon dioxide supply unit 101 may supply a mixed gas in which the carbon dioxide is mixed with other gases.

The carbon dioxide supply unit 101 may supply the carbon dioxide so that the carbon dioxide is injected into the outside. The carbon dioxide supply unit 101 may supply the carbon dioxide so that the carbon dioxide is diffused to the outside.

The carbon dioxide supply unit 101 may supply the carbon dioxide so that the carbon dioxide reaches the target area and the carbon dioxide concentration in the target area is a predetermined concentration. The predetermined concentration may refer to the carbon dioxide concentration having the value within the numerical range that satisfies the safety and effectiveness described in 3 (Considerations upon supplying carbon dioxide for sleep assistance).

The carbon dioxide supply unit 101 may supply the carbon dioxide for a predetermined time. The predetermined time may refer to a time that satisfies the safety described in 3 (Considerations upon supplying carbon dioxide for sleep assistance). The predetermined time may be changed based on a predetermined concentration. For example, when the predetermined time is a first time (e.g., 15 minutes), the predetermined concentration may be a first concentration (e.g., 15,000 [ppm]). For another example, when the predetermined time is a second time (e.g., 20 minutes) longer than the first time, the predetermined concentration may be a second concentration (e.g., 13,500 [ppm]) smaller than the first concentration.

The carbon dioxide supply unit 101 may store the carbon dioxide. The carbon dioxide supply unit 101 may include a container configured to store the carbon dioxide. For example, the carbon dioxide supply unit 101 may include a cylinder in which the carbon dioxide is stored. Alternatively, the carbon dioxide supply unit 101 may include a spray in which the carbon dioxide is stored.

The carbon dioxide supply unit 101 may supply the stored carbon dioxide to the outside. For example, the carbon dioxide supply unit 101 may supply the carbon dioxide to the outside using the carbon dioxide stored in the cylinder.

The carbon dioxide supply unit 101 may store the carbon dioxide in a gaseous, liquid, or solid state. The carbon dioxide supply unit 101 may supply the carbon dioxide in the gaseous, liquid, or solid state to the outside in a gaseous, liquid or solid state. For example, the carbon dioxide supply unit 101 may store the carbon dioxide in the liquid state, and supply the carbon dioxide in the gaseous state to the outside using the stored carbon dioxide in the liquid state.

The carbon dioxide supply unit 101 may extract the carbon dioxide from the atmosphere. The carbon dioxide supply unit 101 may supply the carbon dioxide extracted from the atmosphere.

The carbon dioxide supply unit 101 may extract the carbon dioxide from the user's exhalation. The carbon dioxide supply unit 101 may supply the carbon dioxide extracted from the user's exhalation.

The carbon dioxide supply unit 101 may be disposed so that a point at which the carbon dioxide starts to be supplied from the carbon dioxide supply unit 101 to the outside is positioned at an upper portion of the carbon dioxide supply unit. The injection port of the carbon dioxide supply unit 101 may be positioned at the upper portion of the carbon dioxide supply unit 101 in the sleep assisting device 100.

The carbon dioxide in the liquid state is stored inside the container, and the stored carbon dioxide in the liquid state may be supplied to the outside in the gaseous state. At this time, the carbon dioxide in the liquid state and the carbon dioxide in the gaseous state may coexist inside the container. Accordingly, when the injection port is positioned at a lower portion of the carbon dioxide supply unit, the carbon dioxide in the liquid state may interfere with a path through which the carbon dioxide in the gaseous state is supplied because the carbon dioxide in the liquid state having a greater density than that of the carbon dioxide in the gaseous state is positioned at a lower portion inside the container. Accordingly, in order to smoothly supply the carbon dioxide in the gaseous state to the outside, the injection port may be positioned at the upper portion of the carbon dioxide supply unit 101.

The supply adjustment unit 102 may adjust the carbon dioxide supply pressure.

The supply adjustment unit 102 may be connected to the carbon dioxide supply unit 101 and configured to adjust the supply pressure of the carbon dioxide supplied by the carbon dioxide supply unit 101.

The supply pressure used in this specification may refer to a carbon dioxide supply pressure of the carbon dioxide supply unit 101.

The supply pressure may refer to a carbon dioxide supply pressure of the carbon dioxide supply unit 101 determined by the supply adjustment unit 102.

The supply pressure may refer to a pressure difference between the inside of the carbon dioxide supply unit 101 and the outside of the carbon dioxide supply unit 101. For example, when the pressure outside the carbon dioxide supply unit is an atmospheric pressure (e.g., 1 atmosphere), the supply pressure may refer to a pressure difference between the carbon dioxide supply unit and the atmospheric pressure.

The supply pressure may be converted into a supply amount of the carbon dioxide. In addition, the supply pressure may be converted into the supply amount of the carbon dioxide per unit time.

When the supply pressure is zero, the carbon dioxide supply unit 101 may be in a state of not supplying the carbon dioxide to the outside.

The supply adjustment unit 102 may adjust the carbon dioxide supply pressure by adjusting a difference between a pressure inside the carbon dioxide supply unit 101 and a pressure outside the carbon dioxide supply unit 101.

According to one embodiment, the supply adjustment unit 102 may include a means for adjusting the pressure of the carbon dioxide gas (e.g., a solenoid valve, a regulator, or a pressure relief valve) and a means for adjusting the supply amount of the carbon dioxide gas (e.g., a step motor or a proportional flow control valve).

According to one embodiment, the supply adjustment unit 102 may include at least any one of the solenoid valve, the pressure relief valve, the regulator, the step motor, and the proportional flow control valve.

According to one embodiment, the supply adjustment unit 102 may include the regulator and the step motor, and decrease the supply pressure to a first pressure primarily using the regulator and then determine the supply amount at which the gas supplied at the first pressure secondarily using the step motor is supplied to the outside.

According to another embodiment, the supply adjustment unit 102 may include the regulator and the proportional flow control valve, and decrease the supply pressure to the first pressure primarily using the regulator and then determine the supply amount at which the gas supplied at the first pressure secondarily using the proportional flow control valve is supplied to the outside.

The supply adjustment unit 102 may increase or decrease the carbon dioxide supply pressure. The supply adjustment unit 102 may increase or decrease the supply amount of the carbon dioxide. The supply adjustment unit 102 may increase or decrease the supply amount of the carbon dioxide per unit time.

According to one embodiment, the supply adjustment unit 102 may increase the carbon dioxide supply pressure from zero to the first pressure. In addition, the supply adjustment unit 102 may increase the carbon dioxide supply pressure from the first pressure to a second pressure greater than the first pressure.

According to another embodiment, the supply adjustment unit 102 may decrease the carbon dioxide supply pressure from the second pressure to the first pressure. In addition, the supply adjustment unit 102 may decrease the carbon dioxide supply pressure from the first pressure to zero. At this time, when the supply pressure of the carbon dioxide supply unit 101 is zero, it may be a state in which the carbon dioxide is not supplied.

According to another embodiment, the supply adjustment unit 102 may increase or decrease the carbon dioxide supply pressure by a predetermined pressure.

For example, the supply adjustment unit 102 may increase or decrease the carbon dioxide supply pressure by $\beta$ (e.g., 0.1 [bar]). Specifically, the supply adjustment unit 102 may increase the carbon dioxide supply pressure from $\alpha$ [bar] (e.g., 0.1 [bar]) to $(\alpha+\beta)$ (e.g., 0.2 [bar]). In addition, the supply adjustment unit 102 may increase the carbon dioxide supply pressure from $(\alpha+\beta)$ (e.g., 0.2 [bar]) to $(\alpha+2*\beta)$ (e.g., 0.3 [bar]).

The supply adjustment unit 102 may include the pressure relief valve and the step motor, and increase or decrease the carbon dioxide supply pressure by a predetermined pressure as the step motor is adjusted by a predetermined interval.

The supply adjustment unit 102 may increase or decrease the carbon dioxide supply pressure over a plurality of times. For example, the supply adjustment unit 102 may primarily decrease the supply pressure from the first pressure to the second pressure, and then secondarily decrease the supply pressure from the second pressure to a third pressure smaller than the second pressure.

The control unit 103 may control the operations of the sleep assisting device 100 and/or each unit included in the sleep assisting device 100.

For example, the control unit 103 of the sleep assisting device 100 according to the first embodiment may change the supply pressure of the supply adjustment unit 102 by controlling the supply adjustment unit 102.

The control unit 103 may acquire an input, and control the operations of the sleep assisting device 100 and/or each unit included in the sleep assisting device 100 based on the acquired input. Here, the input may refer to an input acquired through a user interface included in the sleep assisting device 100. In addition, the input may refer to an input acquired through an external device. For example, the external device may refer to a user terminal, a tablet PC, a PC, or a remote controller. For another example, the external device may refer to a device for acquiring information on a surrounding area where the sleep assisting device 100 is positioned. As a more specific example, the external device may refer to a device for acquiring information on the carbon dioxide concentration, temperature, humidity, flow rate, illuminance, and the like of the surrounding area where the sleep assisting device 100 is positioned.

The control used in this specification may include generating a control signal. The control may include generating the control signal and transmitting the generated control signal to each unit to be controlled or the external device.

The control unit 103 may control a power source of the sleep assisting device 100. The control unit 103 may control the sleep assisting device to be turned off or turned on.

The control unit 103 may control the sleep assisting device 100 so that the sleep assisting device 100 performs the sleep assisting operation. The control unit 103 may control each unit included in the sleep assisting device 100 so that the sleep assisting device 100 performs the sleep assisting operation.

The control unit 103 may control the sleep assisting device 100 so that the sleep assisting device 100 supplies carbon dioxide. The control unit 103 may control the supply adjustment unit 102 so that the carbon dioxide supply unit 101 supplies the carbon dioxide. Specifically, the control unit 103 may control the supply adjustment unit 102 so that the carbon dioxide supply pressure is changed from zero to the first pressure greater than zero.

The control unit 103 may control the supply adjustment unit 102 so that the supply adjustment unit 102 adjusts the carbon dioxide supply pressure by a predetermined pressure. The control unit 103 may control the supply adjustment unit 102 so that the supply adjustment unit 102 increases or decreases the carbon dioxide supply pressure by the predetermined pressure.

The control unit 103 may control the supply adjustment unit 102 so that the carbon dioxide supply unit 101 supplies the carbon dioxide for a predetermined time. The predetermined time may be a time (e.g., 15 minutes) within the numerical range that satisfies the safety described in 3 (Considerations upon supplying carbon dioxide for sleep assistance).

The control unit 103 may control the supply adjustment unit 102 to increase or decrease the time for which the carbon dioxide supply unit 101 supplies the carbon dioxide. Specifically, the control unit 103 may increase or decrease the supply time for which the carbon dioxide supply unit 101 supplies the carbon dioxide based on the carbon dioxide supply pressure.

For example, the control unit 103 may control the supply adjustment unit 102 so that the carbon dioxide supply unit 101 supplies the carbon dioxide at the first pressure (e.g., 15,000 [ppm]) for the first time (e.g., 15 minutes). The control unit 103 may control the supply adjustment unit 102 so that the carbon dioxide supply unit 101 supplies the carbon dioxide at the second pressure (e.g., 13,500 [ppm]) for the second time (e.g., 20 minutes) longer than the first time. At this time, the second pressure may be smaller than the first pressure.

The sleep assisting device according to the first embodiment may further include a carbon dioxide concentration detection unit (not shown). The carbon dioxide concentration detection unit may include at least one of an infrared gas sensor (NDIR), a semiconductor type sensor, a solid electrolyte sensor, and a thermal conductivity type sensor.

The carbon dioxide concentration detection unit may acquire the carbon dioxide concentration. The carbon dioxide concentration detection unit may acquire information on the carbon dioxide concentration. The carbon dioxide concentration detection unit may acquire a carbon dioxide concentration measurement value based on the information on the carbon dioxide concentration.

The carbon dioxide concentration detection unit may acquire a carbon dioxide concentration at a predetermined point. The carbon dioxide concentration detection unit may acquire information on the carbon dioxide concentration of the gas including at least a part of the carbon dioxide supplied by the carbon dioxide supply unit 101 at the predetermined point.

The carbon dioxide concentration detection unit may acquire information on the carbon dioxide concentration in an area including at least a part of the injection port of the carbon dioxide supply unit 101. The carbon dioxide concentration detection unit may acquire information on the carbon dioxide concentration in an area around the injection port of the carbon dioxide supply unit 101.

In addition, the carbon dioxide concentration detection unit may acquire information on the carbon dioxide concentration in an area spaced apart from the sleep assisting device 100 by a predetermined distance.

In addition, the carbon dioxide concentration detection unit may acquire information on the carbon dioxide concentration in an area including at least a part of the user's respiratory organ. The carbon dioxide concentration detection unit may acquire information on the carbon dioxide concentration in the area around the user's respiratory organ.

In addition, the carbon dioxide concentration detection unit may acquire the carbon dioxide concentration in the target area.

The carbon dioxide concentration detection unit may acquire a measurement value of the carbon dioxide concentration, and transmit the acquired measurement value to each unit of the sleep assisting device 100. For example, the carbon dioxide concentration detection unit may transmit the acquired measurement value of the carbon dioxide concentration to the control unit 103.

The carbon dioxide concentration detection unit may transmit the acquired measurement value to the external device. For example, the carbon dioxide concentration detection unit may transmit the acquired measurement value of the carbon dioxide concentration to the user terminal or the PC. For another example, the carbon dioxide concentration detection unit may also transmit the acquired measurement value to a separate server.

The control unit 103 may be connected to the carbon dioxide concentration detection unit wirelessly or by wire.

The control unit 103 may be connected to the carbon dioxide concentration detection unit and configured to control the operation of the carbon dioxide concentration detection unit.

The control unit 103 may acquire data from the carbon dioxide concentration detection unit. The control unit 103 may acquire the measurement value of the carbon dioxide concentration from the carbon dioxide concentration detection unit.

The control unit 103 may control the operation of the sleep assisting device 100 based on the measurement value of the carbon dioxide concentration acquired from the carbon dioxide concentration detection unit.

For example, when the measurement value acquired through the carbon dioxide concentration detection unit is greater than a predetermined reference value, the control unit 103 may control the sleep assisting device to be turned off.

The control unit 103 may control the operation of the supply adjustment unit 102 based on the measurement value acquired through the carbon dioxide concentration detection unit.

For example, when the measurement value acquired through the carbon dioxide concentration detection unit is smaller than the predetermined reference value, the control unit 103 may control the supply adjustment unit 102 so that the supply adjustment unit 102 increases the carbon dioxide supply pressure.

For another example, when the measurement value acquired through the carbon dioxide concentration detection unit is greater than the predetermined reference value, the control unit 103 may control the supply adjustment unit 102 so that the supply adjustment unit 102 decreases the carbon dioxide supply pressure.

The carbon dioxide concentration detection unit may be present in a state of being physically coupled to the sleep assisting device. The carbon dioxide concentration detection unit may be configured to be detachable from the sleep assisting device 100. The carbon dioxide concentration detection unit may also be present in a state of being physically separated from the sleep assisting device. When the carbon dioxide concentration detection unit is present in the state of being physically separated from the sleep assisting device, the carbon dioxide concentration detection unit may be connected to the sleep assisting device through wireless communication.

4.3 Sleep assisting device according to second embodiment

4.3.1 Object of sleep assisting device according to second embodiment

As described above, the sleep assisting device according to the first embodiment is directed to supplying the carbon dioxide to the user to decrease the oxygen partial pressure in the user's body.

The sleep assisting device according to the second embodiment provided in 4.3 (Object of sleep assisting device according to second embodiment) is directed to supplying the carbon dioxide to the user to decrease the oxygen partial pressure in the user's body further in consideration of efficiency for the supply amount of the carbon dioxide.

The carbon dioxide may be lost by diffusion in the area between the injection port of the sleep assisting device and the user. In order to decrease the amount of carbon dioxide lost due to diffusion, a method of adjusting the distance between the injection port and the user's respiratory organ may be considered.

The distance between the injection port of the sleep assisting device and the user may affect the sleep inducing effect provided to the user. Accordingly, a sleep inducing device having an improved form may be considered so that the distance between the injection port and the user is freely adjusted according to the user's intention, and the injection port is fixed to the adjusted position so that the sleep inducing device may provide a constant sleep inducing environment.

The sleep assisting device according to the second embodiment is directed to decreasing the amount of carbon dioxide lost due to diffusion by providing a movement path of the carbon dioxide to the area between the injection port and the user.

The sleep assisting device may decrease the distance between the injection port and the user without changing the position of the sleep assisting device by providing the movement path of the carbon dioxide. Accordingly, the sleep assisting device may supply the carbon dioxide in the area around the user's respiratory organ.

Accordingly, the sleep assisting device according to the second embodiment is directed to enhancing the user convenience by providing the movement path in which the distance between the injection port and the user is adjusted by the user.

4.3.2 Configuration and operation of device according to second embodiment

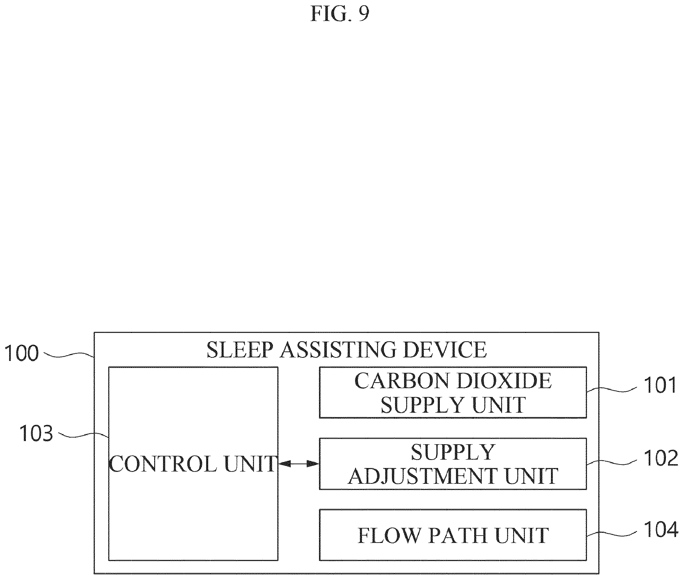

FIG. 9 is a block diagram showing the configuration of the sleep assisting device according to the second embodiment.

The sleep assisting device according to the second embodiment may supply carbon dioxide to the outside.

The sleep assisting device according to the second embodiment may provide a movement path of the carbon dioxide for supplying the carbon dioxide to the outside.

The sleep assisting device according to the second embodiment may supply the carbon dioxide so that the carbon dioxide concentration in an area positioned to be spaced apart from the injection port by a predetermined distance has the value within the numerical range that satisfies the safety and effectiveness described in 3 (Considerations upon supplying carbon dioxide for sleep assistance).

The sleep assisting device according to the second embodiment may supply the carbon dioxide so that a flow rate of a gas in the area positioned to be spaced apart from the injection port by the predetermined distance has the value within the numerical range that satisfies the user convenience and efficiency described in 3 (Considerations upon supplying carbon dioxide for sleep assistance).

Hereinafter, each configuration of the sleep assisting device according to the second embodiment will be described in detail with reference to FIG. 9.

However, since the detailed descriptions of the carbon dioxide supply unit 101, the supply adjustment unit 102, and the control unit 103 that are components common to the sleep assisting device according to the first embodiment may also be applied to the second embodiment, hereinafter, only additional items will be described.

Referring to FIG. 9, the sleep assisting device 100 according to the second embodiment may include a carbon dioxide supply unit 101, a supply adjustment unit 102, a flow path unit 104, and a control unit 103.

The carbon dioxide supply unit 101 may be connected to the flow path unit 104.

The carbon dioxide supply unit 101 may be connected to the flow path unit 104 and configured to supply the carbon dioxide to the flow path unit 104.

The supply adjustment unit 102 may adjust the carbon dioxide supply pressure supplied to the flow path unit 104 by the carbon dioxide supply unit 101. The supply adjustment unit 102 may increase or decrease the carbon dioxide supply pressure supplied to the flow path unit 104 by the carbon dioxide supply unit 101.

The flow path unit 104 may provide a path through which the carbon dioxide moves. The flow path unit 104 may be connected to the carbon dioxide supply unit 101 and configured to provide a path through which the carbon dioxide supplied by the carbon dioxide supply unit 101 moves.

A distal end of one side of the flow path unit 104 may include an injection port configured to supply the carbon dioxide to the outside. The flow path unit 104 may be connected to the carbon dioxide supply unit 101 and configured to provide the path through which the carbon dioxide supplied by the carbon dioxide supply unit 101 moves from the carbon dioxide supply unit 101 to the injection port.

The flow path unit 104 may have a predetermined cross-sectional area so that the flow rate of the carbon dioxide moving along the flow path unit 104 is determined. For example, the cross-sectional area of the flow path unit 104 may be configured to be a first area so that the flow rate of the carbon dioxide moving along the flow path is a first flow rate.

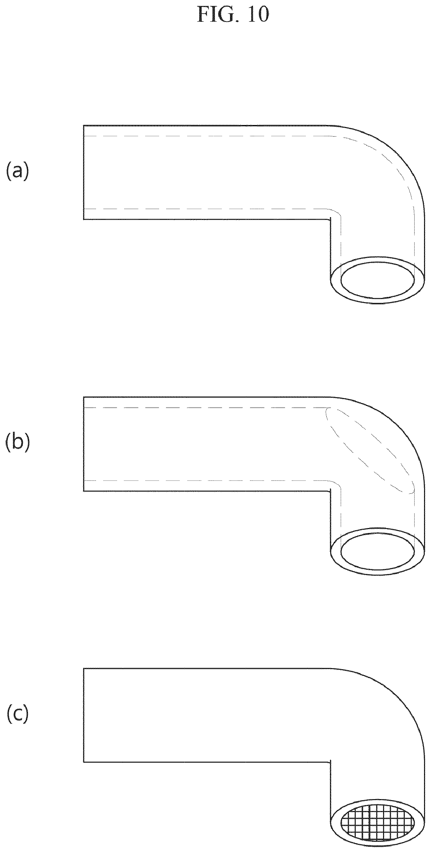

FIG. 10 is a view showing a specific embodiment of the injection port of the sleep assisting device according to the second embodiment.

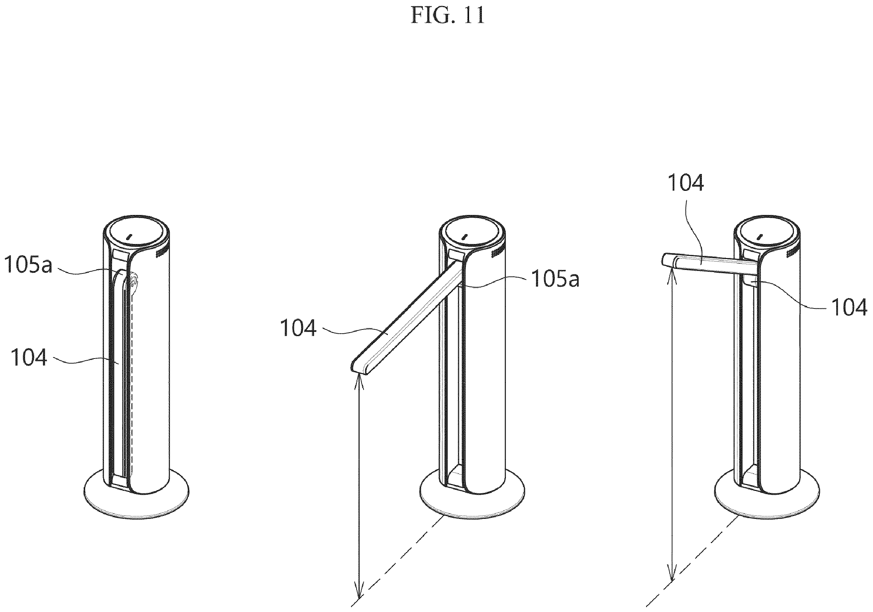

Referring to FIG. 10A, the flow path unit 104 may include a bent portion configured to change a moving direction of the mixed gas. For example, referring to FIG. 11A, a distal end of the flow path unit 104 may include the injection port configured to supply the carbon dioxide moving along the flow path unit 104 to the outside. At this time, a direction in which the carbon dioxide moves in the flow path unit 104 may form a predetermined angle with a direction in which the carbon dioxide is supplied from the injection port to the outside. For example, a constant angle may be 90 degrees.

Referring to FIG. 11B, the flow path unit 104 may include an assisting guide configured to assist a change in the moving direction of the mixed gas. The assisting guide may be positioned on the bent portion. For example, referring to FIG. 11B, an inner wall of the flow path unit 104 may be configured to have a shape different from a shape of an outer wall of the flow path unit 104 so that a resistance between a wall surface of the flow path unit 104 and the carbon dioxide is minimized at a point where the moving direction of the carbon dioxide moving along the flow path unit 104 is changed.

Referring to FIG. 11C, the flow path unit 104 may include an injection guide configured to uniformly inject the mixed gas. For example, referring to FIG. 7C, a distal end of the injection port according to one embodiment may include a net so that the gas is uniformly supplied.

Referring to FIG. 11A to 11C, the injection port may be configured so that the carbon dioxide is supplied to the outside downward.

An average density of the carbon dioxide gas is about 1.6 kg/m$^3$ at 0 °C and 1 atm, and an average density of the atmosphere is about 1.2 kg/m$^3$ at 0 °C and 1 atm. Accordingly, the carbon dioxide or a mixed gas having a greater carbon dioxide concentration than that of the atmosphere diffuses mainly downward in the atmosphere. Accordingly, it may be effective for sleep assistance that the injection port is positioned at a higher position than the position of the user's respiratory organ and configured to supply the carbon dioxide downward.

The flow path unit 104 may be configured as a pipe, a hose, or the like. The flow path unit 104 may be made of a material in which at least any one of a position, shape, and length of the flow path unit may be changed by the user.

The flow path unit 104 may be configured in a form in which at least any one of the position or length of the flow path unit 104 may be changed by the user. For example, the flow path unit 104 may be configured in a form in which an angle formed by a part of one side of the flow path unit 104 and a part of the other side of the flow path unit 104 with respect to any one point of the flow path unit 104 may be changed.

The flow path unit 104 may be configured so that the distance between the injection port and the target area (hereinafter referred to as a "target distance") may be adjusted. The flow path unit 104 may be configured so that the target distance may be adjusted according to an external force applied to the flow path unit 104.

According to the above-described embodiment, the flow path unit 104 may be configured in a form in which an angle formed by the part of one side of the flow path unit 104 and the part of the other side of the flow path unit 104 with respect to any one point may be changed. The angle formed by the part of one side of the flow path unit 104 and the part of the other side of the flow path unit 104 may be changed by an external force applied to the flow path unit 104, so that the position of the injection port or the distance between the injection port and the object may be adjusted.

In general, a user positioned in an area lower than the injection port may create a sleep assisting environment suitable for the individual user by adjusting the distance between the injection port and the user.

Hereinafter, a configuration for adjusting the position of the injection port or the distance between the injection port and the object will be described.

The sleep assisting device 100 according to the second embodiment may further include distance adjustment units 105a and 105b.

The distance adjustment units 105a and 105b may adjust the position of the injection port or the distance between the injection port and the object. The distance adjustment units 105a and 105b may adjust the distance between the injection port and the user. The distance adjustment units 105a and 105b may adjust the distance between the injection port and the user's respiratory organ.

The distance adjustment units 105a and 105b may be connected to the flow path unit 104. The distance adjustment units 105a and 105b may be connected to the flow path unit 104 and configured to adjust the position of the injection port or the distance between the injection port and the object. The distance adjustment units 105a and 105b may be connected to the distal end of the flow path unit, and connected between the plurality of flow path units and disposed so that the target distance may be adjusted.

For example, according to the above-described embodiment, when the flow path unit 104 is configured in the form in which the angle formed by the part of one side of the flow path unit 104 and the part of the other side of the flow path unit 104 with respect to any one point may be changed, the distance adjustment units 105a and 105b may be positioned at any one point.

The distance adjustment units 105a and 105b may adjust the target distance to be a predetermined distance. At this time, the predetermined distance may refer to a distance considering the user convenience and efficiency described in 3 (Considerations upon supplying carbon dioxide for sleep assistance). For example, the predetermined distance may be determined within a range of 20 to 30 [cm] in consideration of the user convenience and efficiency described in 3 (Considerations upon supplying carbon dioxide for sleep assistance). For another example, the predetermined distance may be determined within a range of 30 to 40 [cm] in consideration of the user convenience and efficiency described in 3 (Considerations upon supplying carbon dioxide for sleep assistance).

The distance adjustment units 105a and 105b may increase or decrease the distance between the injection port and the object. The distance adjustment units 105a and 105b may adjust the carbon dioxide concentration and flow rate of the user's intake by increasing or decreasing the distance between the injection port and the object.

The distance adjustment units 105a and 105b may manually or automatically adjust the distance between the injection port and the object. The distance adjustment units 105a and 105b may manually adjust the distance between the injection port and the object by the user's external force.

The distance adjustment units 105a and 105b may automatically adjust the distance between the injection port and the object based on an externally acquired signal. At this time, the signal may be a signal acquired from another unit included in the sleep assisting device 100. At this time, the signal may be a signal acquired from the user through an interface included in the sleep assisting device 100. At this time, the signal may be a signal acquired from a separate device. For example, the separate device may be a terminal used by the user.

FIG. 11 is a view showing a sleep assisting device including one distance adjustment unit according to one embodiment.

Referring to FIG. 11, the sleep assisting device 100 according to the second embodiment may include one distance adjustment unit 105a.

The distance adjustment unit 105a may adjust the distance between the injection port and the object. The distance adjustment unit 105a may be connected to the flow path unit and configured to adjust the distance between the injection port and the object by changing at least one of the position, shape, and length of the flow path unit. When the distance between the injection port and the object is changed by the distance adjustment unit 105a, the position of the target area may be changed.

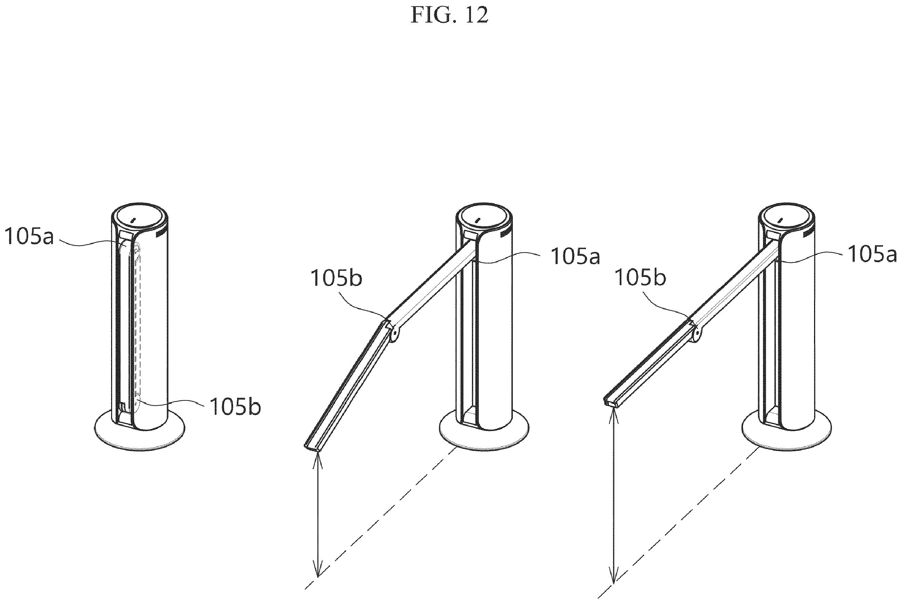

FIG. 12 is a view showing a sleep assisting device including two distance adjustment units according to one embodiment.

The sleep assisting device according to the second embodiment may include a plurality of distance adjustment units 105a and 105b. The plurality of distance adjustment units 105a may adjust the distance between the injection port and the object. The distance adjustment units 105a and 105b may be connected to the flow path unit 104. Specifically, one distance adjustment unit 105a may be connected to the distal end of the flow path unit 104, and the other distance adjustment unit 105b may be connected to a center of the flow path unit 104.

The distance adjustment units 105a and 105b may adjust the distance between the injection port and the object by changing at least one of the position, shape, and length of the flow path unit 104.

Each distance adjustment unit included in the plurality of distance adjustment units 105a and 105b may independently adjust the distance between the injection port and the object. In addition, the plurality of distance adjustment units 105a and 105b may adjust the distance between the injection port and the object in a dependent manner.

The plurality of distance adjustment units 105a and 105b are not limited to two distance adjustment units, and may also include three or more distance adjustment units.

The distance adjustment units 105a and 105b may include a hinge and a motor. However, the distance adjustment units 105a and 105b are not limited to the above-described configuration, and may refer to all devices capable of adjusting the position of the injection port.

The control unit 103 may control operations of the distance adjustment units 105a and 105b. The control unit 103 may acquire a signal from the outside, and control the operations of the distance adjustment units 105a and 105b based on the acquired signal. At this time, the signal may be a signal acquired from a user interface included in the sleep assisting device 100. In addition, the signal may be a signal acquired from each unit included in the sleep assisting device 100. In addition, the signal may be a signal acquired from an external device.

The control unit 103 may control the distance adjustment units 105a and 105b so that the distance adjustment units 105a and 105b adjust the distance between the injection port and the target area.

The sleep assisting device according to the second embodiment may include a distance detection unit (not shown).

The distance detection unit may acquire information on a distance between the distance detection unit and the object. The distance detection unit may acquire information on a distance between the distance detection unit and the user. The distance detection unit may acquire a distance between the injection port and the user based on the acquired distance information. In addition, the distance detection unit may also acquire a distance between the injection port and the target area based on the acquired distance information.

The distance detection unit may acquire information on a state of the unit of the sleep assisting device 100 related to the distance between the injection port and the object. The distance detection unit may acquire information on the states of the distance adjustment units 105a and 105b to acquire information on the distance between the injection port and the object.

When the distance adjustment units 105a and 105b are configured in the form of a hinge, the states of the distance adjustment units 105a and 105b may include information on bent states of the hinges, for example, angles formed by the hinges.

For example, when the angle formed by the hinges of the distance adjustment units 105a and 105b is a first angle, the distance detection unit may acquire state information of the distance adjustment units 105a and 105b corresponding to the first angle.

The state information of the distance adjustment units 105a and 105b may be treated similarly to a value related to the distance between the injection port and the object or the distance between the injection port and the object.

For another example, when the angle formed by the hinges of the distance adjustment units 105a and 105b is a second angle greater than the first angle, the distance detection unit may acquire state information of the distance adjustment units 105a and 105b corresponding to the second angle.

When the distance adjustment units 105a and 105b are configured in the form of a motor, the states of the distance adjustment units 105a and 105b may include information on a rotated level.

For example, when the motors of the distance adjustment units 105a and 105b are rotated by the first angle, the distance detection unit may acquire the state information of the distance adjustment units 105a and 105b corresponding to the first angle.

For another example, when the motors of the distance adjustment units 105a and 105b are rotated by the second angle greater than the first angle, the distance detection unit may acquire the state information of the distance adjustment units 105a and 105b corresponding to the second angle.

In addition, the states of the distance adjustment units 105a and 105b may include information on the position of the flow path unit 104 using the distance adjustment units 105a and 105b as a reference position.

For example, when the angle formed by the part of the flow path unit 104 and the other part of the flow path unit 104 with respect to the distance adjustment units 105a and 105b is the first angle, the distance detection unit may acquire the state information of the distance adjustment units 105a and 105b corresponding to the first angle.

For another example, when the angle formed by the part of the flow path unit 104 and the other part of the flow path unit 104 with respect to the distance adjustment units 105a and 105b is the second angle greater than the first angle, the distance detection unit may acquire the state information of the distance adjustment units 105a and 105b corresponding to the first angle.

The distance detection unit may be connected to the flow path unit 104 and configured to acquire the information on the distance between the injection port and the user. Specifically, the distance detection unit may be connected to the distal end of the flow path unit 104 including the injection port and configured to acquire the information on the distance between the injection port and the user.

The distance detection unit may acquire the information on the distance between the distance detection unit and the user, and acquire a distance measurement value based on the acquired information.

The control unit 103 may control the operations of the sleep assisting device 100 and/or each unit included in the sleep assisting device 100 based on the measurement value acquired through the distance detection unit. The control unit 103 may generate a control signal based on the measurement value acquired through the distance detection unit, and control each unit of the device based on the control signal.

The control unit 103 may control a power source of the sleep assisting device based on the measurement value acquired through the distance detection unit.

For example, when the measurement value acquired through the distance detection unit is smaller than a predetermined reference value, the control unit 103 may control the sleep assisting device to be turned off.

For another example, when the measurement value acquired through the distance detection unit falls within a range of the predetermined reference value, the control unit 103 may control the sleep assisting device to be turned on.

Hereinafter, a method of controlling a power source of the sleep assisting device based on the value related to the distance between the injection port and the object by the control unit will be described in detail with reference to FIG. 11 through a specific embodiment.

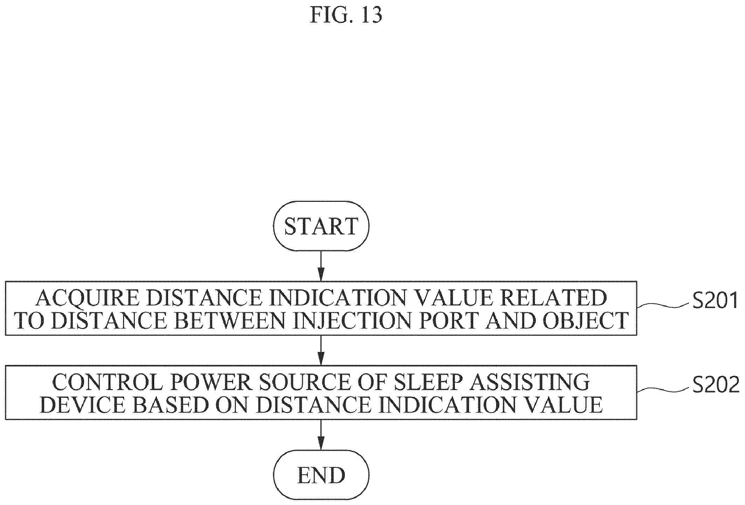

FIG. 13 is a flowchart showing the method of controlling the power source of the sleep assisting device based on a distance indication value related to the distance between the injection port and the object.

Referring to FIG. 13, the method of controlling the power source of the sleep assisting device based on the distance indication value may include an operation of acquiring a distance indication value related to the distance between the injection port and the object (S201), and an operation of controlling the power source of the sleep assisting device based on the distance indication value (S202).

In the operation of acquiring the distance indication value related to the distance between the injection port and the object (S201), the sleep assisting device 100 may acquire the distance indication value related to the distance between the injection port and the object from the distance detection unit or the outside.

In this specification, the distance indication value may refer to a value related to the distance between the injection port and the object acquired from the distance detection unit.

In addition, the distance indication value may refer to a value related to the distance between the injection port and the object acquired from the outside.

In this specification, the distance indication value may have a positive correlation with the distance between the injection port and the object. The distance indication value may have a proportional relationship with the distance between the injection port and the object.

In addition, the distance indication value may have a negative correlation with the distance between the injection port and the object. The distance indication value may have an inversely proportional relationship with the distance between the injection port and the object.

In addition, the distance indication value may refer to a value related to a distance, speed, or time measured by a sensor. The sensor may include any one of a distance sensor, an image sensor, and an optical sensor.

In the operation of controlling the power source of the sleep assisting device based on the distance indication value (S202), the sleep assisting device 100 may be controlled so that the power source of the sleep assisting device is turned on or off based on the acquired distance indication value.

According to one embodiment, when the distance indication value is greater than a first reference value, the sleep assisting device 100 may be controlled so that the power source of the sleep assisting device 100 is turned off.

For example, when the distance indication value acquired through the distance detection unit is greater than the first reference value, the sleep assisting device 100 may be controlled so that the power source of the sleep assisting device 100 is turned off.

For another example, when the distance indication value acquired through the distance detection unit indicates that the distance between the injection port and the object is greater than the first reference distance, the sleep assisting device 100 may be controlled so that the power source of the sleep assisting device is turned off.

According to another embodiment, when the distance indication value is smaller than the first reference value, the sleep assisting device 100 may be controlled so that the power source of the sleep assisting device is turned off.

For example, when the distance indication value acquired through the distance detection unit is smaller than the first reference value, the sleep assisting device 100 may be controlled so that the power source of the sleep assisting device is turned off.

For another example, when the distance indication value acquired through the distance detection unit indicates that the distance between the injection port and the object is smaller than the first reference distance, the sleep assisting device 100 may be controlled so that the power source of the sleep assisting device is turned off.

The control unit 103 may control the operation of the supply adjustment unit 102 based on the measurement value acquired through the distance detection unit.

According to one embodiment, the value acquired through the distance detection unit may be a value having the positive correlation with the distance between the injection port and the object. The value acquired through the distance detection unit may be a value that increases when the distance between the injection port and the object increases.

At this time, when the measurement value acquired through the distance detection unit is greater than a predetermined reference value (or an existing measurement value), the control unit 103 may control the supply adjustment unit 102 to increase the carbon dioxide supply pressure. When the measurement value acquired through the distance detection unit is greater than the predetermined reference value (or the existing measurement value), the distance to the object is regarded as being increased (or greater than or equal to the reference distance), and the control unit 103 may control the supply adjustment unit 102 to increase the carbon dioxide supply pressure.

For another example, when the measurement value acquired through the distance detection unit is smaller than the predetermined reference value (or the existing measurement value), the control unit 103 may control the supply adjustment unit 102 to decrease the carbon dioxide supply pressure. When the measurement value acquired through the distance detection unit is smaller than the reference value (or the existing measurement value), the distance to the object is regarded as being decreased (or smaller than or equal to the reference distance), and the control unit 103 may control the supply adjustment unit 102 to decrease the carbon dioxide supply pressure.

The control unit 103 may also control the supply adjustment unit 102 so that the supply adjustment unit 102 stops the supply of the carbon dioxide by decreasing the supply pressure to zero.

For another example, when the measurement value acquired through the distance detection unit is within a predetermined range, the control unit 103 may control the supply adjustment unit 102 to start the supply of the carbon dioxide. The control unit 103 may acquire a first value that does not fall within the predetermined range and then acquire a second value that falls within the predetermined range through the distance detection unit, and control the supply adjustment unit 102 to start the supply of the carbon dioxide.

At this time, when the measurement value acquired through the distance detection unit is out of the predetermined reference range, the control unit 103 may control the supply adjustment unit 102 so that the supply adjustment unit 102 decreases the carbon dioxide supply pressure. The control unit 103 may acquire the first value that falls within the predetermined range and then acquire the second value that does not fall within the predetermined range through the distance detection unit, and control the supply adjustment unit 102 to decrease (or stop) the supply of the carbon dioxide. At this time, the control unit 103 may also control the supply adjustment unit 102 so that the supply adjustment unit 102 stops the supply of the carbon dioxide by decreasing the supply pressure to zero.

According to another embodiment, the value acquired through the distance detection unit may be a value having the negative correlation with the distance between the injection port and the object. The value acquired through the distance detection unit may be a value that decreases when the distance between the injection port and the object increases.

At this time, when the measurement value acquired through the distance detection unit is greater than the predetermined reference value (or the existing measurement value), the control unit 103 may control the supply adjustment unit 102 to decrease the carbon dioxide supply pressure. When the measurement value acquired through the distance detection unit is greater than the predetermined reference value (or the existing measurement value), the distance to the object is regarded as being decreased (or smaller than or equal to the reference distance), and the control unit 103 may control the supply adjustment unit 102 to decrease the carbon dioxide supply pressure.

For another example, when the measurement value acquired through the distance detection unit is smaller than the predetermined reference value (or the existing measurement value), the control unit 103 may control the supply adjustment unit 102 to increase the carbon dioxide supply pressure. When the measurement value acquired through the distance detection unit is smaller than the reference value (or the existing measurement value), the distance to the object is regarded as being increased (or farther than the reference distance), and the control unit 103 may control the supply adjustment unit 102 to increase the carbon dioxide supply pressure.

The control unit 103 may also control the supply adjustment unit 102 so that the supply adjustment unit 102 stops the supply of the carbon dioxide by decreasing the supply pressure to zero.

For another example, when the measurement value acquired through the distance detection unit is within a predetermined range, the control unit 103 may control the supply adjustment unit 102 to start the supply of the carbon dioxide. The control unit 103 may acquire the first value that does not fall within the predetermined range and then acquire the second value that falls within the predetermined range through the distance detection unit, and control the supply adjustment unit 102 to start the supply of the carbon dioxide.

At this time, when the measurement value acquired through the distance detection unit is out of the range of the predetermined reference value, the control unit 103 may control the supply adjustment unit 102 so that the supply adjustment unit 102 decreases the carbon dioxide supply pressure. The control unit 103 may acquire the first value that falls within the predetermined range and then acquire the second value that does not fall within the predetermined range through the distance detection unit, and control the supply adjustment unit 102 to decrease (or stop) the supply of the carbon dioxide. At this time, the control unit 103 may also control the supply adjustment unit 102 so that the supply adjustment unit 102 stops the supply of the carbon dioxide by decreasing the supply pressure to zero.

The control unit 103 may control the distance adjustment unit based on the measurement value acquired through the distance detection unit. The control unit 103 may change the position of the injection port by controlling the distance adjustment unit based on the measurement value acquired through the distance detection unit. For example, when the measurement value acquired through the distance detection unit is smaller than the predetermined reference value, the control unit 103 may control the distance adjustment units 105a and 105b so that the distance adjustment units 105a and 105b increase the distance between the injection port and the object.

For another example, when the measurement value acquired through the distance detection unit is greater than the predetermined reference value, the control unit 103 may control the distance adjustment units 105a and 105b so that the distance adjustment units 105a and 105b decrease the distance between the injection port and the object.

The control unit 103 may control the operations of the distance adjustment units 105a and 105b based on the measurement value acquired through the carbon dioxide concentration detection unit.

For example, when the measurement value acquired through the carbon dioxide concentration detection unit is smaller than the predetermined reference value, the control unit 103 may control the distance adjustment units 105a and 105b so that the distance adjustment units 105a and 105b decrease the distance between the injection port and the target area.

For another example, when the measurement value acquired through the carbon dioxide concentration detection unit is greater than the predetermined reference value, the control unit 103 may control the distance adjustment units 105a and 105b so that the distance adjustment units 105a and 105b increase the distance between the injection port and the target area.

The distance detection unit may be configured as a device using ultrasonic waves, microwaves, infrared rays, or visible rays. For example, the distance detection unit may be configured as a radar or a LiDAR. For another example, the distance detection unit may be configured as a camera.

However, the type of the distance detection unit is not limited to the described embodiment, and may refer to all devices capable of measuring the distance between the sensor and the object.

The distance detection unit may be physically coupled to the sleep assisting device. However, the distance detection unit may also be present in a state of being physically separated from the sleep assisting device. When the distance detection unit is present in the state of being physically separated from the sleep assisting device, the distance detection unit may be connected to the sleep assisting device by wire or wirelessly.

4.4 Sleep assisting device according to third embodiment

4.4.1 Object of sleep assisting device according to third embodiment

The sleep assisting device according to the second embodiment is directed to further providing the movement path through which the carbon dioxide supplied from the carbon dioxide supply unit moves. Accordingly, the sleep assisting device may be provided so that the position in the area where the carbon dioxide is supplied or the area where the sleep inducing environment is created by the gas containing the carbon dioxide may be adjusted.

In addition, the sleep assisting device according to the second embodiment is directed to providing the movement path having an adjustable distance between the injection port and the user. Accordingly, the position of the injection port, the distance between the injection port and the user, or the position of the target area may be adjusted based on the user, a control signal of the control unit, and the like.

As a result, not only the amount of carbon dioxide lost due to diffusion is decreased but also the user convenience is improved, but there may be a problem in that the amount of carbon dioxide reaching the target area is insufficient in terms of efficiency compared to the supply amount of carbon dioxide.

In order to solve this problem, the sleep assisting device according to the third embodiment may be configured to provide power to the supplied carbon dioxide. For example, the sleep assisting device according to the third embodiment may further include a fan as a device for providing the power to the carbon dioxide.

The fan may introduce outside air. The fan may introduce the outside air to provide the outside air to the movement path of the carbon dioxide or the mixed gas. At this time, the introduced outside air is mixed with the carbon dioxide to form the mixed gas, and the mixed gas may move with directionality.

The fan may also introduce a constant amount of outside air per unit time at a constant rotation speed. The sleep assisting device according to the third embodiment may be designed in consideration of safety, efficiency, and user experience based on an experiment on (the rotation speed of the fan-the carbon dioxide concentration).

4.4.2 Configuration and operation of sleep assisting device according to third embodiment

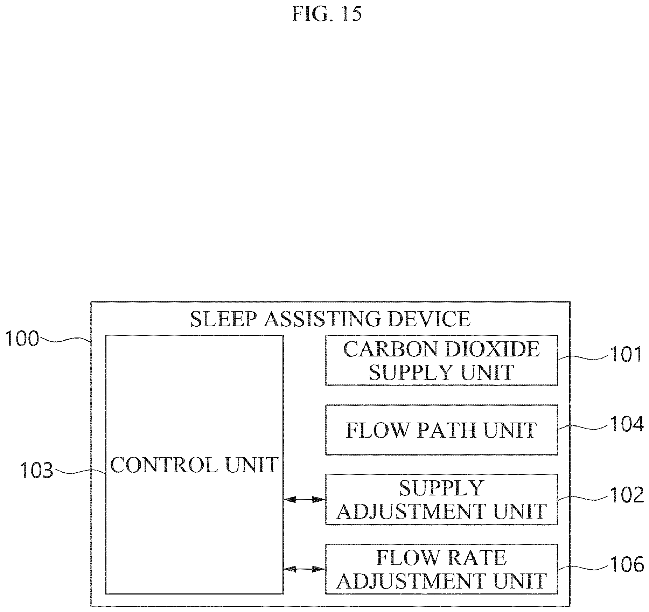

FIG. 15 is a block diagram showing the configuration of the sleep assisting device according to the third embodiment.

Hereinafter, each configuration of the sleep assisting device according to the third embodiment will be described in detail with reference to FIG. 15.

However, since the detailed descriptions of the carbon dioxide supply unit, the supply adjustment unit, the flow path unit, and the control unit, which are components common to the first and second embodiments, may also be commonly applied to the third embodiment, hereinafter, only additional items will be described below.

The sleep assisting device according to the third embodiment may include a carbon dioxide supply unit, a supply adjustment unit, a flow path unit, a flow rate adjustment unit, and a control unit.

The flow path unit 104 may provide a path through which the mixed gas moves. The mixed gas moving along the flow path unit 104 may be supplied to the outside through the injection port.

A flow rate adjustment unit 106 may adjust a flow rate of the mixed gas supplied through the injection port. Specifically, the flow rate adjustment unit 106 may increase or decrease the flow rate of the carbon dioxide or mixed gas supplied through the injection port.

The flow rate of the mixed gas used in this specification may refer to a flow rate of the mixed gas at the injection port. The flow rate of the mixed gas may refer to a flow rate of the mixed gas in a space connected to the injection port. The flow rate of the mixed gas may refer to a flow rate of the mixed gas in the flow path unit 102.

The flow rate of the mixed gas used in this specification may refer to a flow rate of the mixed gas when the state of the mixed gas is a steady state. The flow rate of the mixed gas may refer to a flow rate of the mixed gas at a time point when a predetermined time elapses after the sleep assisting device 100 controls each unit to perform the sleep assistance or the induction operation.

In this specification, the steady state may refer to a state in which the physical amount (e.g., the flow rate or the carbon dioxide concentration) of the mixed gas in which gases having different composition ratios are mixed is constantly (substantially) maintained as the same value without being changed with time and space. The steady state may refer to a state in which a level of change in the physical amount (e.g., the flow rate or the carbon dioxide concentration) of the mixed gas with time and space is a certain level or less and thus is negligibly small. For example, the steady state may refer to a state in which the flow rate and/or carbon dioxide concentration of the mixed gas in which the atmosphere and the carbon dioxide are mixed are maintained as the same values without being changed with time and space.

In this specification, the steady state may refer to a state of the mixed gas at a time point when a predetermined time elapses after the gases having different composition ratios are mixed. For example, the steady state may refer to a state of the mixed gas at the time point when the predetermined time elapses after the atmosphere and the carbon dioxide are mixed.

In this specification, the steady state may refer to a state of the mixed gas at a time point when a predetermined time elapses after a time point when the operation of each unit of the sleep assisting device 100 operated to mix the gases having different composition ratios has started. For example, the steady state may refer to a state of the gas in which the carbon dioxide and the outside air are mixed at the time point when the predetermined time elapses after the time point when the operation of the supply adjustment unit 102 operated to supply the carbon dioxide and the flow rate adjustment unit 106 operated to introduce the outside air have started.

In this specification, the steady state may refer to a state other than a transient state.

In this specification, the transient state may refer to a state in which the physical amount (e.g., the flow rate or the carbon dioxide concentration) of the gas in the steady state is changed with time and space due to an external factor. The transient state may refer to a state in which a level of change in the physical amount (e.g., the flow rate or the carbon dioxide concentration) of the gas due to the external factor is greater than or equal to a predetermined level or a significant level.

In this specification, the transient state may refer to a state at a time point when a predetermined time elapses after the time point when the mixed gas has reached the steady state. For example, the transient state may refer to a state of the mixed gas at the time point when the predetermined time elapses after the time point when the mixed gas has reached the steady state.

The physical amount of the gas used in this specification may refer to a physical amount in which all are in the steady state unless otherwise stated. For example, the carbon dioxide concentration of the mixed gas may refer to a carbon dioxide concentration of the mixed gas in the steady state. For another example, the flow rate of the mixed gas may not refer to a flow rate of the mixed gas in the steady state.

At this time, the mixed gas may refer to a gas in which the carbon dioxide supplied by the carbon dioxide supply unit 101 and the outside air introduced by the flow rate adjustment unit 106 are mixed. Alternatively, the mixed gas may also refer to a gas in which the carbon dioxide supplied by the carbon dioxide supply unit 101 and other gases are mixed in advance.

The flow rate adjustment unit 106 may introduce the outside air into the flow path unit 104. At this time, the introduced outside air may be mixed with the carbon dioxide supplied by the carbon dioxide supply unit 101.

The flow rate adjustment unit 106 may adjust the carbon dioxide concentration of the mixed gas supplied through the injection port by introducing the outside air into the flow path unit 104. The flow rate adjustment unit 106 may decrease the carbon dioxide concentration of the mixed gas supplied through the injection port by increasing the amount of the outside air introduced into the flow path unit 104. The flow rate adjustment unit 106 may increase the carbon dioxide concentration of the mixed gas supplied through the injection port by decreasing the amount of the outside air introduced into the flow path unit 104.

The carbon dioxide concentration of the mixed gas used in this specification may refer to a carbon dioxide concentration of the mixed gas at the injection port. The carbon dioxide concentration of the mixed gas may refer to a carbon dioxide concentration of the mixed gas in the space connected to the injection port. The carbon dioxide concentration of the mixed gas may refer to a carbon dioxide concentration of the mixed gas in the flow path unit 102.

The carbon dioxide concentration of the mixed gas used in this specification may refer to a carbon dioxide concentration of the mixed gas when the state of the mixed gas is the steady state. The carbon dioxide concentration of the mixed gas may refer to a carbon dioxide concentration of the mixed gas at a time point when a predetermined time elapses after the sleep assisting device 100 controls each unit to perform the sleep assisting operation.

The control unit 103 may be connected to the flow rate adjustment unit 106 by wire or wirelessly.

The control unit 103 may control the flow rate adjustment unit 106 so that the flow rate adjustment unit 106 adjusts the flow rate of the mixed gas at the injection port. The control unit 103 may control the flow rate adjustment unit 106 so that the flow rate adjustment unit 106 increases or decreases the flow rate of the mixed gas at the injection port.

The control unit 103 may control the flow rate adjustment unit 106 so that the flow rate adjustment unit 106 introduces a predetermined outside air into the inside of the sleep assisting device. The control unit 103 may control the flow rate adjustment unit 106 so that the flow rate adjustment unit 106 increases or decreases the amount of the outside air introduced per unit time.

The control unit 106 may control the flow rate adjustment unit 106 based on the measurement value acquired through the distance detection unit.

For example, when the measurement value acquired through the distance detection unit is greater than a predetermined reference value, the control unit 103 may control the flow rate adjustment unit 106 so that the flow rate adjustment unit increases or decreases the flow rate of the carbon dioxide or the mixed gas at the injection port.

For another example, when the measurement value acquired through the distance detection unit is smaller than the predetermined reference value, the control unit 103 may control the flow rate adjustment unit 106 so that the flow rate adjustment unit increases or decreases the flow rate of the carbon dioxide or the mixed gas at the injection port.

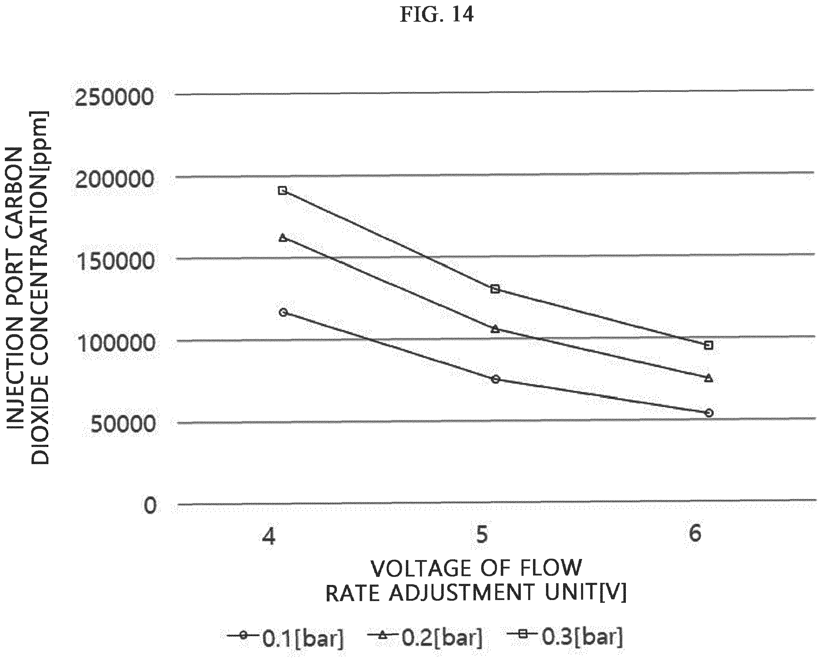

Hereinafter, a method of adjusting the supply adjustment unit 102 and the flow rate adjustment unit 106 based on the value related to the distance between the injection port and the object by the control unit will be described in more detail with reference to FIG. 14.

FIG. 14 is a flowchart showing the method of controlling the supply adjustment unit and the flow rate adjustment unit based on the distance indication value related to the distance between the injection port and the object.

Referring to FIG. 14, the method of controlling the supply adjustment unit of the sleep assisting device based on the distance indication value may include an operation of acquiring a distance indication value related to the distance between the injection port and the object (S301), and an operation of controlling the supply adjustment unit and the flow rate adjustment unit based on the distance indication value (S302).

In the operation of acquiring the distance indication value related to the distance between the injection port and the object (S301), the sleep assisting device 100 may acquire the distance indication value related to the distance between the injection port and the object from the distance detection unit or the outside.

The operation of acquiring the distance indication value related to the distance between the injection port and the object (S201) may be applied to the operation of acquiring the distance indication value related to the distance between the injection port and the object (S301) in the same manner.

In addition, a detailed description of the distance indication value may refer to 4.3.2 (Configuration and operation of device according to second embodiment).

In the operation of controlling the supply adjustment unit and the flow rate adjustment unit based on the distance indication value (S302), the sleep assisting device 100 may control the supply adjustment unit and the flow rate adjustment unit based on the distance indication value.

The sleep assisting device 100 may control the supply adjustment unit 102 based on the distance indication value.

As described above, the distance indication value may have a positive correlation or a negative correlation with the distance between the injection port and the object.

Hereinafter, an embodiment of a case in which the distance indication value has the negative or positive correlation with the distance between the injection port and the object will be described in detail.

According to one embodiment, when the distance indication value is greater than a first reference value and smaller than a second reference value, the sleep assisting device may control the supply adjustment unit 102 to supply the carbon dioxide.

When the distance indication value is smaller than the first reference value or greater than the second reference value, the sleep assisting device 100 may control the supply adjustment unit 102 so as not to supply the carbon dioxide. In addition, when the distance indication value is smaller than the first reference value or greater than the second reference value, the sleep assisting device 100 may control the supply adjustment unit 102 to stop the supply of the carbon dioxide.

When the first value is acquired as the distance indication value, the sleep assisting device 100 may control the supply adjustment unit 102 to supply the carbon dioxide at a first supply pressure. In addition, when the second value greater than the first value is acquired as the distance indication value, the sleep assisting device 100 may control the supply adjustment unit 102 to supply the carbon dioxide at a second supply pressure greater than the first supply pressure.

The sleep assisting device 100 may control the flow rate adjustment unit 106 based on the distance indication value.

Hereinafter, an embodiment of a case in which the distance indication value has the positive correlation with the distance between the injection port and the object will be described in detail. According to one embodiment, when the distance indication value is greater than the first reference value and smaller than the second reference value, the sleep assisting device may control the flow rate adjustment unit 106 to introduce the outside air into the flow path unit 104.

According to another embodiment, when the distance indication value is smaller than the first reference value or greater than the second reference value, the sleep assisting device 100 may control the flow rate adjustment unit 106 so as not to introduce the outside air into the flow path unit 104. In addition, when the distance indication value is smaller than the first reference value or greater than the second reference value, the sleep assisting device 100 may control the flow rate adjustment unit 106 to stop the introduction of the outside air.

Next, hereinafter, an embodiment of a case in which the distance indication value has the negative correlation with the distance between the injection port and the object will be described in detail.

According to one embodiment, when the distance indication value is smaller than the first reference value or greater than the second reference value, the sleep assisting device may control the flow rate adjustment unit 106 to introduce the outside air into the flow path unit 104.

According to another embodiment, when the distance indication value is greater than the first reference value and smaller than the second reference value, the sleep assisting device 100 may control the flow rate adjustment unit 106 so as not to introduce the outside air into the flow path unit 104. In addition, when the distance indication value is greater than the first reference value and smaller than the second reference value, the sleep assisting device 100 may control the flow rate adjustment unit 106 to stop the introduction of the outside air.

According to another embodiment, when the first value is acquired as the distance indication value, the sleep assisting device 100 may control the flow rate adjustment unit 106 to supply the outside air to the flow path unit 104 by a first flow rate. In addition, when the second value greater than the first value is acquired as the distance indication value, the sleep assisting device 100 may control the flow rate adjustment unit 106 to introduce the outside air into the flow path unit 104 by a second flow rate greater than the first flow rate.

The control unit 106 may control the flow rate adjustment unit 106 based on the measurement value acquired through the carbon dioxide concentration detection unit.

For example, when the measurement value acquired through the carbon dioxide concentration detection unit is greater than the predetermined reference value, the control unit 103 may control the flow rate adjustment unit 106 so that the flow rate adjustment unit increases or decreases the flow rate of the carbon dioxide or the mixed gas at the injection port.

For another example, when the measurement value acquired through the carbon dioxide concentration detection unit is smaller than the predetermined reference value, the control unit 103 may control the flow rate adjustment unit 106 so that the flow rate adjustment unit increases or decreases the flow rate of the carbon dioxide or the mixed gas at the injection port.

The sleep assisting device according to the third embodiment may further include a flow rate detection unit (not shown).

The flow rate detection unit may acquire information on the flow rate of the mixed gas at the injection port. In addition, the flow rate detection unit may also acquire information on the flow rate of the mixed gas moving in the flow path unit 104. In addition, the flow rate detection unit may acquire the flow rate of the carbon dioxide or the mixed gas in the target area.

The flow rate detection unit may acquire the information on the flow rate of the mixed gas, and acquire a flow rate measurement value based on the acquired information.

The flow rate detection unit may be configured as at least any one of a differential pressure flowmeter, an area flowmeter, an ultrasonic flowmeter, a turbine flowmeter, a volumetric flowmeter, an eddy flowmeter, an orifice flowmeter, a vortex flowmeter, a thermal mass flowmeter, and a mass flowmeter. However, the embodiment of the flow rate detection unit is not limited to the above-described flow meters, and may be configured as all devices capable of detecting or measuring the flow rate of liquid and gas per unit time.

The control unit 103 may control the flow rate adjustment unit 106 based on the measurement value acquired through the flow rate detection unit. The measurement value below may be a value that increase as the flow rate at a measurement position increases.

For example, when the measurement value acquired through the flow rate detection unit is smaller than the predetermined reference value, the control unit 103 may control the flow rate adjustment unit 106 so that the flow rate adjustment unit 106 increases the flow rate of the carbon dioxide or the mixed gas.

For another example, when the measurement value acquired through the flow rate detection unit is greater than the predetermined reference value, the control unit 103 may control the flow rate adjustment unit 106 so that the flow rate adjustment unit 106 decreases the flow rate of the carbon dioxide or the mixed gas.

The control unit 103 may control the supply adjustment unit and the flow rate adjustment unit to adjust the flow rate and the carbon dioxide concentration at the injection port. The control unit 103 may control the supply adjustment unit 102 and/or the flow rate adjustment unit 106 to adjust the flow rate and the carbon dioxide concentration at the injection port based on the user input.

The control unit 103 may control the supply adjustment unit 102 so that the carbon dioxide concentration at the injection port has the value within the numerical range that satisfies 3.1 (Safety) and 3.2 (Effectiveness), which will be described below. In addition, the control unit 103 may control the flow rate adjustment unit 106 so that the flow rate at the injection port has the value within the numerical range that satisfies 3.3 (User convenience) and 3.4 (Efficiency), which will be described below.

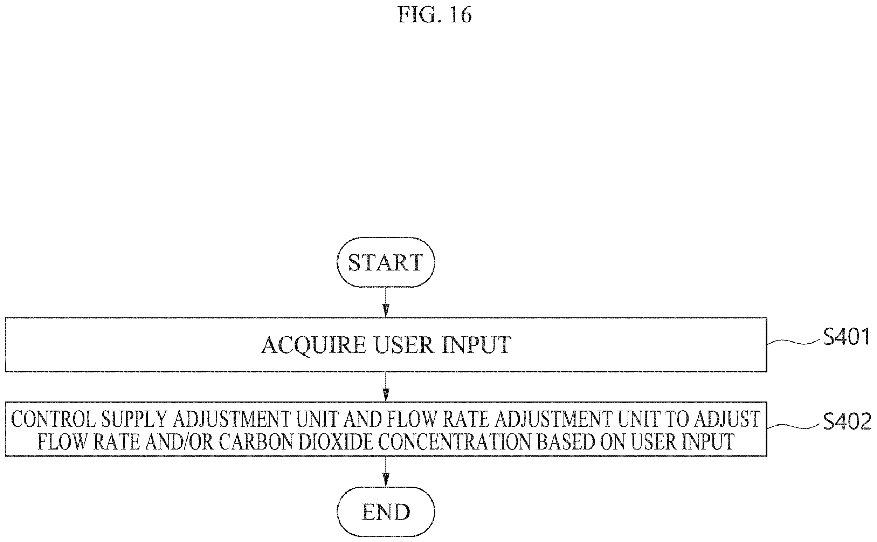

Hereinafter, a method of controlling the supply adjustment unit and the flow rate adjustment unit to adjust the flow rate and/or the carbon dioxide concentration based on the user input will be described with reference to FIG. 16 through a specific embodiment.

FIG. 16 is a flowchart showing the method of controlling the supply adjustment unit and the flow rate adjustment unit to adjust the flow rate and/or the carbon dioxide concentration based on the user input.

Referring to FIG. 16, the method of controlling the supply adjustment unit and the flow rate adjustment unit to adjust the flow rate and/or the carbon dioxide concentration based on the user input may include an operation of acquiring the user input (S401), and an operation of controlling the supply adjustment unit and the flow rate adjustment unit to adjust the flow rate and/or the carbon dioxide concentration based on the user input (S402).

In the operation of acquiring the user input (S401), the sleep assisting device 100 may acquire the user input from a component included in the sleep assisting device 100 or an external device.

The user input may include a command for instructing each of the carbon dioxide concentration and/or the flow rate at the injection port to have a predetermined value.

In addition, the user input may include a command for instructing each of the carbon dioxide concentration and/or the flow rate in the target area to have a predetermined value.

In addition, the user input may include a command for instructing each of the carbon dioxide supply pressure and/or the voltage applied to the flow rate adjustment unit to have a predetermined value.

The sleep assisting device 100 may acquire the user input from the configuration included in the sleep assisting device 100. For example, the sleep assisting device 10 may be acquired from a user interface (e.g., a touch screen or a button) included in the sleep assisting device 100.

The sleep assisting device 100 may acquire the user input from the external device. The sleep assisting device 100 may acquire the user input from the external device connected to the sleep assisting device 100 through wireless or wired communication. For example, the external device may include a user terminal, a tablet PC, a PC, a server, or the like.

In the operation of controlling the supply adjustment unit and/or the flow rate adjustment unit to adjust the flow rate and/or the carbon dioxide concentration based on the user input (S402), the sleep assisting device 100 may control the supply adjustment unit 102 and/or the flow rate adjustment unit 106 based on the user input so that each of the flow rate and/or the carbon dioxide concentration has the predetermined value.

In the operation of controlling the supply adjustment unit and/or the flow rate adjustment unit to adjust the flow rate and/or the carbon dioxide concentration based on the user input (S402), the sleep assisting device may control the supply adjustment unit 102 and/or the flow rate adjustment unit 106 so that each of the flow rate and/or the carbon dioxide concentration at the injection port has the predetermined value.

The sleep assisting device may control the supply adjustment unit 102 and/or the flow rate adjustment unit 106 based on the user input so that the flow rate at the injection port has the value within the numerical range that satisfies 3.3 (User convenience) and 3.4 (Efficiency).

The sleep assisting device 100 may control the supply adjustment unit 102 and/or the flow rate adjustment unit 106 based on the user input so that the flow rate at the injection port is greater than 0.9 [m/s] and smaller than 1.34 [m/s].

The sleep assisting device 100 may control the supply adjustment unit 102 and/or the flow rate adjustment unit 106 so that the flow rate at the injection port is greater than 0.8 [m/s] and smaller than 1.24 [m/s] based on the user input.

The sleep assisting device 100 may control the supply adjustment unit 102 and/or the flow rate adjustment unit 106 based on the user input so that the flow rate at the injection port is greater than 0.9 [m/s] and smaller than 1.44 [m/s].

The sleep assisting device 100 may control the supply adjustment unit 102 and/or the flow rate adjustment unit 106 based on the user input so that the flow rate at the injection port is greater than 0.9 [m/s] and smaller than 1.24 [m/s].

The sleep assisting device 100 may control the supply adjustment unit 102 and/or the flow rate adjustment unit 106 based on the user input so that the flow rate at the injection port is greater than 1.0 [m/s] and smaller than 1.44 [m/s].

The sleep assisting device may control the supply adjustment unit 102 and/or the flow rate adjustment unit 106 based on the user input so that the carbon dioxide concentration at the injection port has the value within the numerical range that satisfies 3.1 (Safety) and 3.2 (Effectiveness).

The sleep assisting device may control the supply adjustment unit 102 and/or the flow rate adjustment unit 106 based on the user input so that the flow rate at the injection port has the value within the numerical range that satisfies 3.3 (User convenience) and 3.4 (Efficiency).

The sleep assisting device 100 may control the supply adjustment unit 102 and/or the flow rate adjustment unit 106 based on the user input so that the carbon dioxide concentration at the injection port is greater than 50,000 [ppm] and smaller than 200,000 [ppm].

The sleep assisting device 100 may control the supply adjustment unit 102 and/or the flow rate adjustment unit 106 based on the user input so that the carbon dioxide concentration at the injection port is greater than 45,000 [ppm] and smaller than 180,000 [ppm].

The sleep assisting device 100 may control the supply adjustment unit 102 and/or the flow rate adjustment unit 106 based on the user input so that the carbon dioxide concentration at the injection port is greater than 55,000 [ppm] and smaller than 220,000 [ppm].

The sleep assisting device 100 may control the supply adjustment unit 102 and/or the flow rate adjustment unit 106 based on the user input so that the carbon dioxide concentration in the target area is greater than 5,000 [ppm] and smaller than 30,000 [ppm].

The sleep assisting device 100 may control the supply adjustment unit 102 and/or the flow rate adjustment unit 106 based on the user input so that the carbon dioxide concentration in the target area is greater than 5,500 [ppm] and smaller than 33,000 [ppm].

The sleep assisting device 100 may control the supply adjustment unit 102 and/or the flow rate adjustment unit 106 based on the user input so that the carbon dioxide concentration in the target area is greater than 4,500 [ppm] and smaller than 27,000 [ppm].

5. Correlation and tendency of change by factor

5.1 Correlation with carbon dioxide supply pressure

5.1.1 Significance of carbon dioxide supply pressure

The carbon dioxide supply pressure used in this specification may refer to a supply pressure of the carbon dioxide supplied by the carbon dioxide supply unit 101. The carbon dioxide supply pressure may refer to a supply pressure of the carbon dioxide supplied by the carbon dioxide supply unit 101 adjusted by the supply adjustment unit 102.

A supply amount of the carbon dioxide supplied per unit time (hereinafter referred to as a "supply amount per unit time") may be determined based on the carbon dioxide supply pressure. The carbon dioxide supply pressure may be replaced with the supply amount per unit time. The carbon dioxide supply pressure may correspond to the supply amount per unit time. The carbon dioxide supply pressure may have a correlation with the supply amount per unit time. The carbon dioxide supply pressure may have a positive correlation with the supply amount per unit time. The carbon dioxide supply pressure may have a proportional relationship with the supply amount per unit time.

As the carbon dioxide supply pressure increases, the amount of the carbon dioxide supplied per unit time may increase. In addition, as the carbon dioxide supply pressure decreases, the amount of the carbon dioxide supplied per unit time may decrease.

Hereinafter, for convenience of description, the carbon dioxide supply pressure may be described as a supply pressure.

In addition, the carbon dioxide concentration of the mixed gas at the injection port may be described as an injection port carbon dioxide concentration.

In addition, the flow rate of the mixed gas at the injection port may be described as an injection port flow rate.

5.1.2 Correlation between carbon dioxide supply pressure and injection port carbon dioxide concentration

The carbon dioxide supply pressure and the injection port carbon dioxide concentration may be related. The carbon dioxide supply pressure may correspond to the injection port carbon dioxide concentration. The injection port carbon dioxide concentration may be determined according to the carbon dioxide supply pressure.

Hereinafter, in the sleep assisting device 100 according to one embodiment, the correlation between the carbon dioxide supply pressure and the injection port carbon dioxide concentration will be described based on experimental data obtained by measuring the carbon dioxide supply pressure and the injection port carbon dioxide concentration according to the carbon dioxide supply pressure.

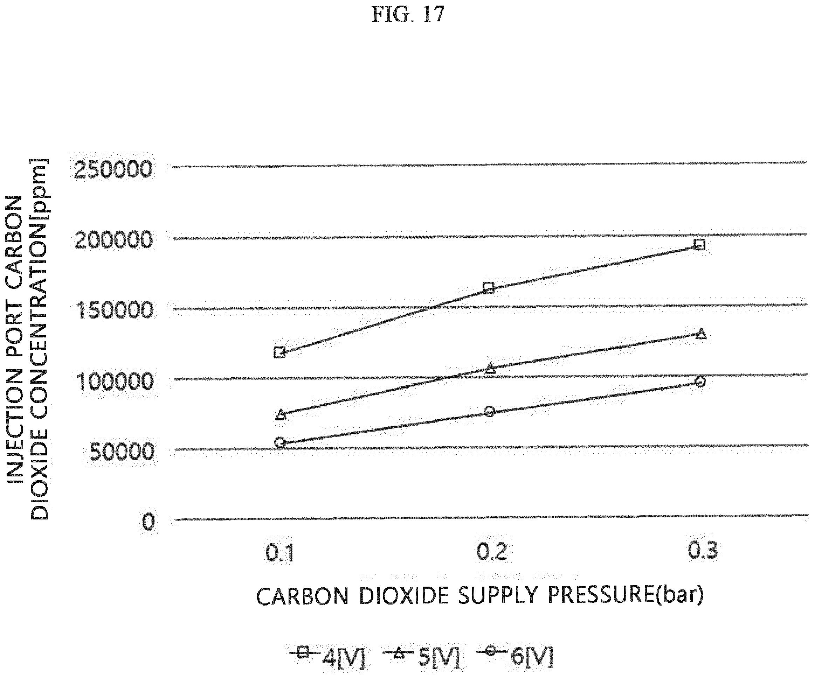

FIG. 17 is seventh experimental data representing the carbon dioxide concentration at the injection port according to the carbon dioxide supply pressure.

The voltages 4 [V], 5 [V], and 6 [V] described in the legend refer to a voltage applied to the flow rate adjustment unit 106.

Referring to FIG. 17, it is shown that when the voltage applied to the flow rate adjustment unit 106 is 4 [V], as a result of measuring the carbon dioxide concentration at the injection port while increasing the carbon dioxide supply pressure from 0.1 [bar] to 0.3 [bar] by 0.1 [bar], the carbon dioxide concentration of the mixed gas at the injection port increases as the carbon dioxide supply pressure increases. It is shown that even when the voltage applied to the flow rate adjustment unit 106 is 5 [V] or 6 [V], the carbon dioxide concentration of the mixed gas at the injection port increases as the carbon dioxide supply pressure increases.

It is shown that when the voltage applied to the flow rate adjustment unit is 4 [V], the carbon dioxide concentration at the injection port increases by an average of 24421 [ppm] as the carbon dioxide supply pressure increases by 0.1 [bar].

It is shown that when the voltage applied to the flow rate adjustment unit is 5 [V], the carbon dioxide concentration at the injection port increases by an average of 22155 [ppm] as the carbon dioxide supply pressure increases by 0.1 [bar].

It is shown that when the voltage applied to the flow rate adjustment unit is 6 [V], the carbon dioxide concentration at the injection port increases by an average of 39802 [ppm] as the carbon dioxide supply pressure increases by 0.1 [bar].

5.1.3 Correlation between carbon dioxide supply pressure and injection port flow rate

The carbon dioxide supply pressure and the injection port flow rate may be related. The carbon dioxide supply pressure may correspond to the injection port flow rate. The injection port flow rate may be determined according to the carbon dioxide supply pressure.

Hereinafter, in the sleep assisting device 100 according to one embodiment, the correlation between the carbon dioxide supply pressure and the injection port flow rate will be described based on experimental data obtained by measuring the carbon dioxide supply pressure and the injection port flow rate.

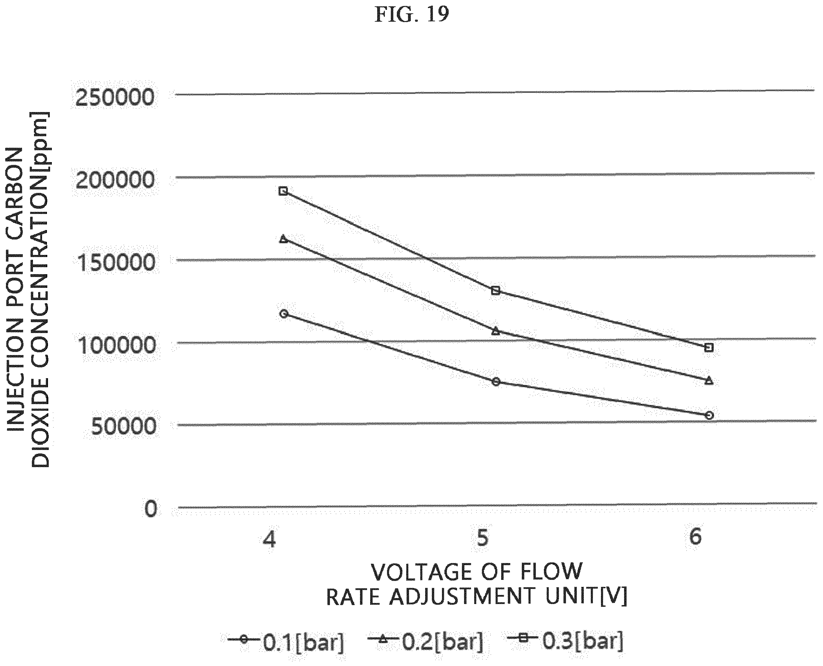

FIG. 19 is ninth experimental data representing the carbon dioxide concentration at the injection port according to the voltage applied to the flow rate adjustment unit. The pressures (0.1 [bar], 0.2 [bar], and 0.3 [bar]) described in the legend refer to the supply pressure of the carbon dioxide supply unit 101.

Referring to FIG. 19, it is shown that when the voltage applied to the flow rate adjustment unit is 4 [V], as a result of measuring the flow rate of the mixed gas at the injection port while increasing the carbon dioxide supply pressure from the carbon dioxide supply unit 101 from 0.1 [bar] to 0.3 [bar] by 0.1 [bar], the flow rate of the mixed gas at the injection port increases as the carbon dioxide supply pressure increases. It is shown that even when the voltage applied to the flow rate adjustment unit 106 is 5 [V] or 6 [V], the flow rate of the mixed gas at the injection port increases as the carbon dioxide supply pressure increases.

It is shown that when the voltage applied to the flow rate adjustment unit is 4 [V], the flow rate of the mixed gas at the injection port increases by 0.25 [m/s] as the carbon dioxide supply pressure increases by 0.1 [bar].

It is shown that when the voltage applied to the flow rate adjustment unit is 5 [V], the carbon dioxide concentration at the injection port increases by an average of 0.24 [m/s] as the carbon dioxide supply pressure increases by 0.1 [bar].

It is shown that when the voltage applied to the flow rate adjustment unit is 6 [V], the carbon dioxide concentration at the injection port increases by an average of 0.26 [m/s] as the carbon dioxide supply pressure increases by 0.1 [bar].

5.2 Correlation with voltage of flow rate adjustment unit

5.2.1 Significance of voltage applied to the flow rate adjustment unit

An amount of the outside air per unit time introduced into the inside of the sleep assisting device 100 (hereinafter referred to as a "introduction amount per unit time") may be determined based on the voltage applied to the flow rate adjustment unit 106. The voltage applied to the flow rate adjustment unit 106 may be replaced with the introduction amount per unit time. The voltage applied to the flow rate adjustment unit 106 may correspond to the introduction amount per unit time. The voltage applied to the flow rate adjustment unit 106 may have a positive correlation with the introduction amount per unit time of the outside air introduced into the sleep assisting device 100 by the flow rate adjustment unit 106. In addition, the voltage applied to the flow rate adjustment unit 106 may have a proportional relationship with the introduction amount per unit time of the outside air.

As the voltage applied to the flow rate adjustment unit 106 increases, the introduction amount per unit time of the introduced outside air may increase. In addition, as the voltage applied to the flow rate adjustment unit 106 decreases, the introduction amount per unit time of the introduced outside air may decrease.

The voltage applied to the flow rate adjustment unit 106 may refer to a rotational speed of the flow rate adjustment unit 106 when the flow rate adjustment unit 106 is configured as a fan. At this time, the voltage applied to the flow rate adjustment unit may have a proportional relationship with the rotational speed of the flow rate adjustment unit.

Hereinafter, for convenience of description, the voltage applied to the flow rate adjustment unit 106 may be described as an application voltage of the flow rate adjustment unit 106.

In addition, the voltage applied to the flow rate adjustment unit 106 may be described as a voltage of the flow rate adjustment unit 106.

5.2.2 Correlation between application voltage of flow rate adjustment unit and flow rate of mixed gas at injection port

The voltage of the flow rate adjustment unit may be related to the injection port flow rate. The voltage of the flow rate adjustment unit may correspond to the injection port flow rate. The injection port flow rate may be determined according to the voltage of the flow rate adjustment unit.

Hereinafter, in the sleep assisting device 100 according to one embodiment, the correlation between the voltage of the flow rate adjustment unit and the injection port flow rate will be described based on the experimental data obtained by measuring the voltage of the flow rate adjustment unit and the injection port flow rate according to the voltage of the flow rate adjustment unit.

Referring to FIG. 12, it is shown that when the carbon dioxide supply pressure is 0.1 [bar], as a result of measuring the flow rate of the mixed gas at the injection port while increasing the voltage of the flow rate adjustment unit 106 from 4 [V] to 6 [V] by 1 [V], the flow rate of the mixed gas at the injection port increases as the voltage of the flow rate adjustment unit increases. It is shown that even when the carbon dioxide supply pressure is 0.1 [bar] or 0.2 [bar], the injection port flow rate increases as the voltage of the flow rate adjustment unit increases.

It is shown that when the carbon dioxide supply pressure is 0.1 [bar], the injection port flow rate increases by an average of 0.25 [m/s] as the voltage of the flow rate adjustment unit increases by 1 [V].

It is shown that when the carbon dioxide supply pressure is 0.2 [bar], the injection port flow rate increases by an average of 0.24 [m/s] as the voltage of the flow rate adjustment unit increases by 1 [V].

It is shown that when the carbon dioxide supply pressure is 0.3 [bar], the injection port flow rate increases by an average of 0.26 [m/s] as the voltage of the flow rate adjustment unit increases by 1 [V].

5.2.3 Correlation between voltage applied to flow rate adjustment unit and injection port carbon dioxide concentration

The voltage of the flow rate adjustment unit may be related to the injection port carbon dioxide concentration. The voltage of the flow rate adjustment unit may correspond to the injection port carbon dioxide concentration. The injection port flow rate may be determined according to the voltage of the flow rate adjustment unit.

Hereinafter, in the sleep assisting device 100 according to one embodiment, the correlation between the voltage of the flow rate adjustment unit and the injection port carbon dioxide concentration will be described based on the experimental data obtained by measuring the voltage of the flow rate adjustment unit and the injection port carbon dioxide concentration according to the voltage of the flow rate adjustment unit.

FIG. 13 is a graph of sixth experimental data obtained by measuring the injection port carbon dioxide concentration according to the voltage of the flow rate adjustment unit. The pressures (0.1 [bar], 0.2 [bar], and 0.3 [bar]) described in the legend refer to a supply pressure of the carbon dioxide supply unit 101.

Referring to FIG. 13, it is shown that when the carbon dioxide supply pressure is 0.1 [bar], as a result of measuring the carbon dioxide concentration at the injection port while increasing the voltage of the flow rate adjustment unit 106 from 4 [V] to 6 [V] by 1 [V], the injection port carbon dioxide concentration decreases as the voltage of the flow rate adjustment unit increases. It is shown that even when the carbon dioxide supply pressure is 0.1 [bar] or 0.2 [bar], the injection port carbon dioxide concentration decreases as the voltage of the flow rate adjustment unit increases.

It is shown that when the carbon dioxide supply pressure is 0.1 [bar], the injection port carbon dioxide concentration decreases by an average of 31544 [ppm] as the voltage of the flow rate adjustment unit increases by 1 [V].

It is shown that when the carbon dioxide supply pressure is 0.2 [bar], the injection port carbon dioxide concentration decreases by an average of 28291 [ppm] as the voltage of the flow rate adjustment unit increases by 1 [V].

It is shown that when the carbon dioxide supply pressure is 0.3 [bar], the injection port carbon dioxide concentration decreases by an average of 28010 [ppm] as the voltage of the flow rate adjustment unit increases by 1 [V].

5.3 Tendency of change in carbon dioxide concentration according to distance from injection port

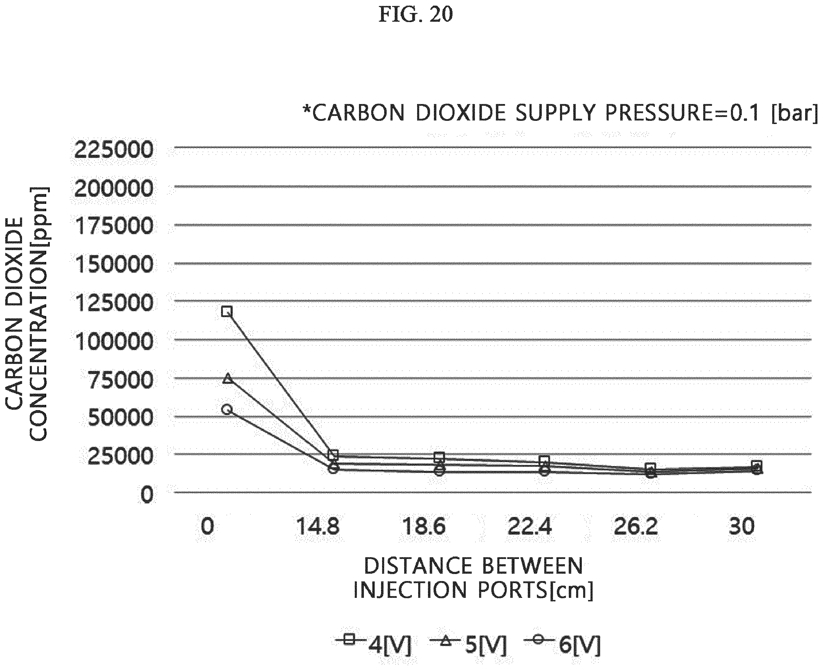

FIGS. 20 to 22 are tenth to twelfth experimental data showing the carbon dioxide concentration according to the distance from the injection port. The voltages 4 [V], 5 [V], and 6 [V] described in the legend refer to a voltage applied to the flow rate adjustment unit 106.

Referring to FIG. 20, when the carbon dioxide supply pressure was 0.1 [bar], the injection port carbon dioxide concentration according to a target distance was measured while increasing the voltage applied to the flow rate adjustment unit 106 from 4 [V] to 6 [V] by 1 [V].

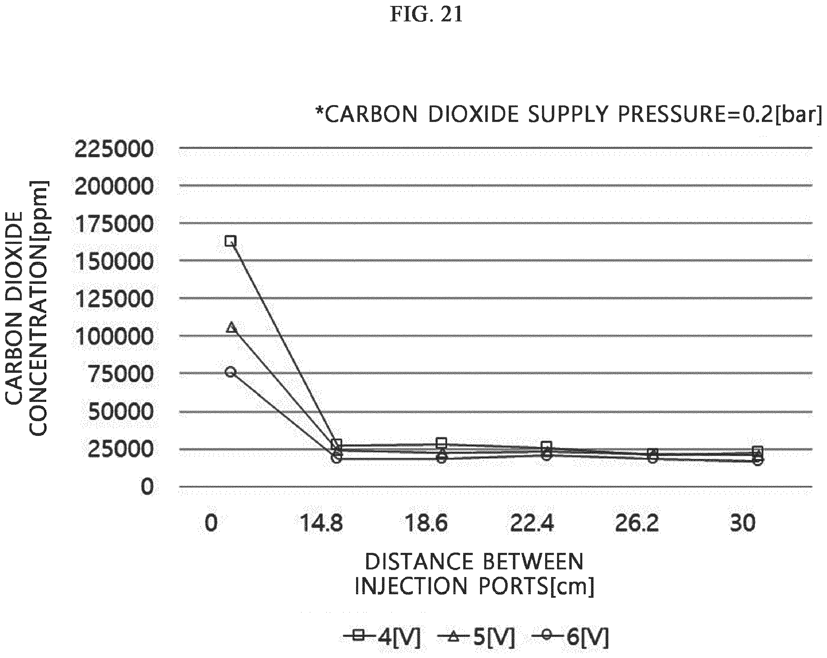

Referring to FIG. 21, when the carbon dioxide supply pressure was 0.2 [bar], the injection port carbon dioxide concentration according to the target distance was measured while increasing the voltage applied to the flow rate adjustment unit 106 from 4 [V] to 6 [V] by 1 [V].

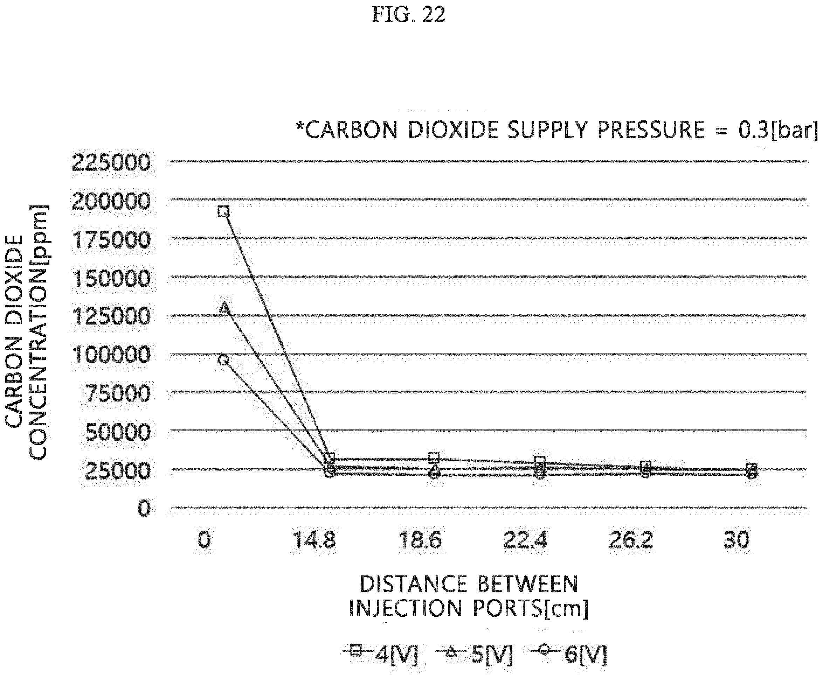

Referring to FIG. 22, when the carbon dioxide supply pressure was 0.3 [bar], the injection port carbon dioxide concentration according to the target distance was measured while increasing the voltage applied to the flow rate adjustment unit 106 from 4 [V] to 6 [V] by 1 [V].

The carbon dioxide concentration was measured at a point where the target distance was 0 [cm]. In addition, the carbon dioxide concentration was measured from a point where the target distance was 14.8 [cm] to a point where the target distance was 30 [cm] at 3.8 [cm] intervals in consideration of the user convenience and efficiency described in 3 (Considerations upon supplying carbon dioxide for sleep assistance).

Referring to FIG. 20, it is shown that when the carbon dioxide supply pressure is 0.1 [bar], the carbon dioxide concentration of the mixed gas decreases as the target distance increases. It is shown that the carbon dioxide concentration of the mixed gas supplied through the injection port decreases as the distance from the injection port increases. Referring to FIGS. 21 and 22, it is shown that even when the carbon dioxide supply pressure is 0.2 [bar] or 0.3 [bar], the carbon dioxide concentration of the mixed gas decreases as the target distance increases. It is shown that the carbon dioxide concentration measured at one position spaced apart from the injection port by a predetermined distance decreases as the distance from the injection port to the one position increases.

It is shown that when the voltage applied to the flow rate adjustment unit 106 is 4 [V], an average of an amount of the carbon dioxide concentration decreased per 1 [cm] from the point where the target distance is 0 [cm] to the point where the target distance is 14.8 [cm] is 6756 [ppm]. In addition, it is shown that the average of the amount of the carbon dioxide concentration decreased per 1 [cm] from the point where the target distance is 14.8 [cm] to a point where the target distance is 30 [cm] is 1155 [ppm].

In consideration of the carbon dioxide concentration at the injection port and a general distance from the injection port to the target area,

it is shown that when the voltage applied to the flow rate adjustment unit 106 is 5 [V], an average of an amount of the carbon dioxide concentration decreased per 1 [cm] from the point where the target distance is 0 [cm] to the point where the target distance is 14.8 [cm] is 2478 [ppm]. In addition, it is shown that the average of the amount of the carbon dioxide concentration decreased per 1 [cm] from the point where the target distance is 14.8 [cm] to the point where the target distance is 30 [cm] is 1303 [ppm].

It is shown that when the voltage applied to the flow rate adjustment unit 106 is 6 [V], an average of an amount of the carbon dioxide concentration decreased per 1 [cm] from the point where the target distance is 0 [cm] to the point where the target distance is 14.8 [cm] is 506 [ppm]. In addition, it is shown that the average of the amount of the carbon dioxide concentration decreased per 1 [cm] from the point where the target distance is 14.8 [cm] to the point where the target distance is 30 [cm] is 1022 [ppm].

Except for the voltage applied to the flow rate adjustment unit 106 of 4 [V] and the carbon dioxide concentrations at the points where the target distances are 14.8 [cm] and 18.6 [cm], it is shown that the carbon dioxide concentrations at the points where the target distances are 14.8 [cm] or more and 30 [cm] or less have the values within the numerical range that satisfies the safety and effectiveness described in 3 (Considerations upon supplying carbon dioxide for sleep assistance) regardless of the voltage applied to the flow rate adjustment unit 106 and the carbon dioxide supply pressure.

The voltage applied to the flow rate adjustment unit 106 is 4 [V] and the carbon dioxide concentration at the point where the target distance is 14.8 [cm] is 31551 [ppm], and the above value is a value that exceeds the maximum value (e.g., 30,000 [ppm]) within the numerical range that satisfies the safety and effectiveness described in 3 (Considerations upon supplying carbon dioxide for sleep assistance) by 5.17 %. In addition, the voltage applied to the flow rate adjustment unit 106 is 4 [V] and the carbon dioxide concentration at the point where the target distance is 18.6 [cm] is 31326 [ppm], and the above value is a value that exceeds the maximum value (e.g., 30,000 [ppm]) within the numerical range that satisfies the safety and effectiveness described in 3 (Considerations upon supplying carbon dioxide for sleep assistance) by 4.42 %. 31551 [ppm] and 31326 [ppm] correspond to values that may satisfy the numerical range that satisfies the safety and effectiveness considering the supply time of the carbon dioxide when the supply time of the carbon dioxide is changed to be shorter than a reference time.

5.4 Tendency of change in flow rate according to distance from injection port

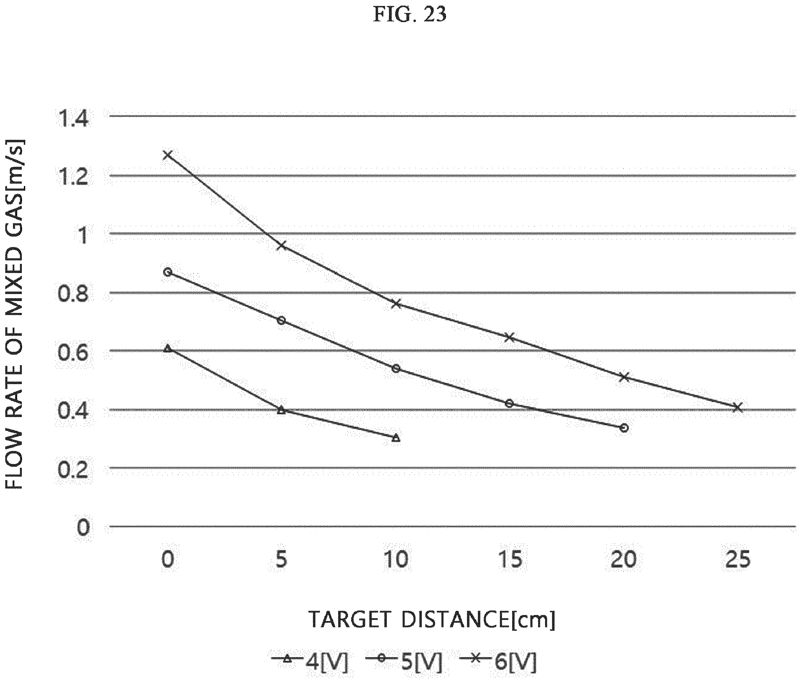

FIG. 23 is thirteenth experimental data representing the flow rate of the gas according to the distance from the injection port (or the target distance). The voltages (4 [V], 5 [V], and 6 [V]) described in the legend refer to voltages applied to the flow rate adjustment unit 106.

In a state in which the carbon dioxide was not supplied, the flow rate of the mixed gas according to the target distance was measured while increasing the voltage applied to the flow rate adjustment unit 106 from 4 [V] to 6 [V] by 1 [V].

The experiment was performed a total of 7 times, and an average value of seven data is shown on the graph. In addition, a minimum measurement value of a flow meter used in this experiment was 0.15 [m/s] or less, and a flow rate at a target distance that could not be measured is not shown on the graph.

When the voltage applied to the flow rate adjustment unit 106 is 4 [V], the flow rate of the mixed gas decreases by an average of 0.17 [m/s] when the target distance increases by 5 [cm]. However, when the voltage applied to the flow rate adjustment unit 106 was 4 [V], the flow rate could not be measured at a point where the target distance was 15 [cm] or more.

When the voltage applied to the flow rate adjustment unit 106 is 5 [V], the flow rate of the mixed gas decreases by an average of 0.20 [m/s] when the target distance increases by 5 [cm]. However, when the voltage applied to the flow rate adjustment unit 106 was 5 [V], the flow rate could not be measured at a point where the target distance was 25 [cm] either.

When the voltage applied to the flow rate adjustment unit 106 is 6 [V], the flow rate of the mixed gas decreases by an average of 0.18 [m/s] when the target distance increases by 5 [cm].

It is shown that the target distance and flow rate of the mixed gas of the sleep assisting device 100 provided in this specification have a negative correlation. It is shown that the flow rate at one position spaced apart from the injection port of the sleep assisting device 100 by a predetermined distance has the negative correlation with the distance at which the one position is spaced apart from the injection port. It is shown that the flow rate at the one position decreases as the distance at which the one position is spaced apart from the injection port increases.

5.5 Comparison of oxygen concentration and carbon dioxide concentration change rates in sleep assisting device

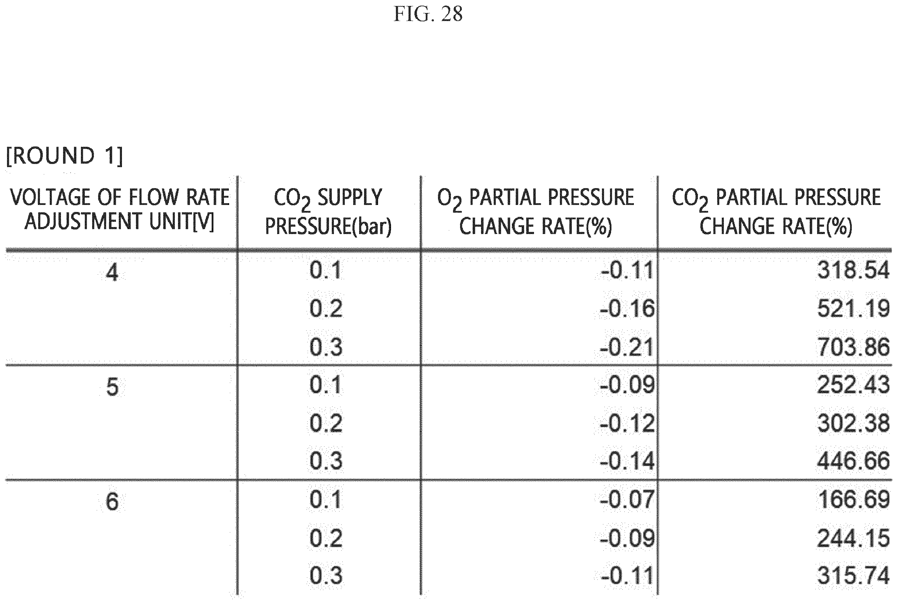

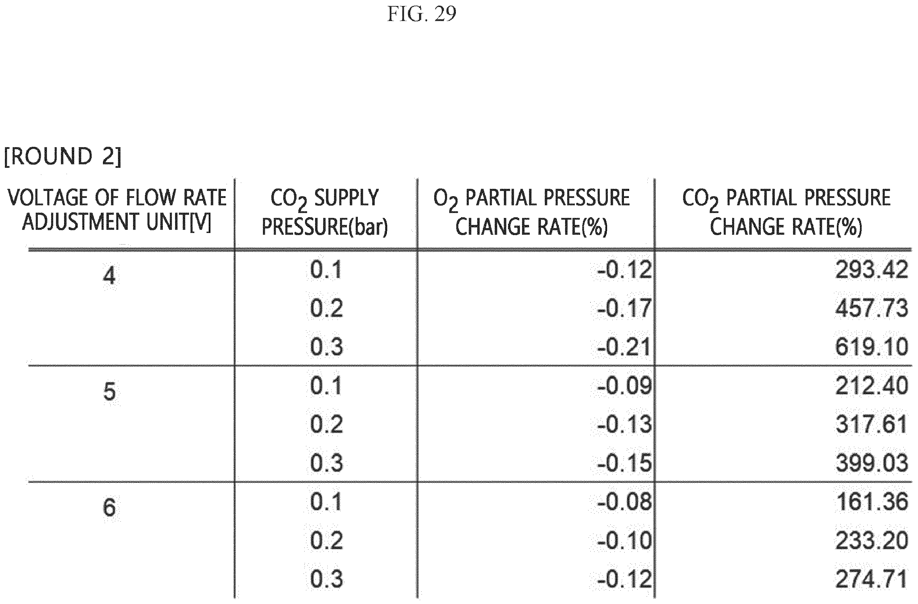

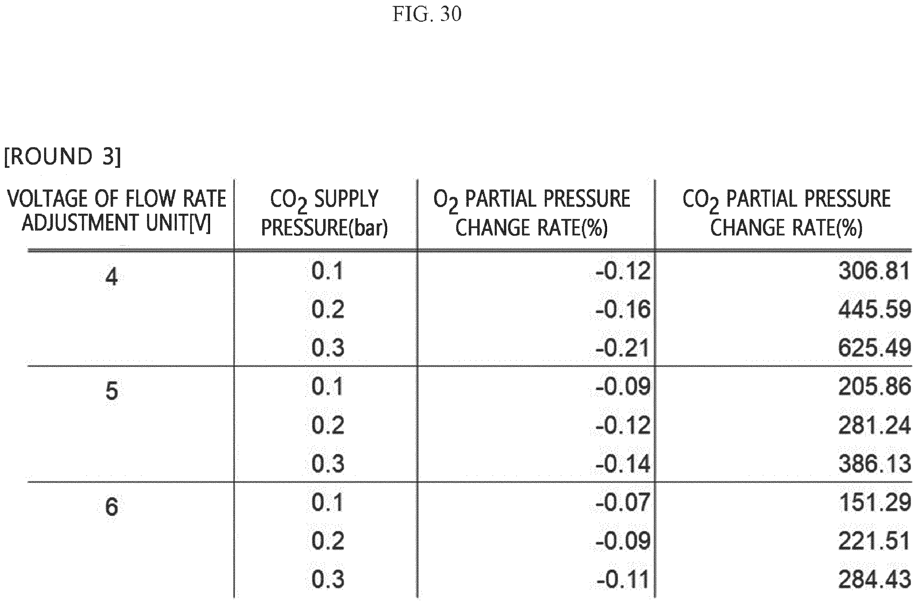

FIGS. 28 to 30 are thirteenth to fifteenth experimental data comparing the oxygen concentration and carbon dioxide concentration change rates according to the sleep assisting operation of the sleep assisting device.

Referring to FIGS. 28 to 30, it can be seen that the oxygen concentration change rate from an area adjacent to the flow rate adjustment unit 106 to the injection port is smaller than the carbon dioxide concentration change rate from the area adjacent to the flow rate adjustment unit 106 to the injection port.

The sleep assisting device 100 may supply the mixed gas so that the oxygen concentration change rate from the area adjacent to the flow rate adjustment unit 106 to the injection port is smaller than the carbon dioxide concentration change rate from the area adjacent to the flow rate adjustment unit 106 to the injection port.

The sleep assisting device 100 may control the supply adjustment unit 102 and/or the flow rate adjustment unit 106 so that the oxygen concentration change rate from the area adjacent to the flow rate adjustment unit 106 to the injection port is smaller than the carbon dioxide concentration change rate from the area adjacent to the flow rate adjustment unit 106 to the injection port.

5.5 Review

5.5.1 Factor determining carbon dioxide concentration (dominant factor)

Referring to 5.1.2 (Correlation between carbon dioxide supply pressure and injection port carbon dioxide concentration), the factor determining the carbon dioxide concentration at the injection port may be the carbon dioxide supply pressure. The carbon dioxide supply pressure may affect the carbon dioxide concentration at the injection port.

Referring to 5.2.3 (Correlation between voltage of flow rate adjustment unit and injection port carbon dioxide concentration), the factor determining the carbon dioxide concentration at the injection port may be a voltage applied to the flow rate adjustment unit 106.

Considering an average increase rate of the injection port carbon dioxide concentration according to the increase in the carbon dioxide supply pressure by 0.1 [bar] described in 5.1.2 (Correlation between carbon dioxide supply pressure and injection port carbon dioxide concentration) and an average decrease rate of the injection port carbon dioxide concentration according to the increase in the application voltage of the flow rate adjustment unit by 1 [V] described in 5.2.3 (Correlation between voltage of flow rate adjustment unit and injection port carbon dioxide concentration), a main factor determining the carbon dioxide concentration of the mixed gas at the injection port may be the carbon dioxide supply pressure and/or the application voltage of the flow rate adjustment unit.

Referring to 5.1.2 (Correlation between carbon dioxide supply pressure and injection port carbon dioxide concentration) and 5.2.3 (Correlation between voltage of flow rate adjustment unit and injection port carbon dioxide concentration), the sleep assisting device 100 may increase the carbon dioxide supply pressure in order to increase the injection port carbon dioxide concentration. In addition, the sleep assisting device 100 may decrease the voltage applied to the flow rate adjustment unit in order to increase the injection port carbon dioxide concentration. In addition, the sleep assisting device 100 may increase the carbon dioxide supply pressure and decrease the voltage applied to the flow rate adjustment unit in order to increase the injection port carbon dioxide concentration.

In addition, the sleep assisting device 100 may decrease the carbon dioxide supply pressure in order to decrease the injection port carbon dioxide concentration. In addition, the sleep assisting device 100 may increase the voltage applied to the flow rate adjustment unit in order to decrease the injection port carbon dioxide concentration. In addition, the sleep assisting device 100 may decrease the carbon dioxide supply pressure and increase the voltage applied to the flow rate adjustment unit in order to decrease the injection port carbon dioxide concentration.

Referring to 5.3 (Tendency of change in concentration of carbon dioxide according to distance from injection port), the main factor determining the carbon dioxide concentration in the target area may be the carbon dioxide concentration at the injection port.

The sleep assisting device 100 may determine the carbon dioxide concentration at the injection port considering the carbon dioxide concentration in a predetermined target area and a tendency of a change in the carbon dioxide concentration according to the target distance.

In order to increase the carbon dioxide concentration in the target area, the sleep assisting device 100 may increase the carbon dioxide concentration at the injection port based on the tendency of the change in the carbon dioxide concentration according to the target distance.

In order to decrease the carbon dioxide concentration in the target area, the sleep assisting device 100 may decrease the carbon dioxide concentration at the injection port based on the tendency of the change in the carbon dioxide concentration according to the target distance.

5.5.2 Factor determining flow rate (dominant factor)

Referring to 5.1.3 (Correlation between carbon dioxide supply pressure and injection port flow rate), a factor determining the flow rate of the mixed gas at the injection port may be the carbon dioxide supply pressure.

Referring to 5.2.2 (Correlation between voltage of flow rate adjustment unit and flow rate of mixed gas at injection port), a factor determining the flow rate of the mixed gas at the injection port may be the voltage applied to the flow rate adjustment unit 106.

Considering an average increase rate of the flow rate of the mixed gas at the injection port according to the increase in the carbon dioxide supply pressure by 0.1 [bar] described in 5.1.3 (Correlation between carbon dioxide supply pressure and injection port flow rate) and an average increase rate of the flow rate of the mixed gas at the injection port according to the increase in the application voltage of the flow rate adjustment unit by 1 [V] described in 5.2.2 (Correlation between voltage of flow rate adjustment unit and flow rate of mixed gas at injection port), the main factor determining the flow rate of the mixed gas at the injection port may be the application voltage of the flow rate adjustment unit. In addition, a sub factor determining the flow rate of the mixed gas at the injection port may be the carbon dioxide supply pressure.

Referring to 5.1.3 (Correlation between carbon dioxide supply pressure and injection port flow rate) and 5.2.2 (Correlation between voltage of flow rate adjustment unit and flow rate of mixed gas at injection port), the sleep assisting device 100 may increase the voltage applied to the flow rate adjustment unit 106 in order to increase the flow rate of the mixed gas at the injection port. In addition, the sleep assisting device 100 may increase the voltage applied to the flow rate adjustment unit 106 and increase the carbon dioxide supply pressure in order to increase the flow rate of the mixed gas at the injection port.

In addition, the sleep assisting device 100 may decrease the voltage applied to the flow rate adjustment unit 106 in order to decrease the flow rate of the mixed gas at the injection port. In addition, the sleep assisting device 100 may decrease the voltage applied to the flow rate adjustment unit 106 and decrease the carbon dioxide supply pressure in order to decrease the flow rate of the mixed gas at the injection port.

Referring to 5.4 (Tendency of change in flow rate according to distance from injection port), the main factor determining the flow rate in the target area may be the flow rate at the injection port.

The sleep assisting device 100 may determine the flow rate of the mixed gas at the injection port considering the flow rate of the mixed gas in the predetermined target area and the tendency of the change in the flow rate of the mixed gas according to the target distance.

The sleep assisting device 100 may determine the flow rate of the mixed gas at the injection port based on the tendency of the change in the flow rate of the mixed gas according to the target distance in order to increase the flow rate of the mixed gas in the target area.

The sleep assisting device 100 may decrease the flow rate of the mixed gas at the injection port based on the tendency of the change in the flow rate of the mixed gas according to the target distance in order to decrease the flow rate of the mixed gas in the target area.

6. Determination of reference value

6.1 Overview

The carbon dioxide concentration of the mixed gas supplied by the sleep assisting device 100 provided in this specification may be determined within the numerical range that satisfies the safety of the sleep assisting method and the effectiveness of the sleep assisting effect as described in 3 (Considerations upon supplying carbon dioxide for sleep assistance).

The flow rate of the mixed gas supplied by the sleep assisting device 100 provided in this specification may be determined within the numerical range that satisfies the user convenience and the efficiency of the carbon dioxide supply as described in 3 (Considerations upon supplying carbon dioxide for sleep assistance).

The sleep assisting device 100 provided in this specification may supply the mixed gas having the carbon dioxide concentration within the numerical range that satisfies the safety and effectiveness described in 3 (Considerations upon supplying carbon dioxide for sleep assistance) to the target area at the flow rate within the numerical range that satisfies the user convenience and efficiency described in 3 (Considerations upon supplying carbon dioxide for sleep assistance). At this time, the carbon dioxide concentration and flow rate of the mixed gas at the injection port may be determined based on the carbon dioxide concentration and flow rate of the mixed gas in the target area.

The sleep assisting device 100 provided in this specification may supply the mixed gas so that the carbon dioxide concentration and flow rate of the mixed gas in the target area have a predetermined concentration and flow rate, respectively.

The sleep assisting device 100 provided in this specification may control the supply adjustment unit 102 and/or the flow rate adjustment unit 106 so that the carbon dioxide concentration and flow rate of the mixed gas in the target area have the predetermined concentration and flow rate, respectively.

Hereinafter, the flow rate and concentration of the mixed gas in the target area may be defined as a target value. In addition, the flow rate and concentration of the mixed gas at the injection port, which allow the target values to have values within the numerical range that satisfies the safety, effectiveness, user convenience, and efficiency described in 3 (Considerations upon supplying carbon dioxide for sleep assistance), may be defined as a reference value.

Hereinafter, a method of performing the sleep assisting operation by the sleep assisting device 100 to satisfy the reference value determined according to the predetermined target value in order to provide the sleep assisting environment to the user positioned in the target area will be described.

6.2 Definition of target value and determination of target value

The sleep assisting device 100 may be operated based on the target value determined to provide an optimal sleep environment to the user. The sleep assisting device 100 may be operated to satisfy the target value.

The target value may refer to target values of the carbon dioxide concentration and the flow rate in the target area spaced apart from the injection port by the target distance. The target value may refer to a target value of the carbon dioxide concentration and/or a target value of the introduction amount in the target area to provide an appropriate sleep assisting environment to the target area. The target value may be determined based on the safety, effectiveness, user convenience, and efficiency described in 3 (Considerations upon supplying carbon dioxide for sleep assistance).

The target value of the concentration (hereinafter referred to as a "concentration target value") may be determined based on the safety and efficiency described in 3 (Considerations upon supplying carbon dioxide for sleep assistance). The concentration target value may be determined within the numerical range that satisfies the safety and efficiency described in 3 (Considerations upon supplying carbon dioxide for sleep assistance). For example, the target concentration value may be determined as a value that is a first concentration (e.g., 5,000 [ppm]) or more and a second concentration (e.g., 30,000 [ppm]) or less.

A minimum concentration target value may refer to a minimum value within the numerical range that satisfies the safety and effectiveness described in 3 (Considerations upon supplying carbon dioxide for sleep assistance). A maximum concentration target value may refer to a maximum value within the numerical range that satisfies the safety and effectiveness described in 3 (Considerations upon supplying carbon dioxide for sleep assistance).

The target value of the flow rate (hereinafter referred to as an "introduction amount target value") may be determined based on the user convenience and efficiency described in 3 (Considerations upon supplying carbon dioxide for sleep assistance). The introduction amount target value may be determined based on the safety and effectiveness described in 3 (Considerations upon supplying carbon dioxide for sleep assistance). For example, the introduction amount target value may be determined as a value that is a first flow rate (e.g., [m/s]) or more and a second flow rate (e.g., [m/s]) or less.

A minimum introduction amount target value may refer to a minimum value within the numerical range that satisfies the user convenience and efficiency described in 3 (Considerations upon supplying carbon dioxide for sleep assistance). A maximum introduction amount target value may refer to a maximum value within the numerical range that satisfies the user convenience and efficiency described in 3 (Considerations upon supplying carbon dioxide for sleep assistance).

The target value may be determined as one value. For example, the target value may be determined as $\alpha$. The target value may be determined as a numerical range. For example, the target value may be determined as a numerical range in which the maximum target value is a*0.9 and the minimum target value is a*1.1. At this time, a may be a concentration or flow rate determined based on the safety and efficiency described in 3 (Considerations upon supplying carbon dioxide for sleep assistance).

6.3 Definition of reference value and determination of reference value

The sleep assisting device 100 may be operated based on the target value determined to provide the optimal sleep environment to the user and the reference value determined according to the target value. The sleep assisting device 100 may be operated to satisfy the reference value determined according to the target value.

The reference values may refer to the carbon dioxide concentration and/or flow rate of the mixed gas at the injection port. The reference values may refer to a reference value of the carbon dioxide concentration of the mixed gas at the injection port and/or a reference value of the introduction amount in order to provide an appropriate sleep assisting environment at the position spaced apart from the injection port by the target distance.

The reference value may be determined based on the target value. The reference value may be determined based on the target value determined to satisfy the safety, effectiveness, user convenience, and efficiency described in 3 (Considerations upon supplying carbon dioxide for sleep assistance).

The reference value of the concentration (hereinafter referred to as a "concentration reference value") may be determined based on the concentration target value. The concentration reference value may be a reference value of the carbon dioxide concentration at the injection port. The concentration reference value may be determined based on the concentration target value and the tendency described in 5.3. For example, the concentration reference value may be determined as a value that is a third concentration (e.g., 50,000 [ppm]) or more and a fourth concentration (e.g., 200,000 [ppm]) or less based on the first concentration target value determined as the value that is the first concentration (e.g., 5,000 [ppm] or more and the second concentration (e.g., 30,000 [ppm]) or less, the target distance (e.g., 25 [cm]), and the tendency of the change in the carbon dioxide concentration according to the distance from the injection port.

A minimum concentration reference value may refer to a minimum value among the reference values determined so that the concentration target value satisfies the safety and effectiveness described in 3 (Considerations upon supplying carbon dioxide for sleep assistance). A maximum concentration reference value may refer to a maximum value among the reference values determined so that the concentration target value satisfies the safety and effectiveness described in 3 (Considerations upon supplying carbon dioxide for sleep assistance).

The reference value of the flow rate (hereinafter referred to as an "introduction amount reference value") may be determined based on the introduction amount target value. The introduction amount reference value may be determined based on the introduction amount target value and the tendency described in 5.4. For example, the introduction amount reference value may be determined as a value that is a third flow rate (e.g., 0.9 [m/s] or more and a fourth flow rate (e.g., 1.34 [m/s]) or less based on a first introduction amount target value determined as a value that is the first flow rate (e.g., 0.05 [m/s]) or more and the second flow rate (e.g., 0.4 [m/s]) or less, a first target distance (e.g., 25 [cm]), and the tendency of the change in concentration at the first target distance.

A minimum introduction amount reference value may refer to a minimum value among the reference values determined so that the introduction amount target value satisfies the user convenience and efficiency described in 3 (Considerations upon supplying carbon dioxide for sleep assistance). A maximum introduction amount reference value may refer to a maximum value among the reference values determined so that the introduction amount target value satisfies the user convenience and efficiency described in 3 (Considerations upon supplying carbon dioxide for sleep assistance).

The reference value may be determined as one value. The reference value may be determined as one value based on the target value determined as one value. For example, the reference value may be determined as $\beta$. The reference value may be determined as $\beta$ based on $\alpha$ that is the target value.

The reference value may be determined as a numerical range. For example, the reference value may be determined as a numerical range that is $\beta*0.9$ or more and $\beta*1.1$ or less based on a numerical range that is $\alpha*0.9$ or more and $\alpha*1.1$ or less, which is the target value.

6.4 Specific embodiment of determination of reference value

6.4.1 Determination of concentration reference values based on concentration target values

When the concentration target value has a value that is the first concentration (e.g., 5,000 [ppm]) or more and the second concentration (e.g., 30,000 [ppm]) or less and the target distance is a first distance (e.g., 20 (cm)), the concentration reference value may be determined as a value that is a third concentration (e.g., 40,000 [ppm]) or more and a fourth concentration (e.g., 100,000 [ppm]) or less in consideration of the tendency described in 5.2 (Correlation with voltage of flow rate adjustment unit).

When the concentration target value has a value that is the first concentration (e.g., 5,000 [ppm]) or more and the second concentration (e.g., 30,000 [ppm]) or less and the target distance is a second distance (e.g., 30 (cm)), the concentration reference value may be determined as a value that is a fifth concentration (e.g., 50,000 [ppm]) or more that is greater than the third concentration and a sixth concentration (e.g., 200,000 [ppm]) or less that is greater than the fourth concentration in consideration of the tendency described in 5.2 (Correlation with voltage of flow rate adjustment unit).

When the target concentration value has a value that is the first concentration*1.1 (e.g., 5,500 [ppm]) or more and the second concentration*1.1 (e.g., 33,000 [ppm]) or less and the target distance is the first distance, the concentration reference value may be determined as a value that is a seventh concentration (e.g., 55,000 [ppm]) or more that is greater than the third concentration and an eighth concentration (e.g., 126,500 [ppm]) or less that is greater than the fourth concentration in consideration of the tendency described in 5.2 (Correlation with voltage of flow rate adjustment unit).

6.4.2 Determination of introduction amount reference value based on introduction amount target value

When the introduction amount target value has a value that is a first flow rate (e.g., 0.35 [m/s]) or more and a second flow rate (e.g., 0.42 [m/s]) or less and the target distance is a first distance (e.g., 20 [cm]), the introduction amount reference value may be determined as a value that is the third flow rate (e.g., 0.9 [m/s]) or more and the fourth flow rate (e.g., 1.34 [m/s] or less in consideration of the tendency described in 5.3 (Tendency of change in concentration of carbon dioxide according to distance from injection port).

When the introduction amount target value has a value that is the first flow rate (e.g., 0.35 [m/s]) or more and the second flow rate (e.g., 0.42 [m/s]) or less and the target distance is a second distance (e.g., 30 [cm]), the introduction amount reference value may be determined as a value that is a fifth flow rate (e.g., 1.0 [m/s]) or more that is faster than the third flow rate and a sixth flow rate (e.g., 1.47 [m/s] or less faster than the fourth flow rate in consideration of the tendency described in 5.3 (Tendency of change in concentration of carbon dioxide according to distance from injection port).

When the introduction amount target value has a value that is the first flow rate*1.1 (e.g., 5,500 [m/s]) or more and the second flow rate*1.1 (e.g., 33,000 [m/s]) or less and the target distance is the first distance, the introduction amount reference value may be determined as a value that is a seventh flow rate (e.g., 1.0 [m/s]) or more that is faster than the third flow rate and an eight flow rate (e.g., 1.47 [m/s] or less faster than the fourth flow rate in consideration of the tendency described in 5.3 (Tendency of change in concentration of carbon dioxide according to distance from injection port).

7. Control of sleep assisting device

7.1 Definition of control variable

The sleep assisting device 100 according to one embodiment may control each unit to perform the sleep assisting operation. The sleep assisting device 100 may control each unit based on the control variable.

The control variable used in this specification may refer to a variable that is the basis of control for performing the sleep assisting operation of the sleep assisting device 100. The control variable may refer to a control signal that is the basis of the control for performing the sleep assisting operation of the sleep assisting device 100.

The control variable may include information for controlling the operation of the sleep assisting device 100. The control variable may include information for controlling each unit of the sleep assisting device 100. The control variable may include information for controlling the supply adjustment unit 102 and/or flow rate adjustment unit 106 of the sleep assisting device 100.

The control variable may include a supply control variable for controlling the supply adjustment unit 102 to supply a mixed gas having a predetermined carbon dioxide concentration and/or flow rate at the injection port.

The control variable may include an introduction amount control variable for controlling the flow rate adjustment unit 106 to supply the mixed gas having the predetermined carbon dioxide concentration and/or flow rate at the injection port.

7.2 Acquisition of control variable

The control variable may be determined based on the reference value. The control variable may be determined in consideration of the correlation between the reference value and the operation of each unit of the sleep assisting device 100. The control variable may be determined based on the correlation between the reference value and the operation of each unit so that the operation of each unit according to the control variable satisfies the reference value. For example, the control variable may be determined based on the correlation between the concentration reference value and the operation of the supply adjustment unit so that the carbon dioxide concentration at the injection port according to the operation of the supply adjustment unit according to the control variable satisfies the concentration reference value.

The control variable may include the supply control variable. The supply control variable may be determined in consideration of the correlation between the concentration reference value and/or the introduction amount reference value and the operation of the supply adjustment unit 102.

The operation (or the supply control variable) of the supply adjustment unit 102 according to the concentration reference value may be determined in consideration of the correlation between the carbon dioxide supply pressure and the injection port carbon dioxide concentration described in 5.1.2 (Correlation between carbon dioxide supply pressure and injection port carbon dioxide concentration) and the factor determining the carbon dioxide concentration at the injection port described in 5.5.1 (Factor determining carbon dioxide concentration).

Based on the correlation between the carbon dioxide supply pressure and the injection port carbon dioxide concentration and the factor determining the carbon dioxide concentration at the injection port described in 5.5.1 (Factor determining carbon dioxide concentration), the correlation of the carbon dioxide concentration at the injection port according to the carbon dioxide supply pressure may be acquired. Based on the correlation of the carbon dioxide concentration at the injection port according to the carbon dioxide supply pressure, a first carbon dioxide supply pressure that allows the carbon dioxide concentration at the injection port to have the concentration reference value may be acquired. Based on the first carbon dioxide supply pressure, the supply control variable for controlling the operation of the supply adjustment unit at the first carbon dioxide supply pressure may be determined.

For example, according to the correlation between the concentration reference value and the operation of the supply adjustment unit 102, the first concentration reference value and a first operation of the supply adjustment unit 102 that allows the supply pressure to have the first pressure may be matched.

In addition, according to the correlation between the concentration reference value and the operation of the supply adjustment unit 102, a second concentration reference value greater than the first concentration reference value and a second operation of the supply adjustment unit 102 that allows the supply pressure to have the second pressure greater than the first pressure may be matched.

For example, based on the correlation between the concentration reference value and the operation of the supply adjustment unit 102, the first concentration reference value and a first supply control variable for controlling the supply adjustment unit 102 so that the supply pressure has the first pressure may be matched.

In addition, based on the correlation between the concentration reference value and the operation of the supply adjustment unit 102, the second concentration reference value greater than the first concentration reference value and a second supply control variable for controlling the supply adjustment unit 102 so that the supply pressure has the second pressure greater than the first pressure may be matched.

In addition, according to the correlation between the concentration reference value and the operation of the supply adjustment unit 102, a third concentration reference value smaller than the first concentration reference value and a third operation of the supply adjustment unit 102 that allows the supply pressure to have the third pressure smaller than the first pressure may be matched.

The correlation between the introduction amount reference value and the operation of the supply adjustment unit 102 may be determined in consideration of the correlation between the carbon dioxide supply pressure and the injection port flow rate described in 5.1.3 (Correlation between carbon dioxide supply pressure and injection port flow rate) and the factor determining the flow rate of the mixed gas at the injection port described in 5.5.2 (Factor determining flow rate).

For example, according to the correlation between the introduction amount reference value and the operation of the supply adjustment unit 102, the first introduction amount reference value and the first operation of the supply adjustment unit 102 that allows the supply pressure to have the first pressure may be matched.

In addition, according to the correlation between the introduction amount reference value and the operation of the supply adjustment unit 102, the first introduction amount reference value and the second operation of the supply adjustment unit 102 that allows the supply pressure to have the second pressure greater than the first pressure may be matched.

In addition, according to the correlation between the introduction amount reference value and the operation of the supply adjustment unit 102, a third introduction amount reference value smaller than the first introduction amount reference value and the third operation of the supply adjustment unit 102 that allows the supply pressure to have the third pressure smaller than the first pressure may be matched.

The introduction amount control variable may be determined in consideration of the correlation between the concentration reference value and/or the introduction amount reference value and the operation of the supply adjustment unit 102.

The correlation between the concentration reference value and the operation of the flow rate adjustment unit 106 may be determined in consideration of the correlation between the voltage of the flow rate adjustment unit and the carbon dioxide concentration at the injection port described in 5.2.3 (Correlation between voltage of flow rate adjustment unit and injection port carbon dioxide concentration) and the factor determining the carbon dioxide concentration at the injection port described in 5.5.1 (Factor determining carbon dioxide concentration).

For example, according to the correlation between the concentration reference value and the operation of the flow rate adjustment unit 106, the first concentration reference value and the first operation of the flow rate adjustment unit 106 that allows the amount of the outside air introduced to have the first introduction amount may be matched.

In addition, according to the correlation between the concentration reference value and the operation of the flow rate adjustment unit 106, the second concentration reference value greater than the first concentration reference value and the second operation of the flow rate adjustment unit 106 that allows the amount of the outside air introduced to have a second introduction amount smaller than the first introduction amount may be matched.

In addition, according to the correlation between the concentration reference value and the operation of the flow rate adjustment unit 106, the third concentration reference value smaller than the first concentration reference value and the third operation of the flow rate adjustment unit 106 that allows the amount of the introduced outside air to have a third introduction amount greater than the first introduction amount may be matched.

The correlation between the introduction amount reference value and the operation of the flow rate adjustment unit 106 may be determined in consideration of the correlation between the voltage of the flow rate adjustment unit and the injection port flow rate described in 5.2.2 (Correlation between voltage of flow rate adjustment unit and flow rate of mixed gas at injection port) and the factor determining the flow rate of the mixed gas at the injection port described in 5.5.2 (Factor determining flow rate).

For example, according to the correlation between the introduction amount reference value and the operation of the flow rate adjustment unit 106, the first introduction amount reference value and the first operation of the flow rate adjustment unit 106 that allows the amount of the introduced outside air to have the first introduction amount may be matched.

In addition, according to the correlation between the introduction amount reference value and the operation of the flow rate adjustment unit 106, the second introduction amount reference value greater than the first introduction amount reference value and the second operation of the flow rate adjustment unit 106 that allows the amount of the introduced outside air to have the second introduction amount greater than the first introduction amount may be matched.

In addition, according to the correlation between the introduction amount reference value and the operation of the flow rate adjustment unit 106, the third introduction amount reference value smaller than the first introduction amount reference value and the third operation of the flow rate adjustment unit 106 that allows the amount of the introduced outside air to have the third introduction amount smaller than the first introduction amount may be matched.

The control variable may be stored in the sleep assisting device 100.

The sleep assisting device 100 may store the control variable determined based on the correlation between the concentration reference value and the operation of the supply adjustment unit 102. The sleep assisting device 100 may store the control variable determined based on the correlation between the concentration reference value and the operation of the supply adjustment unit 102 and the concentration reference value in association with each other. The sleep assisting device 100 may store the concentration target value and/or the concentration reference value determined based on the concentration target value and/or the control variable in association with one another.

The sleep assisting device 100 may store information on the correlation between the concentration reference value and the operation of the supply adjustment unit 102. For example, the sleep assisting device 100 may store the information on the correlation between the concentration reference value and the operation of the supply adjustment unit 102 in the form of a function or look-up table of the concentration reference value according to the operation of the supply adjustment unit 102.

The sleep assisting device 100 may store the control variable determined based on the correlation between the introduction amount reference value and the operation of the supply adjustment unit 102. The sleep assisting device 100 may store the control variable determined based on the concentration between the introduction amount reference value and the operation of the supply adjustment unit 102 and the concentration reference value in association with each other. The sleep assisting device 100 may store the concentration target value and/or the introduction amount reference value determined based on the introduction amount target value and/or the control variable in association with one another. The sleep assisting device 100 may store information on the correlation between the introduction amount reference value and the operation of the supply adjustment unit 102. For example, the sleep assisting device 100 may store the information on the correlation between the introduction amount reference value and the operation of the supply adjustment unit 102 in the form of a function or lookup table of the introduction amount reference value according to the operation of the supply adjustment unit 102.

The sleep assisting device 100 may store the control variable determined based on the correlation between the concentration reference value and the operation of the flow rate adjustment unit 106. The sleep assisting device 100 may store the control variable determined based on the correlation between the concentration reference value and the operation of the flow rate adjustment unit 106 and the concentration reference value in association with each other. The sleep assisting device 100 may store the concentration target value and/or the concentration reference value determined based on the concentration target value and/or the control variable in association with one another.

The sleep assisting device 100 may store information on the correlation between the concentration reference value and the operation of the flow rate adjustment unit 106. For example, the sleep assisting device 100 may store the information on the correlation between the concentration reference value and the operation of the flow rate adjustment unit 106 in the form of a function or lookup table of the concentration reference value according to the operation of the flow rate adjustment unit 106.

The sleep assisting device 100 may store the control variable determined based on the correlation between the introduction amount reference value and the operation of the flow rate adjustment unit 106. The sleep assisting device 100 may store the control variable determined based on the correlation between the introduction amount reference value and the operation of the flow rate adjustment unit 106 and the introduction amount reference value in association with each other. The sleep assisting device 100 may store the introduction amount target value and/or the introduction amount reference value determined based on the introduction amount target value and/or the control variable in association with one another.

The sleep assisting device 100 may store information on the correlation between the introduction amount reference value and the operation of the flow rate adjustment unit 106. For example, the sleep assisting device 100 may store the information on the correlation between the introduction amount reference value and the operation of the flow rate adjustment unit 106 in the form of a function or lookup table of the introduction amount reference value according to the operation of the flow rate adjustment unit 106.

7.3 Control according to control variable

FIG. 18 is a flowchart showing a method of controlling the sleep assisting device for performing the sleep assisting operation based on the control variable according to one embodiment.

Referring to FIG. 18, the method of controlling the sleep assisting device 100 for performing the sleep assisting operation may include an operation of acquiring the control variable and an operation of controlling each unit based on the control variable.

In the operation of acquiring the control variable (S101), the sleep assisting device 100 may acquire the control variable for performing the sleep assisting operation.

The sleep assisting device 100 may acquire the control variable for controlling each unit. The sleep assisting device 100 may acquire the supply control variable for controlling the supply adjustment unit 102. The sleep assisting device 100 may acquire the introduction amount control variable for controlling the flow rate adjustment unit 106.

The sleep assisting device 100 may acquire the control variable for supplying the mixed gas that satisfies the reference value determined based on the target value. The sleep assisting device 100 may acquire the control variable for supplying the mixed gas that satisfies the reference value based on the information on the correlation between the reference value and the operation of each unit.

The sleep assisting device 100 may acquire the control variable from the unit included in the sleep assisting device 100. For example, the sleep assisting device 100 may acquire the control variable from the control unit 103. For another example, the sleep assisting device 100 may acquire the control variable from a storage unit (not shown).

For another example, the sleep assisting device 100 may acquire the control variable from the outside.

The sleep assisting device 100 may acquire the control variable from an external device. For example, the sleep assisting device 100 may acquire the control variable from a user terminal, a tablet PC, a PC, or a remote controller connected to the sleep assisting device 100 wirelessly or by wire.

The sleep assisting device 100 may acquire the control variable from a server. For example, the sleep assisting device 100 may acquire the control variable from the server connected to the sleep assisting device 100 wirelessly or by wire.

The sleep assisting device 100 may acquire the supply control variable based on the correlation between the concentration reference value and the operation of the supply adjustment unit 102. The sleep assisting device 100 may control the supply adjustment unit 102 based on the acquired supply control variable.

For example, when acquiring the first supply control variable in which the first concentration reference value and the first operation of the supply adjustment unit 102 that allows the supply pressure to have the first supply pressure are matched based on the correlation, the sleep assisting device 100 may control the supply adjustment unit 102 to perform the first operation based on the first supply control variable.

The sleep assisting device 100 may acquire the introduction amount control variable based on the correlation between the introduction amount reference value and the operation of the supply adjustment unit 102. The sleep assisting device 100 may control the supply adjustment unit 102 based on the acquired introduction amount control variable.

For example, when acquiring the second introduction amount control variable in which the second introduction amount reference value and the second operation of the supply adjustment unit 102 that allows the supply pressure to have the second supply pressure are matched based on the correlation, the sleep assisting device 100 may control the supply adjustment unit 102 to perform the second operation based on the second introduction amount control variable.

The sleep assisting device 100 may acquire the introduction amount control variable based on the correlation between the concentration reference value and the operation of the flow rate adjustment unit 106. The sleep assisting device 100 may control the flow rate adjustment unit 102 based on the acquired introduction amount control variable.

For example, when acquiring the third introduction amount control variable in which the third concentration reference value and the third operation of the flow rate adjustment unit 106 that allows the amount of the introduced outside air to have the third introduction amount are matched based on the correlation, the sleep assisting device 100 may control the flow rate adjustment unit 106 to perform the third operation based on the third introduction amount control variable.

The sleep assisting device 100 may acquire the introduction amount control variable based on the correlation between the introduction amount reference value and the operation of the flow rate adjustment unit 106. The sleep assisting device 100 may control the flow rate adjustment unit 102 based on the acquired introduction amount control variable.

For example, when acquiring the fourth introduction amount control variable in which the third introduction amount reference value and the fourth operation of the flow rate adjustment unit 106 that allows the amount of the introduced outside air to have the fourth introduction amount are matched based on the correlation, the sleep assisting device 100 may control the flow rate adjustment unit 106 to perform the fourth operation based on the fourth introduction amount control variable.

7.4 Feedback control of sleep assisting device

The sleep assisting device 100 may perform the sleep assisting operation based on information on the mixed gas supplied according to the sleep assisting operation. The sleep assisting device 100 may perform the sleep assisting operation based on the information on the carbon dioxide concentration and flow rate of the mixed gas supplied according to the sleep assisting operation.

The sleep assisting device 100 may perform the sleep assisting operation based on information acquired from at least one of the carbon dioxide concentration detection unit, the distance detection unit, and the flow rate detection unit.

When the information acquired from the carbon dioxide concentration detection unit indicates that the carbon dioxide concentration of the mixed gas is smaller than the reference concentration, the sleep assisting device 100 may control each unit to increase the carbon dioxide concentration of the mixed gas.

For example, when the information acquired from the carbon dioxide concentration detection unit indicates that the carbon dioxide concentration of the mixed gas is smaller than the reference concentration, the sleep assisting device 100 may control the supply adjustment unit 102 to increase the carbon dioxide supply pressure.

For another example, when the information acquired from the carbon dioxide concentration detection unit indicates that the carbon dioxide concentration of the mixed gas is greater than the reference concentration, the sleep assisting device 100 may control the supply adjustment unit 102 to decrease the carbon dioxide supply pressure.

For example, when the information acquired from the carbon dioxide concentration detection unit indicates that the carbon dioxide concentration of the mixed gas is smaller than the reference concentration, the sleep assisting device 100 may control the flow rate adjustment unit 106 to increase the injection port carbon dioxide concentration.

For another example, when the information acquired from the carbon dioxide concentration detection unit indicates that the carbon dioxide concentration of the mixed gas is greater than the reference concentration, the sleep assisting device 100 may control the flow rate adjustment unit 106 to increase the injection port carbon dioxide concentration.

When the information acquired from the distance detection unit indicates that the distance between the injection port and the object is smaller than the reference distance, the sleep assisting device 100 may control each unit based on the acquired information.

For example, when the information acquired from the distance detection unit indicates that the distance between the injection port and the object is smaller than the reference distance, the sleep assisting device 100 may control the supply adjustment unit 102 to decrease the carbon dioxide supply pressure.

For another example, when the information acquired from the distance detection unit indicates that the distance between the injection port and the object is greater than the reference distance, the sleep assisting device 100 may control the supply adjustment unit 102 to increase the carbon dioxide supply pressure.

8. Sleep assisting system

According to one embodiment, the sleep assisting system including the sleep assisting device described in this specification may be provided. Hereinafter, unless otherwise specified, the contents of the sleep assisting device described throughout this specification may be similarly applied thereto.

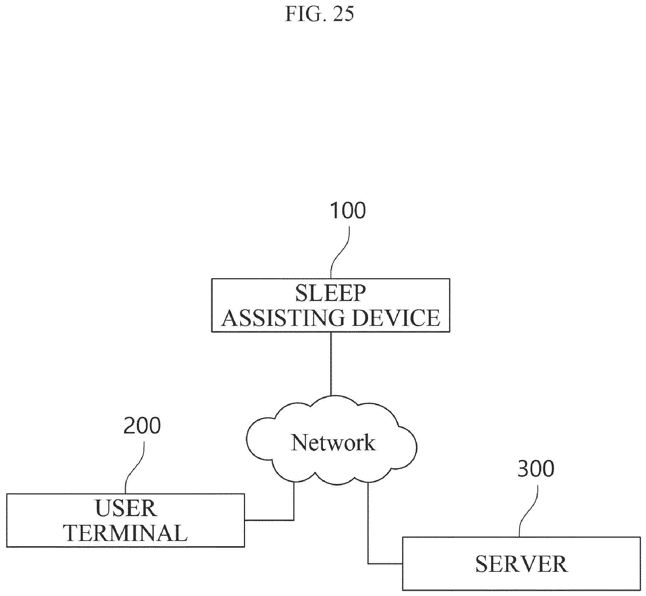

FIG. 25 is a block diagram showing a configuration of the sleep assisting system.

Referring to FIG. 25, the sleep assisting system may include the sleep assisting device 100 and a user terminal 200.

The user terminal 200 may be connected to the sleep assisting device. The user terminal 200 may be connected to the sleep assisting device wirelessly or by wire. The user terminal 200 may be wirelessly connected to the sleep assisting device through wireless communication such as Bluetooth or Wi-Fi.

The user terminal 200 may control the operation of the sleep assisting device. The user terminal 200 may be connected to the sleep assisting device and configured to control the operation of the sleep assisting device.

The user terminal 200 may be connected to the sleep assisting device and configured to acquire an input for controlling the sleep assisting device from the user and control the sleep assisting device based on the acquired input.

The user terminal 200 may acquire sleep data. The user terminal 200 may be connected to the sleep assisting device and configured to acquire the sleep data from the sleep assisting device. The user terminal 200 may acquire the sleep data related to a user's sleep state through a sensor built in the user terminal 200 or the sleep assisting device. The user terminal 200 may receive the sleep data from the user.

The sleep data may include data about a sleep environment to be described below. The sleep data may refer to data related to a sleep pattern to be described below. The sleep data may refer to data about a state of each unit of the sleep assisting device 100. The sleep data may refer to data acquired by each unit of the sleep assisting device 100. The sleep data may refer to data acquired according to a result in which the sleep assisting device 100 has performed the sleep assisting operation.

The user terminal 200 may acquire analysis data obtained based on the sleep data. The user terminal 200 may acquire the analysis data based on the sleep data, or acquire the analysis data acquired based on the sleep data from an external device.

The analysis data may include analysis data obtained by analyzing information on the sleep environment to be described below. The analysis data may include analysis data obtained by analyzing information on the sleep pattern to be described below.

The user terminal 200 may control the sleep assisting device based on the acquired sleep data and/or the analysis data obtained by analyzing the sleep data.

The user terminal 200 may be connected to the sleep assisting device and configured to acquire data from the sleep assisting device and provide the user with the acquired data and new data generated by analyzing the acquired data.

The user terminal 200 may acquire, store, and/or analyze the sleep data, and also provide the sleep assisting device and the user with the sleep data or the analysis data obtained based on the sleep data.

The user terminal 200 may acquire a user input from the user through an interface.

The user input may include an input for controlling the sleep assisting device. Specifically, the input may include an input for controlling the power source of the sleep assisting device. In addition, the input may include an input for controlling the operation of the sleep assisting device. For example, the input may include an input for setting a value or level of the carbon dioxide concentration supplied by the sleep assisting device. In addition, the input may include an input for setting a value or level of the flow rate of the mixed gas supplied by the sleep assisting device. In addition, the input may include an input for setting the supply time of the mixed gas of the sleep assisting device.

The user input may include an input related to user personal information. For example, the user input may include an input including information on the user's gender, age, height, and weight. For example, the user input may be an input including information on a sleep environment preferred by the user.

In addition, the input may include an input related to the user's subjective evaluation. For example, the input may include an input including evaluation information on the user's sleep state. The input may include an input including evaluation information on at least any one of a sleep time, a sleep depth, and sleep quality of the user who uses the sleep assisting device 100.

The sleep inducing system may be configured in a manner of controlling the sleep assisting device 100 through the user terminal 200.

The sleep assisting system may be configured in a manner of controlling the operations of the sleep assisting device 100 and/or each unit for performing the sleep assisting operation through the user terminal 200.

The sleep assisting device 100 may acquire a signal from the user terminal 200, and may be operated based on the acquired signal.

The user terminal 200 may control the power source of the sleep assisting device 100.

The user terminal 200 may control the power source of the sleep assisting device 100 based on the input acquired from the user.

For example, the user terminal 200 may acquire an input including a power-on or power-off command from the user, and control the power source of the sleep assisting device 100 based on the acquired input.

The user terminal 200 may control the power source of the sleep assisting device 100 based on the information acquired from the sleep assisting device 100.

The user terminal 200 may acquire information on the states of the sleep assisting device 100 and/or each unit from the sleep assisting device 100, and control the power source of the sleep assisting device 100 based on the acquired information.

For example, the user terminal 200 may acquire information on a remaining amount of the carbon dioxide in the carbon dioxide supply unit 101 dfrom the sleep assisting device 100, and control the power source of the sleep assisting device 100 based on the acquired information.

The user terminal 200 may control the sleep assisting device 100 to perform the sleep assisting operation. The user terminal 200 may acquire the user input, and control the sleep assisting device 100 to perform the sleep assisting operation based on the acquired user input.

The user terminal 200 may control the sleep assisting device 100 to adjust the supply of the mixed gas. The user terminal 200 may acquire a user input for instructing to perform the sleep assisting operation, and control the sleep assisting device 100 to adjust the supply of the mixed gas based on the acquired user input.

The user terminal 200 may control the sleep assisting device 100 to adjust the carbon dioxide concentration of the mixed gas. The user terminal 200 may control the sleep assisting device 100 to increase or decrease the carbon dioxide concentration of the mixed gas.

The user terminal 200 may acquire a user input for instructing an increase or decrease in the carbon dioxide concentration, and control the sleep assisting device 100 to increase or decrease the carbon dioxide concentration of the supplied mixed gas based on the acquired user input.

The user terminal 200 may control the sleep assisting device 100 to adjust the flow rate of the mixed gas. The user terminal 200 may control the sleep assisting device 100 to increase or decrease the flow rate of the mixed gas.

The user terminal 200 may acquire a user input for instructing an increase or decrease in the flow rate, and control the sleep assisting device 100 to increase or decrease the flow rate of the supplied mixed gas based on the acquired user input.

The user terminal 200 may control the sleep assisting device 100 to adjust the target distance of the sleep assisting device 100. The user terminal 200 may control the sleep assisting device 100 to increase or decrease the target distance.

The user terminal 200 may acquire a user input for instructing an increase or decrease in the target distance, and control the sleep assisting device 100 to increase or decrease the flow rate of the supplied mixed gas based on the acquired user input.

The sleep assisting system may be configured in a manner of providing the user with information on the sleep assisting device 100 through the user terminal 200.

The user terminal 200 may provide information on the carbon dioxide supply unit 101 to the sleep assisting device 100. The user terminal 200 may provide the sleep assisting device 100 with the information on the remaining amount of the carbon dioxide stored in the carbon dioxide supply unit 101.

The sleep assisting device 100 may perform the sleep assisting operation based on the information on the remaining amount of the carbon dioxide in the carbon dioxide supply unit 101 acquired through the user terminal 200.

For example, the sleep assisting device 100 may control the supply adjustment unit 102 based on the information on the remaining amount of the carbon dioxide in the carbon dioxide supply unit 101 acquired through the user terminal 200. More specifically, when the sleep assisting device 100 acquires information indicating that the remaining amount of the carbon dioxide is smaller than a reference remaining amount through the user terminal 200, the sleep assisting device 100 may control the supply adjustment unit 102 to adjust the supply pressure.

For another example, the sleep assisting device 100 may display the information on the remaining amount of the carbon dioxide through the interface included in the sleep assisting device 100 based on the information on the remaining amount of the carbon dioxide in the carbon dioxide supply unit 101 acquired through the user terminal 200.

The sleep assisting device 100 may provide the user terminal 200 with information on the sleep environment.

The user terminal 200 may provide the user with the information on the sleep environment acquired through the sleep assisting device 100. The user terminal 200 may provide the user with an analysis result based on the information on the sleep environment acquired through the sleep assisting device 100. The user terminal 200 may provide the user with recommendation information on the sleeping environment based on the information on the sleep environment acquired through the sleep assisting device 100.

Since the information on the sleep environment, the analysis result based on the sleep environment, and the recommendation information on the sleep environment will be described in 9.1 (Configuration and operation of sleep environment), detailed descriptions thereof will be omitted.

The sleep assisting device 100 may provide the user terminal 200 with the information on the sleep pattern.

The user terminal 200 may provide the user with the information on the sleep pattern acquired through the sleep assisting device 100. The user terminal 200 may provide the user with an analysis result based on the information on the sleep pattern acquired through the sleep assisting device 100. The user terminal 200 may provide the user with recommendation information on the sleep pattern based on the information on the sleep pattern acquired through the sleep assisting device 100.

Since the information on the sleep pattern, the analysis result based on the sleep pattern, and the recommendation information on the sleep pattern will be described in 9.2 (Configuration and operation of sleep pattern), detailed descriptions thereof will be omitted.

The user terminal 200 may provide the user with the information on the sleep assisting device 100. The user terminal 200 may provide the user with the information acquired through the sleep assisting device 100. The user terminal 200 may provide the information on the sleep assisting device 100 and/or the information acquired through the sleep assisting device 100 through the user interface.

The user terminal 200 may provide the user with information on an operation state of the sleep assisting device 100.

The operation state may refer to a power source state of the sleep assisting device 100. In addition, the operation state may refer to the type of the operation performed by the sleep assisting device 100. For example, the operation state may refer to any one of a sleep assisting operation and a wake-up assisting operation that are being performed by the sleep assisting device 100.

The user terminal 200 may provide the user with information on a result of performing the sleep assisting operation of the sleep assisting device 100.

For example, the user terminal 200 may provide the user with a record regarding the supply of the mixed gas for sleep assistance of the sleep assisting device 100. The record regarding the supply of the mixed gas may include at least any one of pieces of information on the carbon dioxide concentration, flow rate, supply time, and supply point of time of the mixed gas.

In addition, the sleep assisting system may further include a server 300.

The server 300 may be configured as a local server or a cloud server. The server 300 may be connected to the user terminal 200 through wireless or wired communication. The server 300 may be connected to the sleep assisting device 100 through wireless or wired communication.

The server 300 may receive the information and data acquired through the user terminal 200 and the sleep assisting device 100. The server 300 may receive the information and data acquired through the user terminal 200 and the sleep assisting device 100, and store the received information and data. The server 300 may transmit the stored information and data to the user terminal 200 and the sleep assisting device 100.

The sleep assisting system may be configured in a manner of connecting the sleep assisting device 100 and the user terminal 200 through the server 300.

The sleep assisting device 100 may be connected to the user terminal 200 through the server 300. The plurality of sleep assisting devices 100 may be connected to the plurality of user terminals 200 through the server 300.

The server 300 may match and store the information on the sleep assisting device 100 and the information on the user terminal 200 owned by the user who uses the sleep assisting device 100.

The information and data acquired from the plurality of sleep assisting devices 100 may be provided to the plurality of user terminals 200 through the server 300. The information and data acquired from the plurality of user terminals 200 may be provided to the plurality of sleep assisting devices 100 through the server 300.

The sleep assisting system may be configured in a manner of storing the information acquired from the sleep assisting device 100 in the server 300.

The sleep assisting system may be configured in a manner of storing the information acquired from the user terminal 200 in the server 300.

9. Additional configuration and operation of sleep assisting device

Hereinafter, the operation of the sleep assisting device will be described with reference to some embodiments. The operation of the sleep assisting device described below may be performed by the sleep assisting device or the sleep assisting system including the sleep assisting device described above in this specification unless otherwise specified.

9.1 Configuration and operation of sleep environment

The sleep assisting device 100 provided in this specification may acquire information on the sleep environment. The sleep assisting device 100 may acquire an analysis result by analyzing the information on the sleep environment.

The sleep environment may refer to an environmental factor that affects the user's sleep in a space where the sleep assisting device 100 is positioned. For example, the sleep environment may refer to information on at least any one of the temperature, humidity, and illuminance in the space where the sleep assisting device 100 is positioned. For another example, the sleep environment may refer to information on at least any one of the temperature and humidity of the gas supplied by the sleep assisting device 100.

The sleep assisting device 100 provided in this specification may perform the sleep assisting operation using the information on the sleep environment.

The sleep assisting operation using the information on the sleep environment may include at least any one of the adjustment of the temperature and/or humidity of the mixed gas used for sleep assistance, the adjustment of the temperature and/or humidity of wind used for wake-up induction, and the adjustment of the temperature, humidity and/or illuminance in the space where the sleep assisting device is positioned.

The sleep assisting device 100 provided in this specification may provide the user with the information on the sleep environment and/or the recommendation information on the sleep environment.

The recommendation information on the sleep environment may include recommendation information on a temperature condition, a humidity condition, and/or an illuminance condition for creating a comfortable sleep environment.

The sleep assisting device 100 may include a temperature detection unit (not shown).

The temperature detection unit may acquire a temperature of the mixed gas supplied by the sleep assisting device 100. The temperature detection unit may acquire information on the temperature of the mixed gas supplied by the sleep assisting device 100, and acquire a temperature measurement value based on the acquired information. The temperature detection unit may transmit the acquired temperature measurement value to an external device.

The temperature detection unit may acquire a temperature in an area where the sleep assisting device 100 is positioned. The temperature detection unit may acquire information on the temperature in the area where the sleep assisting device 100 is positioned, and acquire a temperature measurement value based on the acquired information. The temperature detection unit may transmit the temperature measurement value in the area where the sleep assisting device 100 is positioned to the external device.

The sleep assisting device 100 may include a humidity detection unit (not shown).

The humidity detection unit may acquire information on the humidity of the mixed gas supplied by the sleep assisting device 100. The humidity detection unit may acquire a measurement value related to the humidity of the mixed gas supplied by the sleep assisting device 100. The humidity detection unit may transmit the measurement value obtained by measuring the humidity of the mixed gas to the external device.

The humidity detection unit may acquire information on the humidity in the area where the sleep assisting device 100 is positioned. The humidity detection unit may acquire the information on the humidity in the area where the sleep assisting device 100 is positioned, and acquire a measurement value based on the acquired humidity information. The humidity detection unit may transmit the humidity measurement value in the area where the sleep assisting device 100 is positioned to the external device.

The sleep assisting device 100 may include an illuminance detection unit (not shown).

The illuminance detection unit may acquire information on the illuminance in the area where the sleep assisting device 100 is positioned. The illuminance detection unit may acquire the information on the illuminance in the area where the sleep assisting device 100 is positioned, and acquire an illuminance measurement value based on the acquired information. The illuminance detection unit may transmit the illuminance measurement value in the area where the sleep assisting device 100 is positioned to the external device.

The external device may include a user terminal, a PC, a tablet, or a server.

The sleep assisting device 100 may acquire the information on the sleep environment.

The sleep assisting device 100 may acquire the information on the sleep environment using at least any one of the temperature detection unit, the humidity detection unit, and the illuminance detection unit.

The sleep assisting device 100 may acquire the information on the sleep environment from the outside. The sleep assisting device 100 may acquire the information on at least any one of temperature, humidity, and illuminance from the outside.

For example, the sleep assisting device 100 may acquire the information on the sleep environment from the user terminal.

For another example, the sleep assisting device 100 may acquire the information on the sleep environment from the server.

The sleep assisting device 100 may analyze the sleep environment. The sleep assisting device 100 may analyze the sleep environment based on the measurement value acquired by measuring the sleep environment. The sleep assisting device 100 may acquire an analysis result by analyzing the sleep environment.

The sleep environment analysis may refer to comparing a reference value and the information on the sleep environment. The reference value may be a predetermined value or a value received from the user.

For example, the sleep environment analysis may refer to comparing a predetermined temperature reference value and the temperature measurement value. For another example, the sleep environment analysis may refer to comparing a predetermined humidity reference value and the humidity measurement value. For another example, the sleep environment analysis may refer to comparing a predetermined illuminance reference value and the illuminance measurement value.

The sleep environment analysis result may refer to a determination result of determining whether the area where the sleep assisting device 100 is positioned is in an environment suitable for sleep induction based on the comparison result obtained by comparing the predetermined reference value and the information on the sleep environment. For example, when the comparison result includes information indicating that the temperature measurement value is greater than the temperature reference value and the humidity measurement value is greater than the humidity reference value, the sleep environment analysis result may include recommendations of a decrease in temperature and humidity in order to create the environment suitable for sleep induction.

The sleep assisting device 100 may perform the sleep assisting operation using the information on the sleep environment acquired from at least any one of the temperature detection unit, the humidity detection unit, and the illuminance detection unit.

The sleep assisting device 100 may adjust at least any one of the temperature and humidity of the mixed gas used for sleep assistance using the information on the sleep environment acquired from at least any one of the temperature detection unit, the humidity detection unit, and the illuminance detection unit.

The sleep assisting device 100 may adjust at least any one of the temperature and humidity of the wind used for wake-up induction using the information on the sleep environment acquired from at least any one of the temperature detection unit, the humidity detection unit, and the illuminance detection unit.

The sleep assisting device 100 may adjust at least any one of the temperature, humidity, and illuminance in the space where the sleep assisting device 100 is positioned using the information on the sleep environment acquired from at least any one of the temperature detection unit, the humidity detection unit, and the illuminance detection unit.

The sleep assisting device 100 may provide the user with the information on the sleep environment.

For example, the sleep assisting device 100 may provide the information on the sleep environment through the user interface included in the sleep assisting device 100.

For another example, the sleep assisting device 100 may provide the user with the information on the sleep environment through at least any one of a user terminal, a PC, a tablet, and a remote controller.

The sleep assisting device 100 may provide the recommendation information on the sleep environment based on the information on the sleep environment.

For example, the sleep assisting device 100 may provide the user with information including at least any one of the pieces of information on the temperature condition, humidity condition, and illuminance condition for creating the comfortable sleep environment based on the information on the sleep environment.

For another example, the sleep assisting device 100 may provide the user with information including at least any one of the pieces of information on the temperature condition, humidity condition, and illuminance condition in the sleeping environment preferred by the user based on the information on the sleep environment.

9.2 Configuration and operation of sleep pattern

The sleep assisting device 100 provided in this specification may measure information on the sleep pattern. The sleep assisting device 100 may acquire the information on the sleep pattern. The sleep assisting device 100 may acquire an analysis result by analyzing the information on the sleep pattern.

The information on the sleep pattern may include a result obtained by determining a sleep experience performed by the user who uses the sleep assisting device 100. The information on the sleep pattern may include a result obtained by determining the sleep state of the user who uses the sleep assisting device 100. The information on the sleep pattern may include a result obtained by determining a time when the user who uses the sleep assisting device 100 is in a sleep state. The information on the sleep pattern may include information on the sleep depth, stability, or satisfaction of the user who uses the sleep assisting device 100. The information on the sleep pattern may be generated based on information input by the user.

The information on the sleep pattern may include a measurement result obtained by measuring the user's sleep state. The information on the sleep pattern may include a measurement result obtained by measuring whether the user sleeps. The information on the sleep pattern may include information on the user's sleep time. The information on the sleep pattern may include a measurement result obtained by measuring the user's sleep depth. The information on the sleep pattern may include a measurement result obtained by measuring at least any one of the user's brainwave, heart rate, body temperature, respiration rate, and movement, and whether the user's eyes are closed.

The information on the sleep pattern may include an analysis result obtained by analyzing at least any one of information on the result of determining whether the user is in the sleep state or information on the measurement result of measuring the sleep state.

The sleep assisting device 100 provided in this specification may perform the sleep assisting operation based on the information on the sleep pattern.

The sleep assisting operation using the information on the sleep pattern may include any one of the adjustments of the carbon dioxide concentration, flow rate, target distance, and supply time of the mixed gas used for sleep assistance.

The sleep assisting device 100 provided in this specification may provide recommendation information on the sleep pattern based on the information on the sleep pattern.

The recommendation information on the sleep pattern may include recommendation information on a sleep time, and the carbon dioxide concentration, flow rate, target distance, and supply time of the mixed gas used for sleep assistance.

The sleep assisting device 100 or the terminal may include a sleep pattern measuring unit (not shown) configured to measure the sleep pattern. The following operation of managing the sleep pattern such as measuring the sleep pattern may be performed by the terminal or the server, but hereinafter, for convenience, the sleep assisting device 100 will be described.

The sleep pattern measuring unit may measure the user's sleep state. The sleep pattern measuring unit may acquire a measurement result by measuring the user's sleep state. The sleep pattern measuring unit may measure at least one of whether the user sleeps, the sleep time, and the sleep depth. The sleep pattern measuring unit may measure at least one of the user's brainwave, heart rate, body temperature, respiration rate, blood pressure, and movement, and whether the user's eyes are closed.

The sleep assisting device 100 may acquire the information on the sleep pattern.

The sleep assisting device 100 may measure the sleep pattern, and acquire the information on the sleep pattern based on the measurement result.

The sleep assisting device 100 may acquire the information on the sleep pattern from the external device. For example, the sleep assisting device 100 may acquire the information on the sleep pattern from the user terminal.

The sleep assisting device 100 may acquire the information on the sleep pattern from the server. For example, the sleep assisting device 100 may acquire the information on the sleep pattern from an external server related to medical care.

The sleep assisting device 100 may acquire an analysis result obtained by analyzing the information on the sleep pattern.

The sleep pattern analysis may refer to comparing a predetermined reference value with the information on the sleep pattern. For example, the sleep pattern analysis may refer to comparing an average heart rate upon sleeping and a user's heart rate upon sleeping. For another example, the sleep pattern analysis may refer to comparing the average number of tossing and turning upon sleeping and the number of tossing and turning when the user sleeps. For another example, the sleep pattern analysis may refer to comparing an average blood pressure upon sleeping and a user's blood pressure upon sleeping.

The sleep pattern analysis result may refer to a determination result of determining whether the sleep assisting operation performed by the sleep assisting device 100 is suitable for the user's sleep induction based on the comparison result obtained by comparing the predetermined reference value and the information on the sleep pattern. For example, when the comparison result includes information indicating that the user's heart rate upon sleeping is higher than the average heart rate and the average blood pressure upon sleeping is higher than the user's blood pressure upon sleeping, the sleep pattern analysis result may include a content that evaluates the sleep quality based on the analysis result.

For example, the sleep assisting device 100 may acquire the analysis result by analyzing the result obtained by determining the sleep experience performed by the user.

For another example, the sleep assisting device 100 may acquire the analysis result by analyzing the information including at least one of the user's brainwave, heart rate, body temperature, respiration rate, and movement, and whether the user's eyes are closed. Here, the analysis result may include evaluation information on the sleep state of the user who uses the sleep assisting device 100.

The information on the sleep pattern may be acquired from a unit or an external device configured to measure the sleep pattern.

In addition, the information on the sleep pattern may be acquired based on the user input acquired from the user who uses the sleep assisting device 100.

The information on the sleep pattern may include a result obtained by measuring and determining whether the user who uses the sleep assisting device 100 is in the sleep state. The information on the sleep pattern may include a result obtained by measuring and determining a time when the user who uses the sleep assisting device 100 is in the sleep state. The information on the sleep pattern may include a result obtained by measuring and determining the sleep depth of the user who uses the sleep assisting device 100.

The sleep assisting device 100 may include a sleep pattern acquiring unit (not shown) configured to acquire the sleep pattern.

The sleep pattern acquiring unit may acquire the information on the sleep pattern of the user who uses the sleep assisting device 100. The sleep pattern acquiring unit may measure the information on the sleep pattern of the user who uses the sleep assisting device 100.

The sleep pattern acquiring unit may acquire and measure the information on the sleep pattern of the user who uses the sleep assisting device 100, and acquire the information on the analysis result obtained by analyzing the sleep pattern of the user based on the information on the sleep pattern of the user.

The sleep pattern measuring unit may include a camera. For example, the camera may include at least any one of an IR camera, an RGB camera, and a thermal camera.

The sleep pattern measuring unit may include a sensor. The sleep pattern measuring unit may include at least any one of an image sensor and a pressure sensor.

The sleep pattern measuring unit may include at least any one of the sensor, the camera, and the device configured to measure at least any one of the brainwave, the heart rate, the body temperature, the respiration rate, and the blood pressure.

The sleep assisting device 100 provided in this specification may acquire the information on the sleep pattern.

The sleep assisting device 100 may measure the sleep pattern, and acquire the information on the sleep pattern based on the measurement result obtained by measuring the sleep pattern.

The sleep assisting device 100 may acquire the information on the sleep pattern from an external device configured to measure the sleep pattern. For example, the sleep assisting device 100 may acquire the information on the sleep pattern from an external device including an electroencephalogram (EEG) sensor configured to measure the sleep pattern. For another example, the sleep assisting device 100 may acquire the information on the sleep pattern from an external device including a heart rate sensor configured to measure the sleep pattern.

The sleep assisting device 100 may acquire the information on the sleep pattern from the external device or an external server. For example, the sleep assisting device 100 may acquire the information on the sleep pattern from the user terminal. For another example, the sleep assisting device 100 may acquire the information on the sleep pattern from a server related to a medical institution.

The sleep assisting device 100 may perform the sleep assisting operation based on the information on the sleep pattern.

The sleep assisting device 100 may supply the mixed gas based on the information on the sleep pattern. The sleep assisting device 100 may supply the mixed gas to satisfy the target value and/or the reference value based on the information on the sleep pattern.

The sleep assisting device 100 may supply the mixed gas to satisfy an increased or decreased target value and/or reference value based on the information on the sleep pattern.

For example, the sleep assisting device 100 may supply the mixed gas to satisfy a first concentration target value and/or a second concentration reference value. At this time, when the acquired information on the sleep pattern includes information indicating that the user's heart rate upon sleeping is higher than an average heart rate upon sleeping, the sleep assisting device 100 may supply the mixed gas to satisfy the second concentration target value greater than the first concentration target value based on the information on the sleep pattern.

For another example, the sleep assisting device 100 may supply the mixed gas for a first time. At this time, when the acquired information on the sleep pattern includes information indicating that the user's sleep time is shorter than an average sleep time, the sleep assisting device 100 may supply the mixed gas for a second time longer than the first time based on the information on the sleep pattern.

The sleep assisting device 100 may provide the recommendation information on the sleep pattern based on the information on the sleep pattern.

For example, when the information on the sleep pattern indicates that the user's sleep time is shorter than the average sleep time, the recommendation information on the sleep pattern may include a content that recommends the user to increase the supply time of the sleep assisting device 100.

For another example, when the information on the sleep pattern indicates that the user's sleep depth is smaller than a reference sleep depth, the recommendation information on the sleep pattern may include a content that recommends the user to increase at least one of the concentration reference value and/or concentration target value, or carbon dioxide supply pressure of the sleep assisting device 100.

The sleep assisting device 100 may determine the supply time for supplying the mixed gas based on the information on the sleep pattern.

For example, when the user's sleep time is shorter than a reference time, the sleep assisting device 100 may increase the supply time based on the information on the sleep pattern including the comparison result of comparing the user's sleep time and the reference time.

For another example, when the evaluation value obtained by evaluating the user's sleep depth is smaller than a sleep reference value, the sleep assisting device 100 may increase the supply time based on the information on the sleep pattern including the comparison result of comparing the evaluation value and the sleep reference value.

For another example, when the measurement value obtained by measuring the user's heart rate is greater than a heart rate reference value, the sleep assisting device 100 may increase the supply time based on the information on the sleep pattern including the comparison result of comparing the heart rate measurement value and the heart rate reference value.

The sleep assisting device 100 may provide the information on the sleep pattern to the outside.

The sleep assisting device 100 may provide the user terminal with the information on the sleep pattern. The sleep assisting device 100 may provide the user terminal with the information on the sleep pattern to be readable by the user.

For example, the sleep assisting device 100 may provide the user terminal with information including at least any one of the pieces of information on the user's sleep time, sleep depth, heart rate upon sleeping, and brainwave state upon sleeping to be readable by the user.

The sleep assisting device 100 may provide the information on the sleep pattern to the external server. The sleep assisting device 100 may provide the information on the sleep pattern to the external server to be readable through the external server.

For example, the sleep assisting device 100 may provide the external server with the information including at least any one of the pieces of information on the user's sleep time, sleep depth, heart rate upon sleeping, and brainwave state upon sleeping to be readable through the external server.

9.3 Configuration and operation for recognizing remaining amount of carbon dioxide

The sleep assisting device 100 provided in this specification may recognize unique information included in the carbon dioxide supply unit 101. The sleep assisting device 100 may acquire the unique information included in the carbon dioxide supply unit 101.

The unique information may refer to identification information given to individual carbon dioxide storage containers when the carbon dioxide supply unit 101 includes a replaceable carbon dioxide storage container.

The unique information may include information composed of any one of numbers, letters, and symbols. The unique information may include information composed of any one of a barcode and a QR code. The unique information may be built in a chip recognizable through short-range wireless communication.

The sleep assisting device 100 provided in this specification may recognize and acquire the unique information included in the carbon dioxide supply unit 101, and acquire information on the remaining amount of the carbon dioxide stored in the carbon dioxide supply unit 101 based on the unique information.

The sleep assisting device 100 provided in this specification may provide the information on the remaining amount of the carbon dioxide stored in the carbon dioxide supply unit 101.

The sleep assisting device 100 may include a recognition unit configured to recognize the unique information included in the carbon dioxide supply unit 101.

The sleep assisting device 100 may be connected to the recognition unit configured to acquire the unique information included in the carbon dioxide supply unit 101. The sleep assisting device 100 may be connected to an external device configured to recognize the unique information included in the carbon dioxide supply unit 101 wirelessly or by wire. For example, the recognition unit may be the user terminal, and the sleep assisting device 100 may be wirelessly connected to the user terminal.

The recognition unit may acquire the unique information included in the carbon dioxide supply unit 101. The recognition unit may be connected to the carbon dioxide supply unit 101 through wireless or wired communication and configured to recognize the unique information included in the carbon dioxide supply unit 101. For example, the recognition unit may be connected to the carbon dioxide supply unit 101 by wireless communication such as Wi-Fi, Bluetooth, or NFC.

The recognition unit may acquire the unique information included in the carbon dioxide supply unit 101. The recognition unit may recognize the unique information included in the carbon dioxide supply unit 101, and acquire the unique information based on the recognized result. The recognition unit may acquire data about the unique information included in the carbon dioxide supply unit 101, and acquire the unique information based on the acquired data.

When the carbon dioxide supply unit 101 is disposed at a predetermined position, the recognition unit may recognize the unique information included in the carbon dioxide supply unit 101. When the recognition unit is disposed within a predetermined distance from the carbon dioxide supply unit 101, the recognition unit may recognize the unique information included in the carbon dioxide supply unit 101.

The carbon dioxide supply unit 101 may include a unique information recognition unit including the unique information. For example, the unique information recognition unit may include an electronic circuit chip, NFC chip, QR code or barcode including the unique information of the carbon dioxide supply unit 101.

The sleep assisting device 100 may recognize and acquire the unique information of the carbon dioxide supply unit 101 connected to the sleep assisting device 100.

The sleep assisting device 100 may be connected to the carbon dioxide supply unit 101 through wireless or wired communication and configured to recognize and acquire the unique information of the carbon dioxide supply unit 101.

For example, the sleep assisting device 100 may be connected to the carbon dioxide supply unit 101 through NFC wireless communication and configured to recognize and acquire the unique information of the carbon dioxide supply unit 101.

For another example, the sleep assisting device 100 may recognize and acquire the unique information of the carbon dioxide supply unit 101 by recognizing the QR code or barcode included in the carbon dioxide supply unit 101.

The sleep assisting device 100 may acquire information on the remaining amount of the carbon dioxide in the carbon dioxide supply unit 101 corresponding to the acquired unique information.

The sleep assisting device 100 may acquire the information on the remaining amount of the carbon dioxide in the carbon dioxide supply unit 101 using information on at least any one of the carbon dioxide supply pressure, the carbon dioxide supply amount, the supply time of the carbon dioxide, the number of times of supplying the carbon dioxide, and the number of times of supplying the mixed gas.

For example, when supplying the carbon dioxide by a first amount by supplying the carbon dioxide at a certain concentration for a first time by the sleep assisting device 100 is set to supplying the carbon dioxide once, the sleep assisting device 100 may acquire the information on the remaining amount of the carbon dioxide in the carbon dioxide supply unit 101 through an equation of (the number to times of supplying carbon dioxide)*(first supply amount).

For another example, when the sleep assisting device 100 supplies the carbon dioxide at a first pressure per unit time for the first time so that the supply amount of the carbon dioxide has K1*(first pressure)*(first time) (K1 refers to any constant), and the sleep assisting device 100 supplies the carbon dioxide at a second pressure per unit time for a second time so that the supply amount of the carbon dioxide has K2*(second pressure)*(second time) (K2 refers to any constant), the sleep assisting device 100 may acquire the information on the remaining amount of the carbon dioxide supply unit 101 based on an equation of (total consumption of the carbon dioxide)=K1*(first pressure)*(first time)+K2*(second pressure)*(second time).

The information on the remaining amount of the carbon dioxide may be stored and/or managed. The information on the remaining amount of the carbon dioxide may be stored and/or managed together with unique information of a corresponding carbon dioxide storage container. The server, the user terminal, or the sleep assisting device may store and/or manage the information on the remaining amount of the carbon dioxide in association with the unique information of the carbon dioxide storage container.

For example, the sleep assisting device 100 may acquire information on a weight of the carbon dioxide storage container included in the carbon dioxide supply unit 101 corresponding to the acquired unique information. The sleep assisting device 100 may acquire the information on the acquired weight of the storage container, and acquire the information on the remaining amount of the carbon dioxide based on the acquired information on the number of times of supply.

9.4 Wake-up assisting operation of sleep assisting device

The sleep assisting device 100 provided in this specification may perform the wake-up assisting operation. The wake-up assisting operation may be used in the same meaning as the wake-up induction or the wake-up induction operation.

The wake-up assisting operation may mean that the sleep assisting device 100 performs an operation for providing a user with an environment suitable for wake-up.

The wake-up assisting operation may mean that the sleep assisting device 100 provides the user with an environment suitable for wake-up using at least any one of wind, lighting, music, alarm, and air freshener.

The wake-up assisting operation may mean that the sleep assisting device 100 provides the user with stimulation suitable for wake-up by stimulating at least any one of the user's tactile, visual, auditory, and olfactory senses using at least any one of gas supply, lighting, music, alarm, and air freshener.

The sleep assisting device 100 may perform any one of the sleep assisting operation and the wake-up assisting operation.

The sleep assisting device 100 may perform any one of the sleep assisting operation and the wake-up assisting operation based on the user input.

Hereinafter, a method of operating the sleep assisting device in a mode determined based on the user input will be described in detail with reference to FIG. 25.

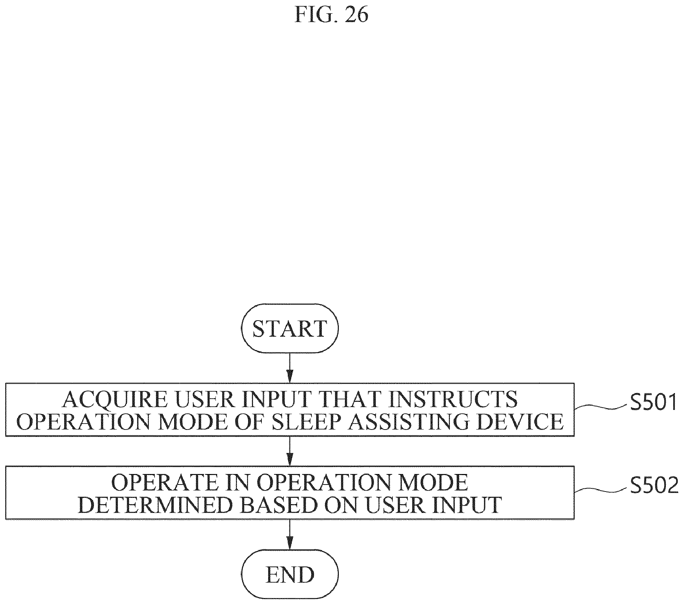

FIG. 26 is a flowchart showing the method of operating the sleep assisting device in the operation mode determined based on the user input.

Referring to FIG. 26, the method of operating the sleep assisting device in the mode determined based on the user input may include an operation of acquiring a user input indicating an operation mode of the sleep assisting device (S501) and an operation of operating the sleep assisting device in the operation mode determined based on the user input (S502).

In the operation of acquiring the user input that instructs the operation mode of the sleep assisting device (S501), the sleep assisting device may acquire the user input from a user interface included in the sleep assisting device 100 or an external device.

The user input may include information that instructs the operation mode of the sleep assisting device 100. The sleep assisting device 100 may acquire the user input that instructs a sleep assisting operation mode so that the sleep assisting device 100 performs the sleep assisting operation. The sleep assisting device 100 may acquire the user input that instructs a wake-up assisting operation mode so that the sleep assisting device 100 performs the wake-up assisting operation.

In the operation of acquiring the user input that instructs the operation mode of the sleep assisting device (S502), the sleep assisting device 100 may be operated in the operation mode determined based on the acquired user input.

According to one embodiment, when the sleep assisting device 100 acquires the user input that instructs the sleep assisting operation mode so that the sleep assisting device 100 performs the sleep assisting operation, the sleep assisting device 100 performs the sleep assisting operation based on the user input.

When the sleep assisting device 100 acquires the user input that instructs the sleep assisting operation mode so that the sleep assisting device 100 performs the sleep assisting operation, the sleep assisting device 100 may perform the sleep assisting operation by supplying the mixed gas containing carbon dioxide to the outside based on the user input.

When the sleep assisting device 100 acquires the user input that instructs the sleep assisting operation mode so that the sleep assisting device 100 performs the sleep assisting operation, the sleep assisting device 100 may control the supply adjustment unit 102 and the flow rate adjustment unit 106 to supply the mixed gas containing carbon dioxide to the outside based on the user input.

In another embodiment, when the sleep assisting device 100 acquires the user input that instructs the wake-up assisting operation mode so that the sleep assisting device 100 performs the wake-up assisting operation, the sleep assisting device 100 may perform the wake-up assisting operation based on the user input.

When the sleep assisting device 100 acquires the user input that instructs the wake-up assisting operation mode so that the sleep assisting device 100 performs the wake-up assisting operation, the sleep assisting device 100 may perform the wake-up assisting operation by supplying a gas to the outside based on the user input.

When the sleep assisting device 100 acquires the user input that instructs the wake-up assisting operation mode so that the sleep assisting device 100 performs the wake-up assisting operation, the sleep assisting device 100 may control the flow rate adjustment unit 106 to supply the gas to the outside based on the user input.

As described above, when the sleep assisting device 100 is operated in the sleep assisting mode, the sleep assisting device 100 may provide the user with a carbon dioxide-rich mixed gas to assist the user's sleep by decreasing the oxygen partial pressure in the user's body. However, when the sleep assisting device 100 is operated in the wake-up assisting mode, the addition of the carbon dioxide to the gas supplied to the user may rather act as a factor that interferes with the user's wake-up assistance. Accordingly, when the sleep assisting device 100 is operated in the wake-up assisting mode, the sleep assisting device 100 may supply the gas for assisting the user's wake-up using only the introduced outside air.

Accordingly, when the sleep assisting device 100 according to one embodiment is operated in the sleep assisting operation mode, the carbon dioxide concentration in the first gas supplied from the injection port may be greater than the carbon dioxide concentration in the second gas supplied from the injection port when the sleep assisting device 100 is operated in the wake-up assisting mode.

In addition, the carbon dioxide concentration in the first gas supplied to the target area when the sleep assisting device 100 is operated in the sleep assisting operation mode may be greater than the carbon dioxide concentration in the second gas supplied to the target area when the sleep assisting device 100 is operated in the wake-up assisting mode.

As described above, when the sleep assisting device 100 is operated in the wake-up assisting mode, the sleep assisting device 100 may assist the user's wake-up by supplying the gas to the outside at a flow rate sufficient to stimulate the user's tactile sense. However, when the sleep assisting device 100 is operated in the sleep assisting mode, since the supply of the gas supplied by the sleep assisting device 100 at a flow rate of the reference flow rate or more may act as a factor that interferes with the user's sleep, the flow rate of the gas may be determined as the value within the numerical range described in 3.3 (User convenience).

Accordingly, the flow rate of the first gas supplied from the injection port when the sleep assisting device 100 according to one embodiment is operated in the sleep assisting operation mode may be smaller than the flow rate of the second gas supplied from the injection port when the sleep assisting device 100 is operated in the wake-up assisting operation mode.

Accordingly, the flow rate of the first gas supplied to the target area when the sleep assisting device 100 according to one embodiment is operated in the sleep assisting operation mode may be smaller than the flow rate of the second gas supplied to the target area when the sleep assisting device 100 is operated in the wake-up assisting operation mode.

Hereinafter, among the wake-up assisting operations, a wake-up assisting operation in which the sleep assisting device 100 provides the user with an environment suitable for waking up using a gas supply (e.g., wind) will be described in detail.

The sleep assisting device 100 provided in this specification may perform the wake-up assistance operation by supplying the gas to the outside. The sleep assisting device 100 may perform the wake-up assisting operation by introducing the gas and supplying the introduced gas to the outside.

The sleep assisting device 100 may perform the wake-up assisting operation by controlling the flow rate adjustment unit 106. The sleep assisting device 100 may perform the wake-up assisting operation by introducing the gas and controlling the flow rate adjustment unit 106 to supply the introduced gas to the outside. The sleep assisting device 100 may perform the wake-up assisting operation by controlling the flow rate adjustment unit 106 so that the flow rate of the gas introduced from the injection port is a predetermined reference flow rate.

The reference flow rate may be a predetermined flow rate. The sleep assisting device 100 may induce the user's wake-up by supplying the gas to the user at the reference flow rate to stimulate the user's tactile sense.

The reference flow rate having the level capable of stimulating the user's tactile sense may be higher than the flow rate of the mixed gas that does not stimulate the user's tactile sense described in 3 (Considerations upon supplying carbon dioxide for sleep assistance).

The sleep assisting device 100 may perform the wake-up assisting operation by controlling the flow rate adjustment unit 106 so that the flow rate of the gas at the injection port gradually reaches the reference flow rate.

For example, when the reference flow rate of the sleep assisting device 100 has a third flow rate, the sleep assisting device 100 may control the flow rate adjustment unit 106 in a first state (e.g., state in which the applied voltage is 4 [V]) so that the flow rate of the gas at the injection port has a first flow rate (e.g., 0.5 [m/s]) smaller than the third flow rate (e.g., 1.5 [m/s]).

After controlling the flow rate adjustment unit 106 in the first state for a predetermined time, the sleep assisting device 100 may control the flow rate adjustment unit 106 in a second state (e.g., state in which the applied voltage is 5 [V]) so that the flow rate of the gas at the injection port has a second flow rate (e.g., 1.0 [m/s]) that is greater than the first flow rate and smaller than the third flow rate.

After controlling the flow rate adjustment unit 106 in the second state for a predetermined time, the sleep assisting device 100 may control the flow rate adjustment unit 106 in a third state (e.g., state in which the applied voltage is 6 [V]) so that the flow rate of the gas at the injection port has the third flow rate (e.g., 1.5 [m/s]).

As described above, the sleep assisting device 100 may provide the user with a comfortable wake-up assisting environment in the sleep state by supplying the gas at the gradually increasing flow rate.

The sleep assisting device 100 may start the wake-up induction operation at a predetermined time. The sleep assisting device 100 may start the wake-up assisting operation at a predetermined reservation time based on information on a reservation time acquired from the user.

The sleep assisting device 100 may perform the wake-up assisting operation by stimulating the user's visual sense. The sleep assisting device 100 may perform the wake-up assisting operation by stimulating the visual sense using a visual stimulation unit.

The visual stimulation unit may include any one of a light source and an imaging device. For example, the visual stimulation unit may include any one of a light emitting diode (LED), an incandescent lamp, and a fluorescent lamp. For another example, the visual stimulation unit may include a display.

The sleep assisting device 100 may perform the wake-up assisting operation by stimulating the user's auditory sense. The sleep assisting device 100 may perform the wake-up assisting operation by stimulating the auditory sense using an auditory stimulation unit.

The auditory stimulation unit may include a sound source. For example, the auditory stimulation unit may include a speaker.

The sleep assisting device 100 may perform the wake-up assisting operation by providing a material that stimulates the olfactory sense. The sleep assisting device 100 may perform the wake-up assisting operation by stimulating the olfactory sense using an olfactory stimulation unit. The olfactory stimulation unit may include a material that stimulates the olfactory sense. For example, the olfactory stimulation unit may include a material that provides aroma scent.

The sleep assisting device 100 may perform the wake-up assisting operation using the flow rate adjustment unit 106 and the olfactory stimulation unit. The sleep assisting device 100 may perform the sleep assisting operation by introducing the outside air through the flow rate adjustment unit 106 to supply the material included in the olfactory stimulation unit to the outside.

The sleep assisting device 100 may perform the wake-up assisting operation based on the distance indication value acquired through the distance detection unit.

The sleep assisting device 100 may perform the wake-up assisting operation based on the distance indication value determined according to a positional state of the injection port configured to supply the gas to the outside. The sleep assisting device 100 may perform a first wake-up assisting operation or a second wake-up assisting operation based on the distance indication value determined according to the positional state of the injection port configured to supply the gas to the outside.

For example, when the flow path unit including the injection port is foldably coupled to a main body of the sleep assisting device, the gas supplied by the sleep assisting device through the injection port may not be provided to the user when the flow path unit is in a state of being folded from the sleep assisting device. In this case, a method of assisting the user's wake-up without supplying the gas may be considered. For example, a method of stimulating any one of the user's auditory and visual senses may be considered.

When the distance indication value determined according to the positional state of the injection port configured to supply the gas to the outside indicates a first wake-up assisting mode, the sleep assisting device 100 may perform the wake-up assisting operation by supplying the gas to the outside. For example, when the flow path unit including the injection port is foldably coupled to the main body of the sleep assisting device, the sleep assisting device 100 may perform the wake-up assisting operation by supplying the gas to the outside based on the distance indication value determined according to the positional state of the injection port configured to supply the gas to the outside when the flow path unit is in a state of being folded from the sleep assisting device.

The sleep assisting device 100 may perform the wake-up assisting operation by stimulating the user's auditory or visual sense when the distance indication value determined according to the positional state of the injection port configured to supply the gas to the outside indicates a second wake-up assisting mode. For example, when the flow path unit including the injection port is foldably coupled to the main body of the sleep assisting device, the sleep assisting device 100 may perform the wake-up assisting operation by stimulating the user's visual and/or auditory senses based on the distance indication value determined according to the positional state of the injection port configured to supply the gas to the outside when the flow path unit is in a state of being open from the sleep assisting device.

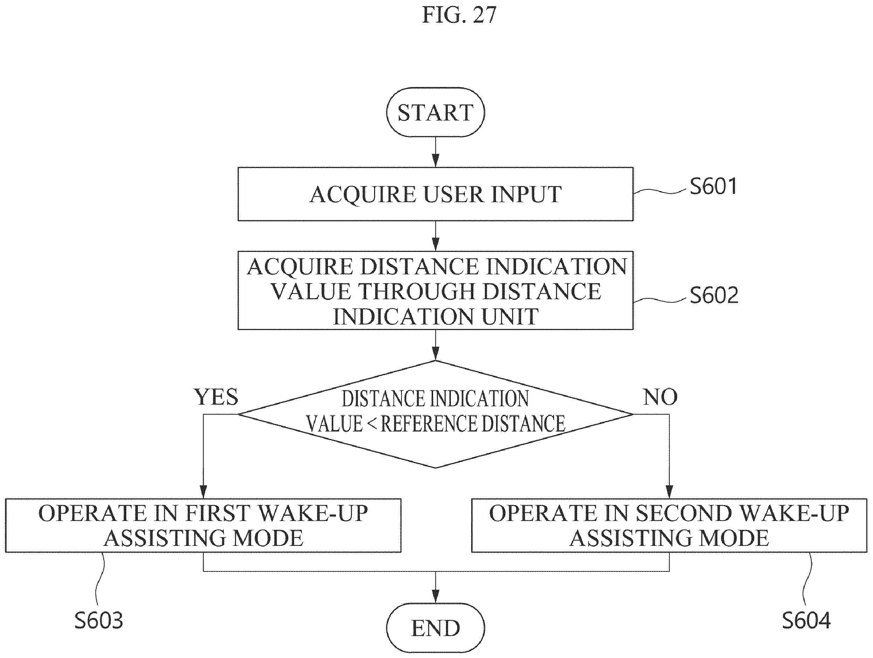

Hereinafter, a method of performing the wake-up assisting operation based on the acquired user input will be described in detail with reference to FIG. 27.

FIG. 27 is a flowchart showing a method of operating the sleep assisting device in the wake-up assisting operation mode determined based on the user input.

Referring to FIG. 27, the method of performing the wake-up induction operation based on the user input may include an operation of acquiring the user input (S601), an operation of acquiring the distance indication value through the distance detection unit (S602), an operation of operating the sleep assisting device in a first wake-up induction mode when the distance indication value is smaller than a reference distance (S603), and an operation of operating the sleep assisting device in a second wake-up induction mode when the distance indication value is greater than the reference distance (S604).

Here, the operation of acquiring the user input that instructs the operation mode of the sleep assisting device (S501) in FIG. 25 may be applied to the operation of acquiring the user input (S601) in the same manner.

Hereinafter, matters added to the operation of acquiring the user input (S601) will be described.

In the operation of acquiring the user input (S601), the sleep assisting device 100 may acquire the user input that instructs the sleep assisting device 100 to perform the wake-up assisting operation by supplying the gas to the outside.

Since the operation of acquiring the distance indication value related to the distance between the injection port and the object (S201) in FIG. 13 may be applied to the operation of acquiring the distance indication value through the distance detection unit (S602) in the same manner, a detailed description thereof will be omitted.

In the operation of acquiring the distance indication value through the distance detection unit (S602), the sleep assisting device 100 may acquire the distance indication value based on the user input.

The sleep assisting device 100 may acquire the distance indication value through the distance detection unit based on the user input. The sleep assisting device 100 may acquire the distance indication value in response to the user input that instructs to operate in the sleep assisting operation mode.

When the distance indication value is smaller than the reference distance, in the operation of operating the sleep assisting device in the first wake-up induction mode (S603), when the distance indication value is smaller than the reference distance, it may mean that the distance between the injection port and the object indicated by the distance indication value is smaller than the predetermined reference distance. In addition, when the distance indication value is smaller than the reference distance, it may mean instructing the sleep assisting device 100 to be operated in the first wake-up induction mode that assists the user's wake-up by stimulating the user's auditory or olfactory sense.

When the distance indication value is smaller than the reference distance, the sleep assisting device 100 may be operated in the first wake-up induction mode. When the distance indication value is smaller than the reference distance, the sleep assisting device 100 may be operated in the first wake-up induction mode that assists the user's wake-up by stimulating the user's auditory or olfactory sense.

When the distance indication value is smaller than the reference distance, in the operation of operating the sleep assisting device in the first wake-up induction mode (S604), when the distance indication value is greater than the reference distance, it may mean that the distance between the injection port and the object indicated by the distance indication value is greater than the predetermined reference distance. In addition, when the distance indication value is greater than the reference distance, it may mean instructing the wake-up assisting operation of the sleep assisting device to be performed in the second wake-up induction mode that assists the user's wake-up by supplying the gas. In addition, when the distance indication value is greater than the reference distance, it may mean instructing the wake-up assisting operation of the sleep assisting device to be performed in the second wake-up induction mode that assists the user's wake-up by supplying the gas and the material that stimulates the olfactory sense.

When the distance indication value is greater than the reference distance, the sleep assisting device 100 may be operated in the second wake-up induction mode. When the distance indication value is greater than the reference distance, the sleep assisting device 100 may be operated in the first wake-up induction mode that assists the user's wake-up by supplying the gas. In addition, when the distance indication value is greater than the reference distance, the sleep assisting device 100 may be operated in the first wake-up induction mode that assists the user's wake-up by stimulating the gas and the user's olfactory sense.

For example, when the distance indication value acquired through the distance detection unit is greater than the reference value, the sleep assisting device 100 may perform the wake-up assisting operation by supplying the gas to the outside. For a specific example, when the distance indication value acquired through the distance detection unit is greater than the reference value, the sleep assisting device 100 may perform the wake-up assisting operation by controlling the flow rate adjustment unit 106 to supply the gas to the outside. For a specific example, when the distance indication value acquired through the distance detection unit is greater than the reference value, the sleep assisting device 100 may perform the wake-up assisting operation by controlling the flow rate adjustment unit 106 to supply the gas and the material that stimulates the user's olfactory sense to the outside.

For another example, when the distance indication value acquired through the distance detection unit is smaller than the reference value, the sleep assisting device 100 may perform the wake-up assisting operation using at least any one of lighting, music, and alarm, which will be described below, without supplying the gas.

When the sleep assisting device 100 performs the wake-up assisting operation, the carbon dioxide supply unit 101 may not supply the carbon dioxide. When the sleep assisting device 100 performs the wake-up assisting operation, the control unit 103 may control the supply adjustment unit 102 so as not to supply the carbon dioxide. When the sleep assisting device 100 performs the wake-up assisting operation, the control unit 103 may control the supply adjustment unit 102 to stop the supply of the carbon dioxide.

According to one embodiment, the sleep assisting device 100 may assist the user's wake-up by providing light whose frequency is related to the user's biosignal using a light source. For example, the biosignal may refer to any one of the user's heart rate, respiration rate, and brainwave.

According to another embodiment, the sleep assisting device 100 may provide light while gradually increasing an intensity of the light.

The sleep assisting device 100 may include the auditory stimulation unit. The auditory stimulation unit may include a sound source. For example, the auditory stimulation unit may include a speaker.

The sleep assisting device 100 may assist the user's wake-up by providing sound using the auditory stimulation unit.

According to one embodiment, the sleep assisting device 100 may assist the user's wake-up by providing the sound whose frequency is related to the user's biosignal using the sound source. For example, the biosignal may refer to any one of the user's heart rate, respiration rate, and brainwave.

According to another embodiment, the sleep assisting device 100 may provide sound while gradually increasing the intensity of the sound.

10. Experimental examples

Hereinafter, through experimental examples, the configuration and effect of the sleep assisting device provided by this specification will be described in more detail. The experimental examples are only for illustrating the contents disclosed by this specification, and it will be apparent to those skilled in the art that the scope of the contents disclosed by this specification is construed as being not limited by these experimental examples.

Each unit of the sleep assisting device described above may be composed of the following experimental examples.

The carbon dioxide supply unit may include a carbon dioxide cylinder.

The carbon dioxide cylinder may be composed of

- Volume: 0.5 - 1 (L).

In addition, the carbon dioxide supply unit may include a carbon dioxide supply hose.

The carbon dioxide supply hose may be composed of

- Body material: Aluminum
- Inlet port thread: 6AMCE (new inlet design, O-ring sealing)
- Outlet port thread: 1/8NPT
- Outlet pressure: 0-3 (bar)
- Pipe connection caliber: 1/4in, 1/8in one touch fitting.

The supply adjustment unit may include a regulator.

The regulator may be composed of

- Body material: Aluminum
- Inlet port thread: 6AMCE (new inlet design, O-ring sealing)
- Outlet port thread: 1/8NPT
- Outlet pressure: 0-3 (bar)
- Pipe connection caliber: 1/4in, 1/8in one touch fitting.

In addition, the supply adjustment unit may include a sol-valve.

The sol-valve may be composed of

- Type: KCAS 2port
- Pipe connection caliber: 1/4in one touch fitting
- Orifice size: 0.6 (mm)
- Valid area: 0.26 mm2
- Pressure: 0 - 7 kgf/cm2 (0-0.7MPa)
- Maximum cycle: 10 cycle/sec
- Response time (5 kgf/cm2 hour): 20 ms or less
- Type: 2/2 way type
- SEAL material: NBR
- BODY material: Aluminum (standard), brass

- Power consumption: DC 2.8 W and AC 3.8/3.3 VA (50/60 Hz)
- Surrounding temperature and use fluid temperature 5 °C ~ 60 °C
- Type of coil insulation Corresponding to F type
- Weight: 58g.

The flow path unit may include a pipe.

The pipe may be composed of

- The Bore: 16 mm
- Material: PVC
- Length 1 m.

The flow rate adjustment unit may include a blower fan.

The blower fan may be composed of

- Model RDH8025S1-Blower
- Fan dimension 75*75*30 mm
- Bearing type sleeve bearing
- Rated voltage 12 VDC
- Rated current 0.23 A
- Rated power 2.76 W
- Air flow13.8 CFM
- Connector 2 pin
- Line 305 mm.

The carbon dioxide concentration detection unit may include a concentration measuring sensor.

The concentration measuring sensor may be composed of

Sensing method: Solid-state Non-Dispersive InfraRed(NDIR) absorption and Patented solid-state: LED and detector and Patented gold-plated optics

Sample Method: Diffusion
Measurement range: 0-5 % and 0~20 % and 0-100 %
Accuracy: $\pm$(70 ppm+5 % of reading) and (100 % range $\pm$ (300 ppm+5 % of reading)

Pressure dependence: 0.15 % of reading per mbar in normal atmospheric conditions

Operating pressure range: 500 mbar - 10 bar
Response time: 10 seconds - 2 minutes.
The flow rate detection unit may include a flow meter.
The flow meter may be composed of

- Measuring range selectable by Jumper: 0...1 m/s and 0...1.5 m/s and 0...2 m/s
- Response time(at constant temperature): typ. 4 sec. or typ. 1 sec.
- utput Digital Modbus: RTU or BACnet MS/TP
- Accuracy 45%RH, 1013 Hectopascal: 0.15... 1 m/s$\pm$(0.04 m/s + 2 % of mv) and 0.15...1.5 m/s$\pm$(0.05 m/s + 2 % of mv) and 0.15...2 m/s$\pm$(0.06 m/s + 2 % of mv).

One or more experimental results described throughout this specification may be results obtained by the experiments according to the experimental examples exemplified above.

Although the embodiments have been described above with reference to the limited embodiments and drawings, various modifications and changes are possible by those skilled in the art from the above description. For example, even when the described techniques are performed in an order different from that of the described method, and/or the described components of the system, structure, device, circuit, and the like are coupled or combined in different forms from those of the described method, or replaced or substituted with other components or equivalents, appropriate results may be achieved.

Accordingly, other implementations, other embodiments, and equivalents to the claims also fall within the scope of the appended claims.

**Claims**

1. A sleep assisting device, comprising:

    a flow path unit configured to provide a movement path through which a gas moves;
    a flow rate adjustment unit configured to introduce a first gas into the flow path unit from outside;
    a supply adjustment unit configured to adjust a supply amount of carbon dioxide supplied to the flow path unit;
    an injection port connected to the flow path unit, and configured to provide a second gas in which the first gas and the carbon dioxide supplied to the flow path unit are mixed to outside;
    a distance detection unit configured to acquire a distance indication value related to a distance between the injection port and an object; and
    a control unit configured to control the flow rate adjustment unit to adjust an amount of the first gas introduced into the flow path unit, and control the supply adjustment unit to adjust the supply amount,
    wherein the control unit controls the supply adjustment unit to adjust the carbon dioxide to the flow path unit when the distance indication value acquired through the distance detection unit is greater than a first reference value and smaller than a second reference value.

2. The sleep assisting device of claim 1,

    wherein the control unit controls the supply adjustment unit to supply the carbon dioxide to the flow path unit at a first supply pressure when the distance indication value is a first value that is greater than the first reference value and smaller than the second reference value, and
    controls the supply adjustment unit to supply the carbon dioxide to the flow path unit at a second supply pressure greater than the first supply pressure when the distance indication value is a second value that is greater than the first reference value and
    smaller than the second reference value, and greater than the first value.

3. The sleep assisting device of claim 1,

    wherein the control unit controls the supply adjustment unit to supply the carbon dioxide to the flow path unit at a first supply pressure when the distance indication value is a first value that is greater than the first reference value and smaller than the second reference value, and
    controls the supply adjustment unit to supply the carbon dioxide to the flow path unit at a second supply pressure smaller than the first supply amount when the distance indication value is a second value that is greater than the first reference value and smaller than the second reference value, and greater than the first value.

4. The sleep assisting device of claim 1,
    wherein the control unit controls the supply adjustment unit to supply the carbon dioxide to the flow path unit when the distance indication value indicates that the distance between the injection port and the object is within a reference distance.

5. The sleep assisting device of claim 1,
    wherein the control unit controls the supply adjustment unit to stop the supply of the carbon dioxide to the flow path unit when the distance indication value is smaller than the first reference value.

6. The sleep assisting device of claim 1,
    wherein the control unit controls the supply adjustment unit to stop the supply of the carbon dioxide to the flow path unit when the distance indication value is greater than the second reference value.

7. The sleep assisting device of claim 1,
    wherein the control unit controls the flow rate adjustment unit so as not to introduce the first gas into the flow path unit when the distance indication value is smaller than the first reference value.

8. The sleep assisting device of claim 1,
    wherein the control unit controls the flow rate adjustment unit so as not to introduce the first gas into the flow path unit when the distance indication value is greater than the second reference value.

9. The sleep assisting device of claim 1,

wherein a concentration of intake carbon dioxide of the object is 5,000 [ppm] or more and 30,000 [ppm] or less.

10. A method of controlling a sleep assisting device comprising a flow path unit configured to provide a movement path through which a gas moves, a flow rate adjustment unit configured to introduce a first gas into the flow path unit from the outside, a supply adjustment unit configured to adjust a supply amount of carbon dioxide supplied to the flow path unit, an injection port connected to the flow path unit, and configured to provide a second gas in which the first gas and the carbon dioxide supplied to the flow path unit are mixed to the outside, a distance detection unit configured to acquire a distance indication value related to a distance between the injection port and an object, and a control unit configured to control the flow rate adjustment unit to adjust an amount of the first gas introduced into the flow path unit, and control the supply adjustment unit to adjust the supply amount, the method comprising:

acquiring a first distance indication value related to the distance between the injection port and the object through the distance detection unit; and
controlling the supply adjustment unit to supply the carbon dioxide to the flow path unit by a control unit when the first distance indication value is greater than a first reference value and smaller than a second reference value.

11. The method of claim 10,

wherein the controlling of the supply adjustment unit to supply the carbon dioxide to the flow path unit by the control unit when the first distance indication value is greater than the first reference value and smaller than the second reference value further includes controlling the supply adjustment unit to supply the carbon dioxide to the flow path unit at a first supply pressure by the control unit, and
further comprising:

acquiring a second distance indication value related to the distance between the injection port and the object through the distance detection unit; and
controlling the supply adjustment unit to supply the carbon dioxide to the flow path unit at a second supply pressure greater than the first supply amount -by the control unit when the second distance indication value is greater than the first reference value and smaller than the second reference value, and greater than the first distance indication value.

12. The method of claim 10,

wherein the controlling of the supply adjustment unit to supply the carbon dioxide to the flow path unit by the control unit when the first distance indication value is greater than the first reference value and smaller than the second reference value further includes controlling the supply adjustment unit to supply the carbon dioxide to the flow path unit at a first supply pressure by the control unit, and
further comprising:

acquiring a second distance indication value related to the distance between the injection port and the object through the distance detection unit; and
controlling the supply adjustment unit to supply the carbon dioxide to the flow path unit at a second supply pressure smaller than the first supply amount -by the control unit when the second distance indication value is greater than the first reference value and smaller than the second reference value, and greater than the first distance indication value.

13. The method of claim 10,
wherein the controlling of the supply adjustment unit to supply the carbon dioxide to the flow path unit by the control unit when the first distance indication value is greater than the first reference value and smaller than the second reference value further includes controlling the supply adjustment unit to supply the carbon dioxide to the flow path unit by the control unit when the first distance indication value acquired through the distance detection unit indicates that the distance between the injection port and the object is within a reference range.

14. The method of claim 10,
wherein the acquiring of the first distance indication value related to the distance between the injection port and the object through the distance detection unit by the control unit further includes controlling the supply unit -so as not to supply the carbon dioxide to the flow path unit by the control unit when the first distance indication value is smaller than the first reference value.

**15.** The method of claim 10,
wherein the acquiring of the first distance indication value related to the distance between the injection port and the object through the distance detection unit by the control unit further includes controlling the supply adjustment unit so as not to supply the carbon dioxide to the flow path unit by the control unit when the first distance indication value is greater than the second reference value.

**16.** The method of claim 10,
wherein the acquiring of the first distance indication value related to the distance between the injection port and the object through the distance detection unit by the control unit further includes controlling the flow rate adjustment unit so as not to introduce the first gas into the flow path unit when the first distance indication value is smaller than the first reference value.

**17.** The method of claim 10,
wherein the acquiring of the first distance indication value related to the distance between the injection port and the object through the distance detection unit by the control unit further includes controlling the flow rate adjustment unit so as not to introduce the first gas into the flow path unit by the control unit when the first distance indication value is greater than the second reference value.

**18.** The method of claim 10,
wherein a concentration of intake carbon dioxide of the object is 5,000 [ppm] or more and 30,000 [ppm] or less.

**19.** A computer program stored in a computer-readable recording medium implemented to execute the method of any one of claims 10 to 18.

FIG. 1

USER EXPERIMENT1

## FIG. 2

### USER EXPERIMENT2

EP 4 190 383 A1

## FIG. 3

## USER EXPERIMENT3

EP 4 190 383 A1

FIG. 4

CARBON DIOXIDE CONCENTRATION=10,000[ppm]
FIRST GROUP (20 PERSONS)

% BEFORE USE OF PRODUCT
% AFTER USE OF PRODUCT

EP 4 190 383 A1

## FIG. 5

### CARBON DIOXIDE CONCENTRATION=20,000 [ppm]
### SECOND GROUP (20 PERSONS)

BEFORE USE OF PRODUCT
AFTER USE OF PRODUCT

EP 4 190 383 A1

FIG. 6

FIG. 7

FIG. 8

```
100 ──┐  ┌─────────────────────────────────────────────┐
        │           SLEEP ASSISTING DEVICE              │
        │   ┌──────────────┐  ┌──────────────────────┐  │
        │   │              │  │  CARBON DIOXIDE      │──── 101
        │   │              │  │  SUPPLY UNIT         │  │
103 ──────│ CONTROL UNIT │  └──────────────────────┘  │
        │   │              │  ┌──────────────────────┐  │
        │   │              │←→│     SUPPLY           │──── 102
        │   └──────────────┘  │ ADJUSTMENT UNIT      │  │
        │                     └──────────────────────┘  │
        └─────────────────────────────────────────────┘
```

FIG. 9

100

103

SLEEP ASSISTING DEVICE

CONTROL UNIT ←→

CARBON DIOXIDE
SUPPLY UNIT                101

SUPPLY
ADJUSTMENT UNIT           102

FLOW PATH UNIT            104

FIG. 10

(a)

(b)

(c)

FIG. 11

FIG. 12

105a

105b

105a

105b

105a

105b

FIG. 13

START

ACQUIRE DISTANCE INDICATION VALUE RELATED
TO DISTANCE BETWEEN INJECTION PORT AND OBJECT — S201

CONTROL POWER SOURCE OF SLEEP ASSISTING
DEVICE BASED ON DISTANCE INDICATION VALUE — S202

END

EP 4 190 383 A1

FIG. 14

INJECTION PORT CARBON DIOXIDE CONCENTRATION[ppm] (y-axis: 0, 50000, 100000, 150000, 200000, 250000)

VOLTAGE OF FLOW RATE ADJUSTMENT UNIT[V] (x-axis: 4, 5, 6)

—○—0.1[bar]  —△—0.2[bar]  —□—0.3[bar]

EP 4 190 383 A1

FIG. 15

SLEEP ASSISTING DEVICE

100

103 —— CONTROL UNIT

CARBON DIOXIDE SUPPLY UNIT —— 101

FLOW PATH UNIT —— 104

SUPPLY ADJUSTMENT UNIT —— 102

FLOW RATE ADJUSTMENT UNIT —— 106

FIG. 16

START

ACQUIRE USER INPUT ⸺ S401

CONTROL SUPPLY ADJUSTMENT UNIT AND FLOW RATE ADJUSTMENT UNIT TO ADJUST
FLOW RATE AND/OR CARBON DIOXIDE CONCENTRATION BASED ON USER INPUT ⸺ S402

END

FIG. 17

EP 4 190 383 A1

FIG. 18

FIG. 19

FIG. 20

FIG. 21

*CARBON DIOXIDE SUPPLY PRESSURE=0.2[bar]

FIG. 22

*CARBON DIOXIDE SUPPLY PRESSURE = 0.3[bar]

FIG. 23

FIG. 23 — Graph of FLOW RATE OF MIXED GAS [m/s] versus TARGET DISTANCE [cm], with series 4[V], 5[V], and 6[V].

FIG. 24

```
        ┌─────────┐
        │  START  │
        └────┬────┘
             ▼
┌──────────────────────────────┐
│  ACQUIRE CONTROL VARIABLE    │──── S101
└──────────────┬───────────────┘
               ▼
┌──────────────────────────────┐
│  CONTROL EACH UNIT BASED     │──── S102
│  ON CONTROL VARIABLE         │
└──────────────┬───────────────┘
             ▼
        ┌─────────┐
        │   END   │
        └─────────┘
```

FIG. 25

100

SLEEP
ASSISTING DEVICE

200

USER
TERMINAL

Network

300

SERVER

FIG. 26

```
        ┌─────────┐
        │  START  │
        └────┬────┘
             │
             ▼
┌────────────────────────────────────┐
│  ACQUIRE USER INPUT THAT INSTRUCTS  │──── S501
│ OPERATION MODE OF SLEEP ASSISTING DEVICE │
└────────────────┬───────────────────┘
                 │
                 ▼
┌────────────────────────────────────┐
│       OPERATE IN OPERATION MODE     │──── S502
│    DETERMINED BASED ON USER INPUT   │
└────────────────┬───────────────────┘
                 │
                 ▼
        ┌─────────┐
        │   END   │
        └─────────┘
```

# FIG. 27

```
         ┌─────────┐
         │  START  │
         └────┬────┘
              │
              ▼
   ┌──────────────────────┐
   │  ACQUIRE USER INPUT  │────── S601
   └──────────┬───────────┘
              │
              ▼
   ┌──────────────────────────┐
   │ ACQUIRE DISTANCE         │
   │ INDICATION VALUE THROUGH │────── S602
   │ DISTANCE INDICATION UNIT │
   └──────────┬───────────────┘
              │
              ▼
```

YES ◄────── DISTANCE INDICATION VALUE < REFERENCE DISTANCE ──────► NO

```
   ┌──────────────────────┐          ┌──────────────────────┐
   │ OPERATE IN FIRST     │          │ OPERATE IN SECOND    │
   │ WAKE-UP ASSISTING    │          │ WAKE-UP ASSISTING    │
   │ MODE                 │          │ MODE                 │
   └──────────┬───────────┘          └──────────┬───────────┘
        S603  │                                 │  S604
              │          ┌─────────┐            │
              └─────────►│   END   │◄───────────┘
                         └─────────┘
```

FIG. 28

[ROUND 1]

| VOLTAGE OF FLOW RATE ADJUSTMENT UNIT[V] | $CO_2$ SUPPLY PRESSURE(bar) | $O_2$ PARTIAL PRESSURE CHANGE RATE(%) | $CO_2$ PARTIAL PRESSURE CHANGE RATE(%) |
|---|---|---|---|
| 4 | 0.1 | -0.11 | 318.54 |
| | 0.2 | -0.16 | 521.19 |
| | 0.3 | -0.21 | 703.86 |
| 5 | 0.1 | -0.09 | 252.43 |
| | 0.2 | -0.12 | 302.38 |
| | 0.3 | -0.14 | 446.66 |
| 6 | 0.1 | -0.07 | 166.69 |
| | 0.2 | -0.09 | 244.15 |
| | 0.3 | -0.11 | 315.74 |

EP 4 190 383 A1

FIG. 29

[ROUND 2]

| VOLTAGE OF FLOW RATE ADJUSTMENT UNIT[V] | $CO_2$ SUPPLY PRESSURE(bar) | $O_2$ PARTIAL PRESSURE CHANGE RATE(%) | $CO_2$ PARTIAL PRESSURE CHANGE RATE(%) |
|---|---|---|---|
| 4 | 0.1 | -0.12 | 293.42 |
|  | 0.2 | -0.17 | 457.73 |
|  | 0.3 | -0.21 | 619.10 |
| 5 | 0.1 | -0.09 | 212.40 |
|  | 0.2 | -0.13 | 317.61 |
|  | 0.3 | -0.15 | 399.03 |
| 6 | 0.1 | -0.08 | 161.36 |
|  | 0.2 | -0.10 | 233.20 |
|  | 0.3 | -0.12 | 274.71 |

FIG. 30

[ROUND 3]

| VOLTAGE OF FLOW RATE ADJUSTMENT UNIT[V] | $CO_2$ SUPPLY PRESSURE(bar) | $O_2$ PARTIAL PRESSURE CHANGE RATE(%) | $CO_2$ PARTIAL PRESSURE CHANGE RATE(%) |
|---|---|---|---|
| 4 | 0.1 | -0.12 | 306.81 |
|   | 0.2 | -0.16 | 445.59 |
|   | 0.3 | -0.21 | 625.49 |
| 5 | 0.1 | -0.09 | 205.86 |
|   | 0.2 | -0.12 | 281.24 |
|   | 0.3 | -0.14 | 386.13 |
| 6 | 0.1 | -0.07 | 151.29 |
|   | 0.2 | -0.09 | 221.51 |
|   | 0.3 | -0.11 | 284.43 |

## INTERNATIONAL SEARCH REPORT

| International application No. |
|---|
| **PCT/KR2020/015013** |

| A. | CLASSIFICATION OF SUBJECT MATTER |
|---|---|

**A61M 21/02**(2006.01)i; **A61M 16/10**(2006.01)i; **A61M 16/00**(2006.01)i; **A61M 21/00**(2006.01)i

According to International Patent Classification (IPC) or to both national classification and IPC

| B. | FIELDS SEARCHED |
|---|---|

Minimum documentation searched (classification system followed by classification symbols)

A61M 21/02(2006.01); A61B 3/113(2006.01); A61B 5/04(2006.01); A61B 5/0476(2006.01); A61M 16/12(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 수면보조(sleep aid), 외기(air), 이산화탄소(carbon dioxide), 혼합(mix), 유로(flow path), 분사(injection), 거리 감지(distance sensing), 제어(control)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | US 2004-0144383 A1 (THOMAS, Robert J. et al.) 29 July 2004 (2004-07-29)<br>   See claims 1, 6, 19, 24 and 32; and figure 1. | 1-19 |
| A | US 2004-0255939 A1 (FELDMAN, Spencer) 23 December 2004 (2004-12-23)<br>   See entire document. | 1-19 |
| A | US 2017-0333664 A1 (LUO, Yuanming) 23 November 2017 (2017-11-23)<br>   See entire document. | 1-19 |
| A | KR 10-2012-0108491 A (YU, Namgu) 05 October 2012 (2012-10-05)<br>   See entire document. | 1-19 |
| A | KR 10-2019-0066268 A (KIM, Do Hyun) 13 June 2019 (2019-06-13)<br>   See entire document. | 1-19 |

☐ Further documents are listed in the continuation of Box C.     ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **21 April 2021** | **21 April 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
Information on patent family members

International application No.

**PCT/KR2020/015013**

| Patent document cited in search report | | | Publication date (day/month/year) | Patent family member(s) | | | Publication date (day/month/year) |
|---|---|---|---|---|---|---|---|
| US | 2004-0144383 | A1 | 29 July 2004 | EP | 1590029 | A1 | 02 November 2005 |
| | | | | EP | 1590029 | B1 | 24 June 2009 |
| | | | | EP | 1923088 | A2 | 21 May 2008 |
| | | | | EP | 1923088 | A3 | 03 December 2008 |
| | | | | JP | 2006-513004 | A | 20 April 2006 |
| | | | | US | 7886740 | B2 | 15 February 2011 |
| | | | | WO | 2004-069317 | A1 | 19 August 2004 |
| | | | | WO | 2004-069317 | A8 | 09 December 2004 |
| US | 2004-0255939 | A1 | 23 December 2004 | US | 7299802 | B2 | 27 November 2007 |
| US | 2017-0333664 | A1 | 23 November 2017 | CN | 105748069 | A | 13 July 2016 |
| | | | | CN | 105748069 | B | 23 October 2018 |
| | | | | US | 10898669 | B2 | 26 January 2021 |
| KR | 10-2012-0108491 | A | 05 October 2012 | None | | | |
| KR | 10-2019-0066268 | A | 13 June 2019 | None | | | |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ISSEY TAKAHASHI ; TETSUO TAKAISHI ; KIYOKO YOKOYAMA.** Overcoming Drowsiness by Inducing Cardiorespiratory Phase Synchronization. *IEEE TRANSACTIONS ON INTELLIGENT TRANSPORTATION SYSTEMS,* 2014, vol. 15 (3